(19) Europäisches Patentamt
European Patent Office
Office européen des brevets

(11) Veröffentlichungsnummer: **0 582 902 B1**

# (12) EUROPÄISCHE PATENTSCHRIFT

(45) Veröffentlichungstag der Patentschrift: **29.11.95**

(21) Anmeldenummer: **93112097.6**

(22) Anmeldetag: **29.07.93**

(51) Int. Cl.6: **C07C 333/24**, A01N 47/20, C07C 333/22, C07C 333/20, C07C 329/16, C07C 329/00, C07C 251/40, C07C 271/28, C07C 271/12, C07D 213/75, C07D 317/64, A01N 47/12, A01N 47/06

## (54) Benzylderivate und diese enthaltende Schädlingsbekämpfungsmittel.

(30) Priorität: **08.08.92 DE 4226303**

(43) Veröffentlichungstag der Anmeldung: **16.02.94 Patentblatt 94/07**

(45) Bekanntmachung des Hinweises auf die Patenterteilung: **29.11.95 Patentblatt 95/48**

(84) Benannte Vertragsstaaten: **AT BE CH DE DK ES FR GB GR IE IT LI NL PT SE**

(56) Entgegenhaltungen:
**EP-A- 0 178 826**
**EP-A- 0 253 213**
**EP-A- 0 378 308**

(73) Patentinhaber: **BASF Aktiengesellschaft**
**Carl-Bosch-Strasse 38**
**D-67063 Ludwigshafen (DE)**

(72) Erfinder: **Winger, Horst, Dr.**
**D 3,1**
**D-6800 Mannheim (DE)**
Erfinder: **Sauter, Hubert, Dr.**
**Neckarpromenade 20**
**D-6800 Mannheim (DE)**
Erfinder: **Ammermann, Eberhard, Dr.**
**Von-Gagern-Strasse 2**
**D-6148 Heppenheim (DE)**
Erfinder: **Lorenz, Gisela, Dr.**
**Erlenweg 13**
**D-6730 Neustadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Benzylderivate und Schädlingsbekämpfungsmittel zur Bekämpfung von Schädlingen, insbesondere von Pilzen, Insekten, Nematoden und Spinnmilben. Es ist bekannt, daß bestimmte $\beta$-Methoxyacrylate (vgl. EP 178826) wie auch bestimmte Oximether (vgl. EP 253213) fungizide bzw. insektizide Aktivität besitzen. Ihre Wirkung ist jedoch unbefriedigend.

Es wurde nun überraschend gefunden, daß Benzylderivate der allgemeinen Formel I

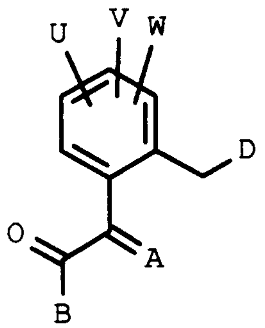

I

in der

A

$CH_2$, CHCl, CH-$C_1$-$C_4$-Alkyl, CH-$C_1$-$C_4$-Alkoxy, CH-$C_1$-$C_4$-Alkylthio, N-$C_1$-$C_4$-Alkoxy bedeutet,

B

OH, $C_1$-$C_4$-Alkylthio, $C_1$-$C_4$-Alkoxy, $C_1$-$C_4$-Alkylamino bedeutet,

U, V, W

gleich oder verschieden sind und Wasserstoff, Halogen, $C_1$-$C_4$-Alkyl oder $C_1$-$C_4$-Alkoxy bedeuten,

D

die Gruppierung

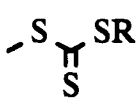 , 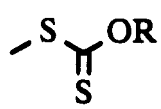 , 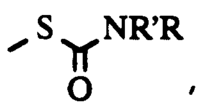 , 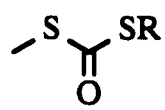

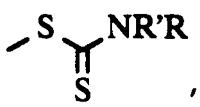 , 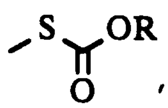 , 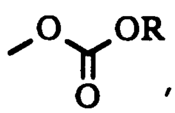 , 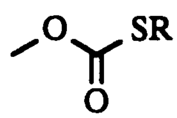

oder 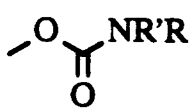

bedeutet,

wobei

R'

Wasserstoff oder $C_1$-$C_4$-Alkyl bedeutet und

R

Wasserstoff, $C_1$-$C_{10}$-Alkyl, $C_3$-$C_6$-Cycloalkyl, $C_1$-$C_4$-Halogenalkyl, $C_3$-$C_6$-Halogencycloalkyl, $C_3$-$C_6$-Cycloalkyl-$C_1$-$C_4$-alkyl, $C_1$-$C_4$Alkoxy-$C_1$-$C_4$-alkyl, $C_1$-$C_4$-Alkylthio-$C_1$-$C_4$-alkyl, ggf. subst. Arylthio-$C_1$-$C_4$-alkyl, ggf. subst. Aryloxy-$C_1$-$C_4$-alkyl, ggf. subst. Aryl, ggf. subst. Aryl-$C_1$-$C_4$-alkyl, ggf. subst. Hetaryl, ggf. subst. Hetaryl-$C_1$-$C_4$-alkyl, ggf. subst. Hetaryloxy-$C_1$-$C_4$-alkyl, ggf. subst. Heterocyclyl bedeutet, wobei "ggf. subst." neben Wasserstoff die Reste Halogen, Cyano, $CO_2$($C_1$-$C_4$-Alkyl), CO($C_1$-$C_4$-Alkyl), Nitro, $C_1$-$C_4$-Alkyl, $C_1$-$C_4$-Alkoxy, $C_1$-$C_4$Halogenalkyl, $C_1$-$C_4$-Halogenalkoxy, $C_1$-$C_4$-Alkoximino-$C_1$-$C_2$-alkyl, Aryl, Aryloxy, Benzyloxy, Hetaryl, Hetaryloxy, $C_3$-$C_6$-Cycloalkyl, Heterocyclyl, Heterocyclyloxy bedeuten,

eine ausgezeichnete fungizide, insektizide, nematizide und akarizide Wirkung haben, die besser ist, als die

der bekannten β-Methoxyacrylate, bzw. Oximether. Die fungizide Wirkung wird bevorzugt.

Die in der allgemeinen Formel I aufgeführten Reste können beispielsweise die folgende Bedeutung haben:

A

kann $C_1$-$C_4$-Alkyliden (z. B. Methyliden, Ethyliden, n- oder iso-Propyliden, n-, iso-, sec.- oder tert.-Butyliden), $C_1$-$C_4$-Alkoxymethyliden (z. B. Methoxy-, Ethoxy-, n- oder iso-Propoxy, n-, iso-, sec.- oder tert.-Butoxymethyliden), $C_1$-$C_4$-Alkylthiomethyliden (z. B. Methyl-, Ethyl-, n- oder iso-Propyl, n-, iso-, sec.- oder tert.-Butylthiomethyliden), $C_1$-$C_4$-Alkoxyimino (z. B. Methoxy-, Ethoxy-, n- oder iso-Propoxy-, n-, iso-, sec.- oder tert.-Butoxyimino), Chlormethyliden bedeuten,

B

kann OH, $C_1$-$C_4$-Alkoxy (z. B. Methoxy, Ethoxy, n- oder iso-Propoxy, n-, iso-, sec.- oder tert.-Butoxy), $C_1$-$C_4$-Alkylthio (z. B. Methylthio, Ethylthio, n- oder iso-Propylthio, n-, iso-, sec.-oder tert.-Butylthio) und $C_1$-$C_4$-Alkylamino (z. B. Methylamino, Ethylamino, n- oder iso-Propylamino, n-, iso-, sec.- oder tert.-Butylamino) bedeuten,

U, V, W

können H, Halogen (z. B. Fluor, Chlor, Brom, Jod), Methyl, Methoxy bedeuten,

D

kann die Gruppierung

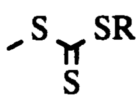 , 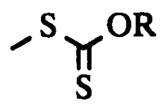 , 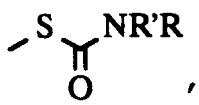 , 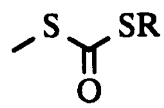

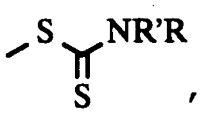 , 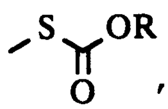 , 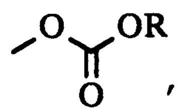 , 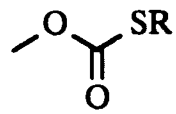

oder 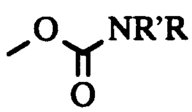

bedeuten,

R' kann

Wasserstoff oder $C_1$-$C_4$-Alkyl (z. B. Methyl, Ethyl, n- oder iso-Propyl, n-, iso-, sec- oder tert.-Butyl) bedeuten und

R kann

ggf. verzweigtes $C_1$-$C_{10}$-Alkyl (z. B. Methyl, Ethyl, n- oder iso-Propyl, n-, iso-, sec.- oder tert.-Butyl, n-, iso-, sec.-, tert.- oder neo-Pentyl, n-Hexyl, n-Decyl),

$C_3$-$C_6$-Cycloalkyl (z. B. Cyclopropyl, Cyclobutyl, Cyclopentyl, Cyclohexyl),

$C_1$-$C_4$-Halogenalkyl (z. B. Trifluormethyl, 2-Fluorethyl, 2,2,2-Trifluorethyl, Pentafluorethyl, Fluordichlormethyl, Difluorchlormethyl, Chlormethyl, Trichlormethyl, 2-Chlorethyl, 2,2,2-Trichlorethyl, Pentachlorethyl),

$C_3$-$C_6$-Halogencycloakyl (z. B. 2,2-Difluorcyclopropyl,2,2-Dichlorcyclopropyl, 2,2-Dibromcyclopropyl, 2,2-Dichlor-3-Methylcyclopropyl, Tetrafluorcyclobutyl),

$C_3$-$C_6$-Cycloalkyl-$C_1$-$C_4$-alkyl (z. B. 1-Methylcyclopropyl, 2,2-Dimethylcyclopropyl, 1-Methylcyclohexy),

$C_1$-$C_4$-Alkoxy-$C_1$-$C_4$-alkyl (z. B Methoxymethyl, Ethoxymethyl, n-oder iso-Propoxylmethyl, n-, iso-, sec.- oder tert.-Butoxymethyl, 2-Methoxy-prop-2-yl, 2-Ethoxyprop-2-yl, 2-n- oder iso-Propoxyprop-2-yl, 2-n-, iso-, sec.- oder tert.-Butoxyprop-2-yl),

$C_1$-$C_4$-Alkylthio-$C_1$-$C_4$-alkyl (z. B. Methylthiomethyl, Ethylthiomethyl, n-, oder iso-Propylthiomethyl, n-, iso-, sec.-oder tert.-Butylthiomethyl, 2-Methylthioprop-2-yl, 2-Ethylthioprop-2-yl, 2-n- oder iso-Propylthio-prop-2-yl, 2-n-, iso-, sec.- oder tert.-Butylthio-prop-2-yl),

Aryl(Phenyl)thio-$C_1$-$C_4$-alkyl (z. B. Phenylthiomethyl, 2-Chlorphenyl-thiomethyl),

ggf. subst. Aryloxy-$C_1$-$C_4$-alkyl (z. B. Phenoxymethyl),

ggf. subst. Aryl (z. B. Phenyl, Naphthyl, Anthryl),

ggf. subst. Aryl-$C_1$-$C_4$-alkyl (z. B. Benzyl, 1-Phenethyl, 2-Phenethyl, 1-Phenylpropyl, 2-Phenylpropyl, 3-Phenylpropyl, 2-Methyl-3-phenylpropyl, 2-Methyl-2-phenylpropyl, 4-Phenylbutyl),

ggf. subst. Heteroaryl (z. B. Pyridyl, 2-Pyridyl, 3-Pyridyl, 4-Pyridyl, Pyrimidinyl, 4-Pyrimidinyl, 2-Pyrimidinyl,

Thienyl, 2-Thienyl, 3-Thienyl, Furyl, 2-Furyl, 3-Furyl, 1-Pyrrolyl, 1-Imidazolyl, 1,2,4-Triazolyl, 1,3,4-Triazolyl, 4-Thiazolyl, 2-Benzothiazolyl),
ggf. subst. Heteroaryl-$C_1$-$C_4$-alkyl (z. B. 2-Pyridylmethyl, 3-Pyridylmethyl),
ggf. subst. Heteroaryloxy-$C_1$-$C_4$-alkyl (z. B. Furfurylmethoxy),
ggf. subst. Heterocyclyl (z. B. Oxiranyl, 1-Aziridinyl, 1-Azetidinyl, 1-Pyrrolidinyl, 2-Tetrahydrofuryl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl) bedeuten.

Die mit "ggf. substituiert" bezeichneten Reste im Vorstehenden sind außer durch Wasserstoff z. B. substituiert durch Fluor, Chlor, Brom, Jod, Cyano, Nitro, Methyl, Ethyl, iso-Propyl, tert.-Butyl, Methoxy, Ethoxy, iso-Propoxy, tert.-Butoxy, Trifluormethyl, Chlormethyl, Dichlormethyl, Trichlormethyl, Trifluormethoxy, 1,1,2,2-Tetrafluorethoxy, Methoxyiminomethyl, Ethoxyiminomethyl, n-Propoxyiminomethyl, n-Butoxyiminomethyl, n-Pentoxyiminomethyl, n-Hexoxyiminomethyl, Allyloxyiminomethyl, Benzyloxyiminomethyl, iso-Propoxyiminomethyl, iso-Butoxyiminomethyl, tert.-Butoxyiminomethyl, Methylimino-1-ethyl, Ethoxyimino-1-ethyl, n-Propoxyimino-1-ethyl, n-Butoxyimino-1-ethyl, n-Pentoxyimino-1-ethyl, n-Hexoxyimino-1-ethyl, Allyloximino-1-ethyl, Benzyloxyimino-1-ethyl, Phenyl, Phenoxy, Benzyloxy, Imidazol-1-yl, Piperazin-1-yl, 4-Morpholinyl, Piperidin-1-yl, Pyridyl-2-oxy, Cyclopropyl, Cyclohexyl, Oxiranyl, 1,3-Dioxan-2-yl, 1,3-Dioxolan-2-yl, Tetrahydropyran-2-yloxy.

Bevorzugt werden Verbindungen der allgemeinen Formel I, in der

A
$CHCH_3$, $CHCH_2CH_3$, $CHOCH_3$, $NOCH_3$
B
$OCH_3$, $NHCH_3$
U, V, W
Wasserstoff
R'
Wasserstoff, Methyl bedeuten und
D, R
die unter Anspruch 1 beschriebene Bedeutung haben.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z. B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die Herstellung der Verbindungen der allgemeinen Formel I gemäß Anspruch 1 erfolgt beispielsweise wie in Schema 1 beschrieben.

Schema 1

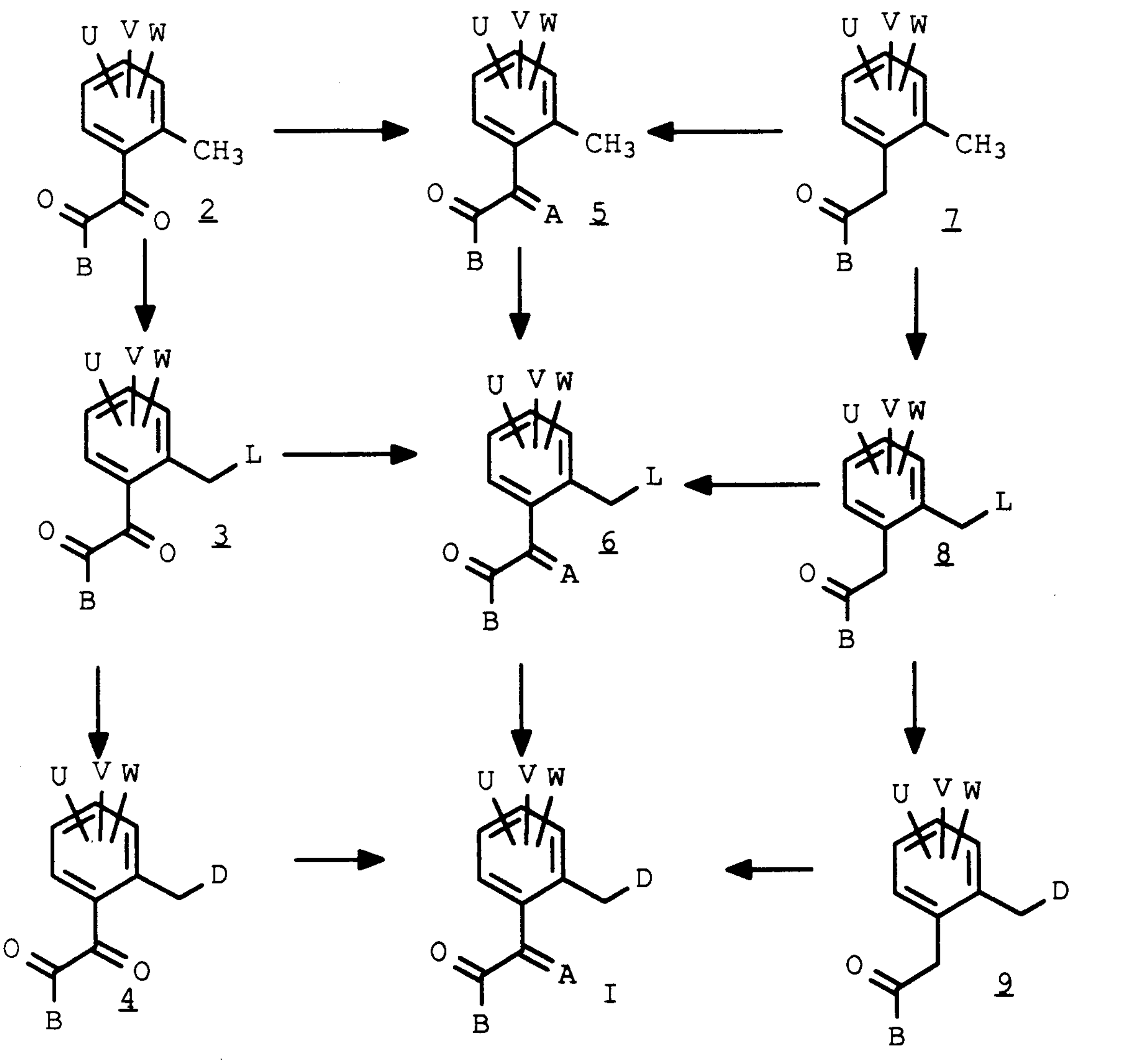

2 5 7 3 6 8 4 I 9

Die Verbindungen der allgemeinen Formel I, in denen A = $CH_2$, CH-Alkyl, CH-Alkoxy ist, lassen sich beispielsweise aus den Ketoesters 4 durch Wittig- oder Wittig-Horner-Reaktion herstellen (vgl. EP 348 766, DE 3 705 389, EP 178 826). Ebenso erhält man die analogen Verbindungen 5 aus den Ketoestern 2.

Alternativ kann auch so vorgegangen werden, daß man Verbindungen der Formel 7 bzw. 9 mit geeigneten Reagentien kondensiert, z. B: für A = $CH_2$ mit Formaldehyd (s. DE 3 317 356), für A = CH-Alkyl a) mit Aldehyden (vgl. D.M. Brown, J. Chem. Soc. 1948, 2147) oder b) zuerst mit N,N-Dimethylformamiddimethylacetal, gefolgt von der Reaktion mit einem Grignardreagenz (analog zu C. Jutz, Chem. Ber. 91, 1867 (1958)), für A = CH-O-Alkyl mit Ameisensäureester gefolgt von einer Alkylierung (s. EP 178 826). Weitere Herstellvorschriften für die Verbindungen der Formel 5 und I mit A = CH-O-Alkyl sind beschrieben in EP 178 826.

Eine weitere Möglichkeit zur Herstellung der Verbindungen der Formel I mit A = CH-Alkyl und B = O-Alkyl ist die Umsetzung von Ketenacetalen mit Phenylchlorcarbonaten (s. N. Slougui, G. Rousseau, Synth. Commun. 12 (5) 401-7 (1982)).

Für Verbindungen der allgemeinen Formel I in der A = CH-S-Alkyl und A = CHCl ist, kann die Herstellung nach den Methoden aus EP 244 077 oder 310 954 erfolgen.

Die Zwischenprodukte der Formel 3, 6 und 8 lassen sich aus den Verbindungen 2, 5 und 7 herstellen, indem man diese nach bekannten Methoden halogeniert, z. B. mit Chlor, Brom, N-Bromsuccinimid in einem inerten Lösungsmittel (z. B. $CCl_4$, Cyclohexan) unter Belichtung mit z. B. einer Hg-Dampflampe oder

Radikalstartern, wie z. B. Dibenzoylperoxid, oder indem man über geeignete Zwischenverbindungen (L = Halogen, OH) die Reste L wie z. B. Mesylat, Tosylat, Acetat oder Triflat einführt.

Die Oximether der Formel I mit A = N-O-Alkyl lassen sich aus 4 a) durch Umsetzung mit O-Alkylhydroxylaminhydrochlorid oder b) mit Hydroxylaminhydrochlorid und nachfolgender Alkylierung mit einem Alkylierungsmittel (wie z. B. Alkyliodid, Dialkylsulfat etc.) herstellen (vgl. DE 3 623 921).

Ebenso kann analog zur Methode im EP 254 426 ein Phenylessigester der Formel 9 mit einer Base (wie z. B. NaOMe, NaH, K-tert.-Butylat, etc.) in einem Lösungsmittel (wie z. B. Diethylether, Toluol, tert.-Butanol etc.) in sein Anion überführt und mit einem geeigneten Nitrosierungsmittel (wie z.B. Methylnitrit, Amylnitrit, tert.-Butylnitrit etc.) oximiert werden. Das resultierende Oximat wird mit einem Alkylierungsmittel (wie z. B. Alkyliodid, Dialkylsulfat) alkyliert.

Dieselben Verfahren sind entsprechend auch auf die Verbindungen der Formel 2 und 7 übertragbar, wobei die resultierenden Oximether 5 wie bekannt (EP 254 426) über die Intermediate 6 (L = z. B. Halogen) in die Zielverbindungen I überführt werden können.

Die neuen Verbindunge der allgemeinen Formel 1 erhält man im Speziellen dadurch, daß man ein Benzylderivat mit einer geeigneten Abgangsgruppe L (z. B. Halogen) 6 mit einem Kohlensäure-salz 10 in einem inerten Lösungs- oder Verdünnungsmittel umsetzt.

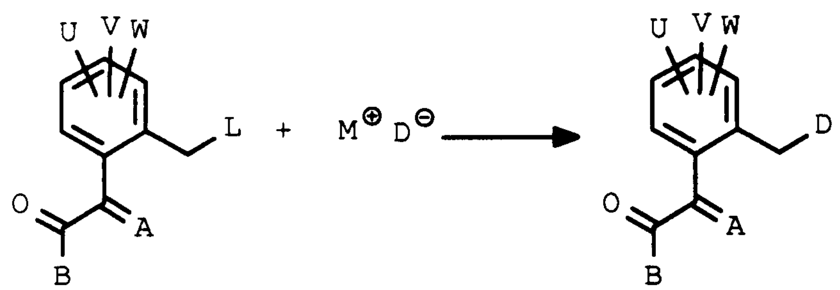

6 10 1

Im Einzelnen erhält man die neuen Verbindungen wie folgt:

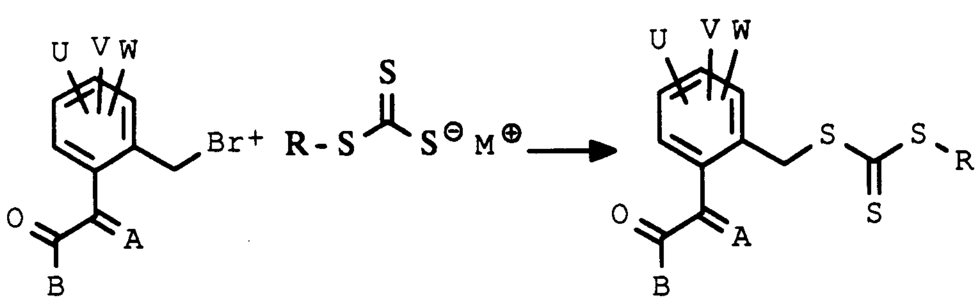

11 12 13

Trithiokohlensäureester 13 erhält man z. B. durch Umsetzung der Metallsalze 12 mit den Benzylbromiden 11 analog zu bekannten Verfahren (vgl. z. B. Houben-Weyl, Bd. E 4, S 451ff (1983)).

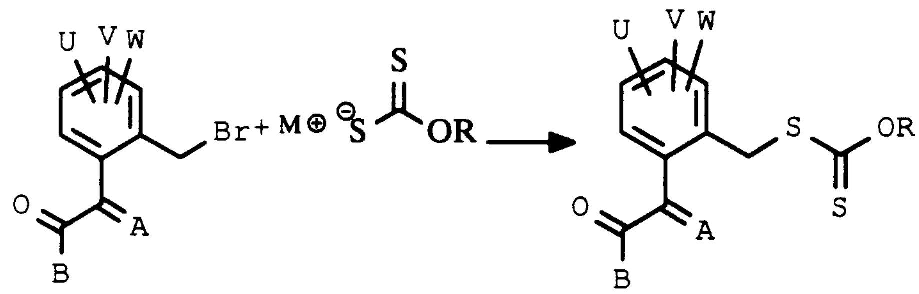

11 14 15

Die Dithiocarbonsäureester 15 lassen sich z. B. durch Umsetzung der Benzylbromide 11 mit den Metallsalzen 14 in Analogie zu bekannten Verfahren darstellen (vgl. z. B. R. Degani, Synthesis, 365 (1975)).

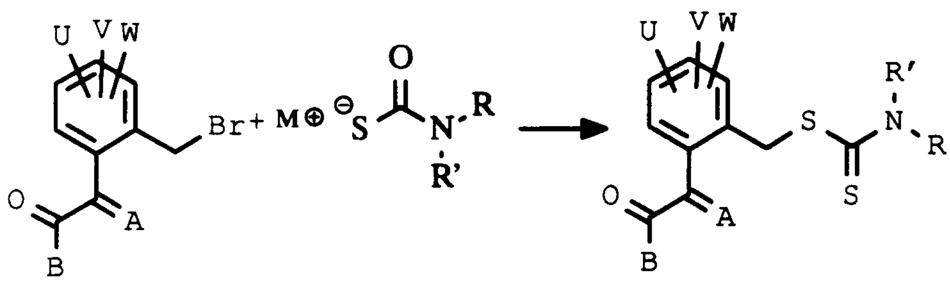

11 16 17

Dithiocarbamate vom Typ 17 erhält man z. B. bei der Reaktion der Benzylbromide 11 mit den Verbindungen 16 analog zu bekannten Verfahren (vgl. z. B. Houben-Weyl, Bd. E 4, S 299 f (1983)).

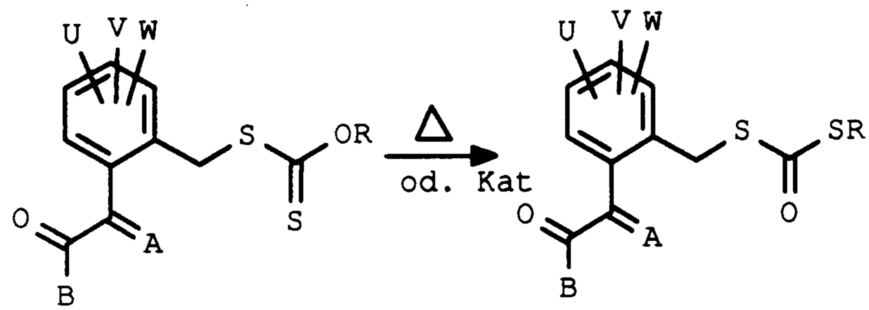


15 18

Die Dithiolkohlensäure-Derivate 18 lassen sich durch thermische oder säurekatalysierte Umlagerung aus den Dithiokohlensäure-Derivaten 15 herstellen (vgl. z. B. Houben-Weyl, Bd E 4, S 133ff (1983)).

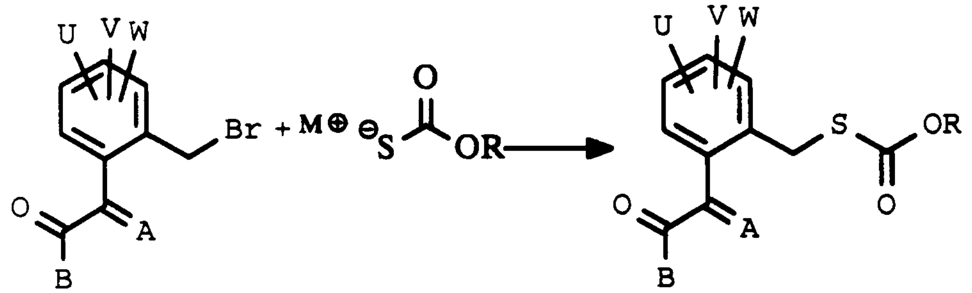

11 19 20

Die Darstellung der Thiokohlensäureester 20 erfolgt z. B. durch Umsetzung der Benzylbromide 11 mit den Thiocarbonaten 19 in Analogie zu bekanntne Verfahren (vgl. z. B. Houben-Weyl Bd E 4, S108f (1983)).

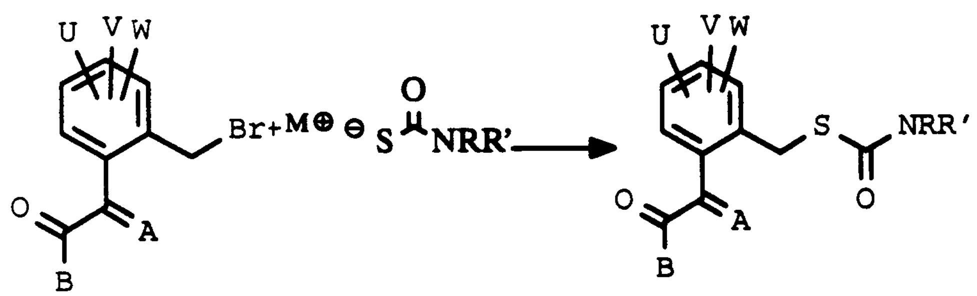

11 21 22

Thiocarbamidsäuren 22 sind durch Umsetzung der Benzylbromide 11 mit den Metallsalzen 21 analog bekannter Verfahren zugänglich (vgl. z. B. Houben-Weyl, Bd E 4, S 299 (1983)).

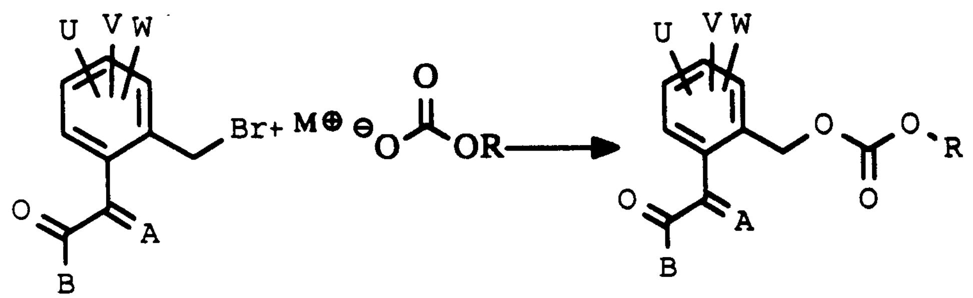

11 23 24

Kohlensäurediester 24 erhält man in Analogie zu bekannten Verfahren durch Umsetzung der Benzylbromide 11 mit Carbonaten 23 (vgl. z. B. V. F. Pozdner, Zh. Org. Khim, 12, 1407 (1976)).

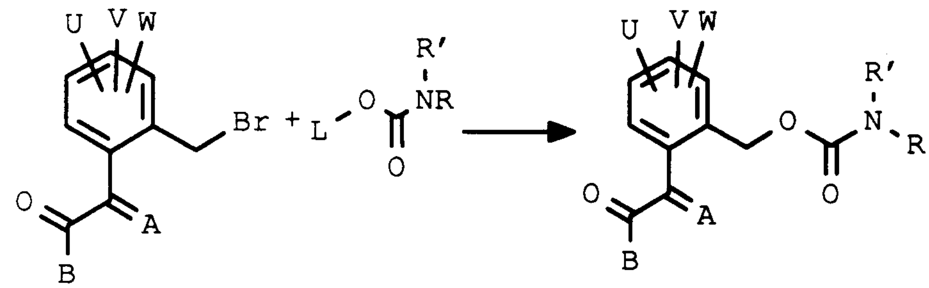

11 25 26

Die Carbamate 26 sind in Analogie zu bekannten Verfahren aus den Benzylbromiden 11 und den Phosphacarbamaten 25 zugänglich (vgl. z.B. M. Aresta, JOC, 53, 4153 (1988)).

Die Kohlensäuremetallsalze 12, 14, 16, 19, 21, 23 und 25 sind bekannt oder lassen sich nach literaturbekannten Verfahren herstellen (vgl. hierzu Houben-Weyl, Bd E 4).

Üblicherweise ist bei den vorbeschriebenen Herstellungsverfahren der Rest B = Alkoxy und U, V, W = H.

Die Verbindungen mit B = OH (28) lassen sich nach literaturbekannten Methoden (Organikum 16. Auflage, S. 415, 622) aus den Verbindungen der allgemeinen Formel I, in der B = O-Alkyl (27) ist, herstellen (s. Schema 2):

Schema 2:

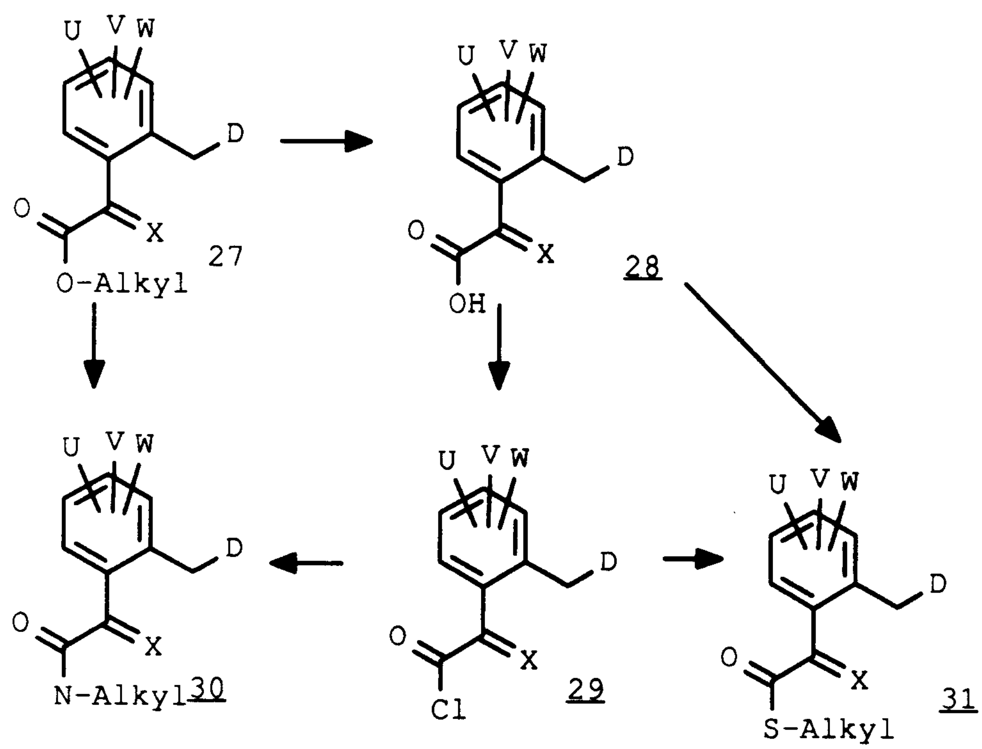

27 28 30 29 31

Aus den so erhaltenen Carbonsäuren 28 lassen sich in an sich bekannter Weise die Säurechloride 29 herstellen (vgl. Organikum 16. Auflage, S. 423f (1985)). Die Umwandlung von 29 in die Amide 30 erfolgt analog zu Organikum 16. Auflage S. 412 (1985).

Die Thiolester 31 erhält man aus den Säurechloriden 29 (analog zu Houben-Weyl Bd. 8, S. 464ff (1952)).

Alternativ können die Thiolester 31 auch aus den Säuren 28 hergestellt werden (analog zu Houben-Weyl Bd. E5, S. 855f (1985)).

Die Herstellung der Amide 30 kann auch nach literaturbekannten Verfahren aus dem Ester 27 erfolgen.

Die Herstellung der Verbindung der allgemeinen Formeln 2 und 7 mit ortho-Methylsubstitution am Aromaten ist bekannt (B = O-Alkyl, U, V, W = H; s. EP 178 826, EP 260 832).

Die beschriebenen Umsetzungen können z. B. in einem inerten Lösungs- oder Verdünnungsmittel (z. B. Aceton, Acetonitril, Dimethylformamid, Dimethylsulfoxid, N-Methylpyrrolidon) unter Verwendung einer Base (z. B. Natriumcarbonat, Kaliumcarbonat, Natriumhydroxid, Kaliumhydroxid, Natriumhydrid, Natriummethylat) durchgeführt werden.

Die Umsetzung kann auch im Zweiphasensystem (z. B. Dichlormethan, Wasser) unter Zusatz eines geeigneten Phasentransferkatalysators (z. B. Cetyltrimethylammoniumchlorid, Benzyltriethylammoniumchlorid) durchgeführt werden.

Beispiele

Beispiel 1

2-Methoxyimino-2-{2-[(methyl-4-methylphenyl-thiocarbamoylsulfonyl)-methyl]-phenyl}-essigsäure-methylester (Verbindung Nr. 157 in Tabelle 2)

Zu einer Lösung von 3,6 g (17,5 mmol) Natrium N-Methyl, N-4-methylphenyldithiocarbamat und 5 g (17,5 mmol) 2-Methoximino-2-(2'-brommethyl)phenylessigsäuremethylester in 50ml Aceton gibt man eine Spatelspitze Kaliumjodid und rührt 15 Stunden bei Raumtemperatur. Anschließend saugt man ab und engt die Mutterlauge ein. Der Rückstand wird in Ether aufgerührt und abgesaugt. Es verbleiben 4,6 g (65 %) der Verbindung Nr. 157, Tab.2 als farbloser Feststoff.

m. p.: 109 - 113 °C

$^1$H-NMR ($CDCl_3$/TMS) : $\delta$ = 2,35; 3,72; 3,77; 3,97 (jeweils s, 3H, $CH_3$); 4,30 (br., 2H, $CH_2$); 7,07 - 7,47 ppm (m, 8H, Aryl-H).

Beispiel 2

Darstellung von Verbindung 157, Tabelle 4

1 g (2,5 mmol) der in Beispiel 1 dargestellten Verbindung wird mit 50 ml 40 %iger N-Methylamin-Lösung 2 Stunden lang auf 40 °C erhitzt. Man extrahiert anschließend dreimal mit Ether, trocknet die vereinigten organischen Phasen und engt ein. Es verbleibt 1 g der N-Methylamid-Verbindung (Nr. 157, Tab 4) als farbloser Feststoff.

$^1$H-NMR ($CDCl_3$/TMS): $\delta$ = 2,35; 3,72; 3,88 (jeweils s, 3H, $CH_3$); 2,89 (d, 3H, $NHCH_3$); 4,32 (s, 2H, $CH_2$); 6,67 (br, 1H, NH); 7,05 - 7,43 ppm (m, 8H, Aryl-H).

Beispiel 3

Darstellung von Verbindung Nr. 157 Tab. 1

Zu einer Lösung von 1,4 g (7 mmol) Natrium N-Methyl-, N-4-methylphenyldithiocarbamat und 2 g (7 mmol) $\alpha$-(2-Brom-methyl-phenyl)-$\beta$-methoxyacrylsäure-methylester in 50 ml Aceton gibt man eine Spatelspitze Kaliumjodid und rührt 15 Stunden bei Raumtemperatur. Anschließend saugt man ab und engt die Mutterlauge ein. Der verbleibende Rückstand wird an Kieselgel mit Hexan/Methyl-tert.-butylether chromatographiert. Man erhält 1,2 g (43 %) der Verbindung Nr. 157, Tab. 1 als farblosen Feststoff.

m. p.: 98 - 99 °C.

$^1$H-NMR ($CDCl_3$/TMS): $\delta$ = 2,33; 3,57; 3,74; 3,74 (jeweils s, 3H, $CH_3$); 4,35 (s, 2H, $CH_2$); 7,05 - 7,43 (m, 8H, Aryl-H); 7,51 (s, 1H, =CH).

Beispiel 4

Darstellung von 2-Hydroxymethyl-$\alpha$-methoxyimino-phenylessigsäure-monomethylamid

Eine Mischung aus 57 g (200 mmol) 2-Methoximino-2-(2-brommethyl)phenylessigsäuremethylester, 29 g (300 mmol) Kaliumacetat und einer Spatelspitze Kaliumjodid werden in 300 ml Dimethylformamid 30 min auf 90 °C erhitzt. Nach Abkühlen wird das Reaktionsgemisch in Eiswasser eingerührt. Der Niederschlag wird abgesaugt, mit Wasser gewaschen und getrocknet; farblose Kristalle, Smp. 70-72 °C, Ausbeute 50 g (94 % d. Th.).

Die so erhaltene Verbindung (50 g/188 mmol) wird in 500 ml THF gelöst, mit 100 ml (1,12 mol) 40 %iger wäßriger Methylaminlösung versetzt und die Mischung 22 h bei RT gerührt. Man engt ein, nimmt

den Rückstand in Essigester auf, wäscht zweimal mit gesättigter Kochsalzlösung, trocknet über Magnesiumsulfat und engt ein. Der ölige Rückstand wird beim Stehen allmählich fest; beim Verreiben mit Diisopropylether/Petrolether kristallisiert das Produkt. Beigefarbene Kristalle, Smp. 99-103 °C, Ausbeute 32 g (82 % d. Th.).

$^1$H-NMR ($CDCl_3$/δ-Skala): 2,90 (d, 3H, $NCH_3$); 3,48 (br t, 1 H, OH); 2, 3H, $OCH_3$); 4,40 (d, 2H, $CH_2O$); 7.05 (br, 1H, NH); 7,14 (dd, 1H) und 7,2-7,6 (m, 3H, Aromatenprotonen)
IR (KBr, ~ in $cm^{-1}$): 3384, 3306, 3281, 1647, 1035

Beispiel 5

Darstellung von 2-(4-Fluoranilinocarbonyloxymethyl)-α-methoxyimino-phenylessigsäuremonomethylamid (Verb. 293, Tab. 4)

a) 2,2 g (10 mmol) der in Beispiel 4 erhaltenen Verbindung, 1,4 g (10 mmol) 4-Fluorphenylisocyanat und eine Spatelspitze 4-N,N-Dimethylaminopyridin werden in 150 ml Toluol 4 h lang bei 100 °C gerührt. Beim Abkühlen fällt das Produkt aus; es wird abgesaugt, mit Toluol gewaschen und getrocknet. Farblose Kristalle, Smp. 208-209 °C, Ausbeute 2,4 g (67 % d. Th.). Die Verbindung liegt als Gemisch aus zwei Rotameren im Mengenverhältnis 3:1 vor.

$^1$H-NMR ($CDCl_3$/δ-Skala): 2,81 und 2,90 (2d, zus. 3H, $NCH_3$ in beide Rotameren); 3,96 und 4,01 (2s, zus. 3H, $OCH_3$); 5,08 und 5,36 (2s, zus. 2H, $OCH_2$); 6,7-7,5 (mehrere Multipletts, insg. 10H, Aromatenprotonen und beide NH).

b) 2,2 g (10 mmol) der in Beispiel 4 erhaltenen Verbindung wurden in 200 ml Toluol suspendiert auf 60 °C erwärmt und zuerst 1,74 g (10 mmol) Chlorameisensäure-4-fluorphenyl-ester dann 1,5 ml (1,1 g/10 mmol) Triethylamin zugetropft. Nach 2 h Rühren läßt man abkühlen, saugt den Niederschlag ab, wäscht das Filtrat mehrmals mit Wasser, trocknet über Magnesiumsulfat und engt ein. Man erhält 3,0 g (83 % d. Th.) der Verbindung (Nr. 293; Tab. 4).

Die Herstellung der in den folgenden Tabellen aufgeführten Verbindungen erfolgt entsprechend den beschriebenen Beispielen.

Tabelle 1

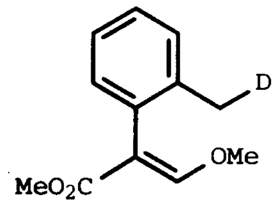

| Nr. | D | R' | R | phys. Daten[a)] |
|---|---|---|---|---|
| 1 | SC(S)NR'R | $CH_3$ | Methyl | |
| 2 | SC(S)NR'R | $CH_3$ | Ethyl | |
| 3 | SC(S)NR'R | $CH_3$ | n-Propyl | |
| 4 | SC(S)NR'R | $CH_3$ | tert.-Butyl | |
| 5 | SC(S)NR'R | $CH_3$ | Cyclopropyl | |
| 6 | SC(S)NR'R | $CH_3$ | Cyclohexyl | 3,70, 3,85 (3H), 4,50 (2H), 7,63 (1H) |
| 7 | SC(S)NR'R | $CH_3$ | Methoxymethyl | |
| 8 | SC(S)NR'R | $CH_3$ | Phenoxymethyl | |
| 9 | SC(S)NR'R | $CH_3$ | Methylthiomethyl | |
| 10 | SC(S)NR'R | $CH_3$ | Phenyl | 3,55; 3,71; 3,76 (3 x 3H); 4,37 (2H); 7,03-7,40 (9H); 7,51 (1H) |
| 11 | SC(S)NR'R | $CH_3$ | 2-Fluorphenyl | |

| Nr. | D | R' | R | phys. Daten[a)] |
|---|---|---|---|---|
| 12 | SC(S)NR'R | $CH_3$ | 3-Fluorphenyl | 102 - 106°C |
| 13 | SC(S)NR'R | $CH_3$ | 4-Fluorphenyl | 3,57; 3,73; 3,74 (3 x 3H); 4,35 (2H); 7,04-7,43 (8H), 7,51 (1H) |
| 14 | SC(S)NR'R | $CH_3$ | Pentafluorphenyl | |
| 15 | SC(S)NR'R | $CH_3$ | 2-Chlorphenyl | |
| 16 | SC(S)NR'R | $CH_3$ | 3-Chlorphenyl | 3,57; 3,72; 3,74 (3 x 3H); 4,34 (2H); 7,04-7,43 (8H); 7,51 (1H) |
| 17 | SC(S)NR'R | $CH_3$ | 4-Chlorphenyl | 3,00, 3,71, 3,86 (3H), 4,35 (2H) |
| 18 | SC(S)NR'R | $CH_3$ | Pentachlorphenyl | |
| 19 | SC(S)NR'R | $CH_3$ | 2,3-Dichlorphenyl | |
| 20 | SC(S)NR'R | $CH_3$ | 2,,4-Dichlorphenyl | |
| 21 | SC(S)NR'R | $CH_3$ | 2,5-Dichlorphenyl | |
| 22 | SC(S)NR'R | $CH_3$ | 2,6-Dichlorphenyl | |
| 23 | SC(S)NR'R | $CH_3$ | 3,4-Dichlorphenyl | |
| 24 | SC(S)NR'R | $CH_3$ | 3,5-Dichlorphenyl | |
| 25 | SC(S)NR'R | $CH_3$ | 2,3,4-Trichlorphenyl | |
| 26 | SC(S)NR'R | $CH_3$ | 2,3,5-Trichlorphenyl | |
| 27 | SC(S)NR'R | $CH_3$ | 2,3,6-Trichlorphenyl | |
| 28 | SC(S)NR'R | $CH_3$ | 2,4,5-Trichlorphenyl | |
| 29 | SC(S)NR'R | $CH_3$ | 2,4,6-Trichlorphenyl | |

| Nr. | D | R' | R | phys. Daten[a)] |
|---|---|---|---|---|
| 30 | SC(S)NR'R | $CH_3$ | 3,4,5-Trichlorphenyl | |
| 31 | SC(S)NR'R | $CH_3$ | 2,3,4,6-Tetrachlorphenyl | |
| 32 | SC(S)NR'R | $CH_3$ | 2,3,5,6-Tetrachlorphenyl | |
| 33 | SC(S)NR'R | $CH_3$ | 2-Bromphenyl | |
| 34 | SC(S)NR'R | $CH_3$ | 3-Bromphenyl | |
| 35 | SC(S)NR'R | $CH_3$ | 4-Bromphenyl | |
| 36 | SC(S)NR'R | $CH_3$ | 2,4-Dibromphenyl | |
| 37 | SC(S)NR'R | $CH_3$ | 3-Brom-4-Fluorphenyl | |
| 38 | SC(S)NR'R | $CH_3$ | 3-Brom-4-Methoxyphenyl | |
| 39 | SC(S)NR'R | $CH_3$ | 2-Jodphenyl | |
| 40 | SC(S)NR'R | $CH_3$ | 3-Jodphenyl | |
| 41 | SC(S)NR'R | $CH_3$ | 4-Jodphenyl | |
| 42 | SC(S)NR'R | $CH_3$ | 2-Chlor-4-Fluorphenyl | |
| 43 | SC(S)NR'R | $CH_3$ | 2-Chlor-5-Fluorphenyl | |
| 44 | SC(S)NR'R | $CH_3$ | 2-Chlor-6-Fluorphenyl | |
| 45 | SC(S)NR'R | $CH_3$ | 2-Chlor-4-Bromphenyl | |
| 46 | SC(S)NR'R | $CH_3$ | 2-Brom-4-Chlorphenyl | |
| 47 | SC(S)NR'R | $CH_3$ | 2-Brom-4-Fluorphenyl | |
| 48 | SC(S)NR'R | $CH_3$ | 3-Brom-4-Fluorphenyl | |

| Nr. | D | R' | R | phys. Daten[a)] |
|---|---|---|---|---|
| 49 | SC(S)NR'R | $CH_3$ | 3-Chlor-4-Fluorphenyl | |
| 50 | SC(S)NR'R | $CH_3$ | 3-Fluor-4-Chlorphenyl | |
| 51 | SC(S)NR'R | $CH_3$ | 2-Cyanophenyl | |
| 52 | SC(S)NR'R | $CH_3$ | 3-Cyanophenyl | |
| 53 | SC(S)NR'R | $CH_3$ | 4-Cyanophenyl | |
| 54 | SC(S)NR'R | $CH_3$ | 2-Nitrophenyl | |
| 55 | SC(S)NR'R | $CH_3$ | 3-Nitrophenyl | |
| 56 | SC(S)NR'R | $CH_3$ | 4-Nitrophenyl | |
| 57 | SC(S)NR'R | $CH_3$ | 2-Methylphenyl | 2,17; 3,54; 3,69; 3,71; (4 x 3H); 4,35 (2H); 7,03-7,41 (8H); 7,51 (1H) |
| 58 | SC(S)NR'R | $CH_3$ | 3-Methylphenyl | 2,34; 3,65; 3,72; 3,74; (4 x 3H); 4,36 (2H); 7,00-7,51 (8H); 7,43 (1H) |
| 59 | SC(S)NR'R | $CH_3$ | 4-Methylphenyl | 98 - 99°C |
| 60 | SC(S)NR'R | $CH_3$ | 2,4-Dimethylphenyl | |
| 61 | SC(S)NR'R | $CH_3$ | 2,6-Dimethylphenyl | 120 - 121°C |
| 62 | SC(S)NR'R | $CH_3$ | 3,4-Dimethylphenyl | |
| 63 | SC(S)NR'R | $CH_3$ | 3,5-Dimethylphenyl | |
| 64 | SC(S)NR'R | $CH_3$ | 2,3,4-Trimethylphenyl | |
| 65 | SC(S)NR'R | $CH_3$ | 2,3,5-Trimethylphenyl | |
| 66 | SC(S)NR'R | $CH_3$ | 2,3,6-Trimethylphenyl | |

| Nr. | D | R' | R | phys. Daten[a)] |
|---|---|---|---|---|
| 67 | SC(S)NR'R | $CH_3$ | 2,4,5-Trimethylphenyl | |
| 68 | SC(S)NR'R | $CH_3$ | 2,4,6-Trimethylphenyl | |
| 69 | SC(S)NR'R | $CH_3$ | 3,4,5-Trimethylphenyl | |
| 70 | SC(S)NR'R | $CH_3$ | Pentamethylphenyl | |
| 71 | SC(S)NR'R | $CH_3$ | 2-Ethylphenyl | |
| 72 | SC(S)NR'R | $CH_3$ | 3-Ethylphenyl | |
| 73 | SC(S)NR'R | $CH_3$ | 4-Ethylphenyl | |
| 74 | SC(S)NR'R | $CH_3$ | 3,5-Diethylphenyl | |
| 75 | SC(S)NR'R | $CH_3$ | 2-n-Propylphenyl | |
| 76 | SC(S)NR'R | $CH_3$ | 3-n-Propylphenyl | |
| 77 | SC(S)NR'R | $CH_3$ | 4-n-Propylphenyl | |
| 78 | SC(S)NR'R | $CH_3$ | 2-iso-Propylphenyl | |
| 79 | SC(S)NR'R | $CH_3$ | 3-iso-Propylphenyl | |
| 80 | SC(S)NR'R | $CH_3$ | 4-iso-Propylphenyl | |
| 81 | SC(S)NR'R | $CH_3$ | 2,4-Di-iso-Propylphenyl | |
| 82 | SC(S)NR'R | $CH_3$ | 3,5-Di-iso-Propylphenyl | |
| 83 | SC(S)NR'R | $CH_3$ | 4-n-Butyphenyl | |
| 84 | SC(S)NR'R | $CH_3$ | 4-sec.-Butylphenyl | |
| 85 | SC(S)NR'R | $CH_3$ | 4-iso-Butylphenyl | |

| Nr. | D | R' | R | phys. Daten[a)] |
|---|---|---|---|---|
| 86 | SC(S)NR'R | $CH_3$ | 4-tert.-Butylphenyl | |
| 87 | SC(S)NR'R | $CH_3$ | 3-tert.-Butylphenyl | |
| 88 | SC(S)NR'R | $CH_3$ | 2-tert.-Butylphenyl | |
| 89 | SC(S)NR'R | $CH_3$ | 2,4-Di-tert.-Butylphenyl | |
| 90 | SC(S)NR'R | $CH_3$ | 3,5-Di-tert.-Butylphenyl | |
| 91 | SC(S)NR'R | $CH_3$ | 4-n-Hexylphenyl | |
| 92 | SC(S)NR'R | $CH_3$ | 4-n-Dodecylphenyl | |
| 93 | SC(S)NR'R | $CH_3$ | 2-Methyl-4-tert.-Butyl-phenyl | |
| 94 | SC(S)NR'R | $CH_3$ | 2-Methyl-6-tert.-Butyl-phenyl | |
| 95 | SC(S)NR'R | $CH_3$ | 2-Methyl-4-iso-Propylphenyl | |
| 96 | SC(S)NR'R | $CH_3$ | 2-Methyl-4-Cyclohexylphenyl | |
| 97 | SC(S)NR'R | $CH_3$ | 2-Methyl-4-Phenylphenyl | |
| 98 | SC(S)NR'R | $CH_3$ | 2-Methyl-4-Benzylphenyl | |
| 99 | SC(S)NR'R | $CH_3$ | 2-Methyl-4-Phenoxyphenyl | |
| 100 | SC(S)NR'R | $CH_3$ | 2-Methyl-4-Benzyloxyphenyl | |
| 101 | SC(S)NR'R | $CH_3$ | 2-Methyl-3-Chlorphenyl | |
| 102 | SC(S)NR'R | $CH_3$ | 2-Methyl-4-Chlorphenyl | |
| 103 | SC(S)NR'R | $CH_3$ | 2-Methyl-5-Chlorphenyl | |
| 104 | SC(S)NR'R | $CH_3$ | 2-Methyl-6-Chlorphenyl | |

| Nr. | D | R' | R | phys. Daten[a)] |
|---|---|---|---|---|
| 105 | SC(S)NR'R | $CH_3$ | 2-Methyl-4-Fluorphenyl | |
| 106 | SC(S)NR'R | $CH_3$ | 2-Methyl-3-Bromphenyl | |
| 107 | SC(S)NR'R | $CH_3$ | 2-Methyl-4-Bromphenyl | |
| 108 | SC(S)NR'R | $CH_3$ | 2-Methyl-3-Methoxyphenyl | |
| 109 | SC(S)NR'R | $CH_3$ | 2-Methyl-4-Methoxyphenyl | |
| 110 | SC(S)NR'R | $CH_3$ | 2-Methyl-5-Methoxyphenyl | |
| 111 | SC(S)NR'R | $CH_3$ | 2-Methyl-6-Methoxyphenyl | |
| 112 | SC(S)NR'R | $CH_3$ | 2-Methyl-4-iso-Propoxy-phenyl | |
| 113 | SC(S)NR'R | $CH_3$ | 2-Methyl-2,5-Dimethoxy-phenyl | |
| 114 | SC(S)NR'R | $CH_3$ | 2-Methoxyphenyl | |
| 115 | SC(S)NR'R | $CH_3$ | 3-Methoxyphenyl | |
| 116 | SC(S)NR'R | $CH_3$ | 4-Methoxyphenyl | |
| 117 | SC(S)NR'R | $CH_3$ | 2,3-Dimethoxyphenyl | |
| 118 | SC(S)NR'R | $CH_3$ | 2,4-Dimethoxyphenyl | |
| 119 | SC(S)NR'R | $CH_3$ | 2,5-Dimethoxyphenyl | |
| 120 | SC(S)NR'R | $CH_3$ | 2,6-Dimethoxyphenyl | |
| 121 | SC(S)NR'R | $CH_3$ | 3,4-Dimethoxyphenyl | |
| 122 | SC(S)NR'R | $CH_3$ | 3,5-Dimethoxyphenyl | |
| 123 | SC(S)NR'R | $CH_3$ | 3,6-Dimethoxyphenyl | |

| Nr. | D | R' | R | phys. Daten[a)] |
|---|---|---|---|---|
| 124 | SC(S)NR'R | $CH_3$ | 2,3,4-Trimethoxyphenyl | |
| 125 | SC(S)NR'R | $CH_3$ | 2,3,5-Trimethoxyphenyl | |
| 126 | SC(S)NR'R | $CH_3$ | 2,3,6-Trimethoxyphenyl | |
| 127 | SC(S)NR'R | $CH_3$ | 2,4,5-Trichlorphenyl | |
| 128 | SC(S)NR'R | $CH_3$ | 2,4,6-Trichloryphenyl | |
| 129 | SC(S)NR'R | $CH_3$ | 2,3,4,6-Tetrachlorphenyl | |
| 130 | SC(S)NR'R | $CH_3$ | 2,3,5,6-Tetrachlorphenyl | |
| 131 | SC(S)NR'R | $CH_3$ | 2-Bromphenyl | |
| 132 | SC(S)NR'R | $CH_3$ | 3-Bromphenyl | |
| 133 | SC(S)NR'R | $CH_3$ | 4-Bromphenyl | |
| 134 | SC(S)NR'R | $CH_3$ | 2,4-Dibromphenyl | |
| 135 | SC(S)NR'R | $CH_3$ | 3-Brom-4-Fluorphenyl | |
| 136 | SC(S)NR'R | $CH_3$ | 3-Brom-4-Methoxyphenyl | |
| 137 | SC(S)NR'R | $CH_3$ | 2-Jodphenyl | |
| 138 | SC(S)NR'R | $CH_3$ | 3-Jodphenyl | |
| 139 | SC(S)NR'R | $CH_3$ | 4-Jodphenyl | |
| 140 | SC(S)NR'R | $CH_3$ | 2-Chlor-4-Fluorphenyl | |
| 141 | SC(S)NR'R | $CH_3$ | 2-Chlor-5-Fluorphenyl | |
| 142 | SC(S)NR'R | $CH_3$ | 2-Chlor-6-Fluorphenyl | |

| Nr. | D | R' | R | phys. Daten[a]) |
|---|---|---|---|---|
| 143 | SC(S)NR'R | $CH_3$ | 2-Chlor-4-Bromphenyl | |
| 144 | SC(S)NR'R | $CH_3$ | 2-Brom-4-Chlorphenyl | |
| 145 | SC(S)NR'R | $CH_3$ | 2-Brom-4-Fluorphenyl | |
| 146 | SC(S)NR'R | $CH_3$ | 3-Brom-4-Chlorphenyl | |
| 147 | SC(S)NR'R | $CH_3$ | 3-Chlor-4-Fluorphenyl | |
| 148 | SC(S)NR'R | $CH_3$ | 3-Fluor-4-Chlorphenyl | |
| 149 | SC(S)NR'R | $CH_3$ | 2-Cyanophenyl | |
| 150 | SC(S)NR'R | $CH_3$ | 3-Cyanophenyl | |
| 151 | SC(S)NR'R | $CH_3$ | 4-Cyanophenyl | |
| 152 | SC(S)NR'R | $CH_3$ | 2-Nitrophenyl | |
| 153 | SC(S)NR'R | $CH_3$ | 3-Nitrophenyl | |
| 154 | SC(S)NR'R | $CH_3$ | 4-Nitrophenyl | |
| 155 | SC(S)NR'R | $CH_3$ | 2-Methylphenyl | |
| 156 | SC(S)NR'R | $CH_3$ | 3-Methylphenyl | |
| 157 | SC(S)NR'R | $CH_3$ | 4-Methylphenyl | |
| 158 | SC(S)NR'R | $CH_3$ | 2,4-Dimethylphenyl | |
| 159 | SC(S)NR'R | $CH_3$ | 2,6-Dimethylphenyl | |
| 160 | SC(S)NR'R | $CH_3$ | 3,4-Dimethylphenyl | |
| 161 | SC(S)NR'R | $CH_3$ | 3,5-Dimethylphenyl | |

| Nr. | D | R' | R | phys. Daten[a)] |
|---|---|---|---|---|
| 162 | SC(S)NR'R | $CH_3$ | 2,3,4-Trimethylphenyl | |
| 163 | SC(S)NR'R | $CH_3$ | 2,3,5-Trimethylphenyl | |
| 164 | SC(S)NR'R | $CH_3$ | 2,3,6-Trimethylphenyl | |
| 165 | SC(S)NR'R | $CH_3$ | 2,4,5-Trimethylphenyl | |
| 166 | SC(S)NR'R | $CH_3$ | 2,4,6-Trimethylphenyl | |
| 167 | SC(S)NR'R | $CH_3$ | 2,4,5-Trimethylphenyl | |
| 168 | SC(S)NR'R | $CH_3$ | Pentamethylphenyl | |
| 169 | SC(S)NR'R | $CH_3$ | 2-Ethylphenyl | |
| 170 | SC(S)NR'R | $CH_3$ | 3-Ethylphenyl | |
| 171 | SC(S)NR'R | $CH_3$ | 4-Ethylphenyl | |
| 172 | SC(S)NR'R | $CH_3$ | 3,5-Diethylphenyl | |
| 173 | SC(S)NR'R | $CH_3$ | 2-n-Propylphenyl | |
| 174 | SC(S)NR'R | $CH_3$ | 3-n-Propylphenyl | |
| 175 | SC(S)NR'R | $CH_3$ | 4-n-Propylphenyl | |
| 176 | SC(S)NR'R | $CH_3$ | 2,4,5-Trimethoxyphenyl | |
| 177 | SC(S)NR'R | $CH_3$ | 2,4,6-Trimethoxyphenyl | |
| 178 | SC(S)NR'R | $CH_3$ | 3,4,5-Trimethoxyphenyl | |
| 179 | SC(S)NR'R | $CH_3$ | 2-Ethoxyphenyl | |
| 180 | SC(S)NR'R | $CH_3$ | 3-Ethoxyphenyl | |

| Nr. | D | R' | R | phys. Daten[a)] |
|---|---|---|---|---|
| 181 | SC(S)NR'R | $CH_3$ | 4-Ethoxyphenyl | |
| 182 | SC(S)NR'R | $CH_3$ | 2-iso-Propoxyphenyl | |
| 183 | SC(S)NR'R | $CH_3$ | 3-iso-Propoxyphenyl | |
| 184 | SC(S)NR'R | $CH_3$ | 4-iso-Propoxyphenyl | |
| 185 | SC(S)NR'R | $CH_3$ | 3-tert.-Butoxyphenyl | |
| 186 | SC(S)NR'R | $CH_3$ | 4-tert.-Butoxyphenyl | |
| 187 | SC(S)NR'R | $CH_3$ | 2-Trifluormethoxyphenyl | |
| 188 | SC(S)NR'R | $CH_3$ | 3-Trifluormethoxyphenyl | |
| 189 | SC(S)NR'R | $CH_3$ | 4-Trifluormethoxyphenyl | |
| 190 | SC(S)NR'R | $CH_3$ | 3-(1',1',2',2'-Tetra-fluor)-ethoxyphenyl | |
| 191 | SC(S)NR'R | $CH_3$ | 4-(1',1',2',2'-Tetra-fluor)-ethoxyphenyl | |
| 192 | SC(S)NR'R | $CH_3$ | 2-Chlormethylphenyl | |
| 193 | SC(S)NR'R | $CH_3$ | 3-Chlormethylphenyl | |
| 194 | SC(S)NR'R | $CH_3$ | 4-Chlormethylphenyl | |
| 195 | SC(S)NR'R | $CH_3$ | 2-Trifluormethylphenyl | |
| 196 | SC(S)NR'R | $CH_3$ | 3-Trifluormethylphenyl | |
| 197 | SC(S)NR'R | $CH_3$ | 4-Trifluormethylphenyl | |
| 198 | SC(S)NR'R | $CH_3$ | 2-(Methoxyiminomethyl)-phenyl | |

| Nr. | D | R' | R | phys. Daten[a)] |
|---|---|---|---|---|
| 199 | SC(S)NR'R | $CH_3$ | 3-(Methoxyiminomethyl)-phenyl | |
| 200 | SC(S)NR'R | $CH_3$ | 4-(Methoxyiminomethyl)-phenyl | |
| 201 | SC(S)NR'R | $CH_3$ | 2-(Ethoxyiminomethyl)-phenyl | |
| 202 | SC(S)NR'R | $CH_3$ | 3-(Ethoxyiminomethyl)-phenyl | |
| 203 | SC(S)NR'R | $CH_3$ | 4-(Ethoxyiminomethyl)-phenyl | |
| 204 | SC(S)NR'R | $CH_3$ | 2-Methyl-4-Methoximinoethyl-phenyl | |
| 205 | SC(S)NR'R | $CH_3$ | 2-Methyl-4-Methoximinomethyl-phenyl | |
| 206 | SC(S)NR'R | $CH_3$ | 2,6-Dimethyl-4-Methoximino-methyl-phenyl | |
| 207 | SC(S)NR'R | $CH_3$ | 2,6-Dimethyl-4-Methoximino-ethyl-phenyl | |
| 208 | SC(S)NR'R | $CH_3$ | 2-Phenylphenyl | |
| 209 | SC(S)NR'R | $CH_3$ | 3-Phenylphenyl | |
| 210 | SC(S)NR'R | $CH_3$ | 4-Phenylphenyl | |
| 211 | SC(S)NR'R | $CH_3$ | 2-Phenoxyphenyl | |
| 212 | SC(S)NR'R | $CH_3$ | 3-Phenoxyphenyl | |
| 213 | SC(S)NR'R | $CH_3$ | 4-Phenoxyphenyl | |
| 214 | SC(S)NR'R | $CH_3$ | 2-Benzyloxyphenyl | |
| 215 | SC(S)NR'R | $CH_3$ | 3-Benzyloxyphenyl | |

| Nr. | D | R' | R | phys. Daten[a)] |
|---|---|---|---|---|
| 216 | SC(S)NR'R | $CH_3$ | 4-Benzyloxyphenyl | |
| 217 | SC(S)NR'R | $CH_3$ | 4-(Imidazol-1'-yl)phenyl | |
| 218 | SC(S)NR'R | $CH_3$ | 4-(Piperazin-1'-yl)phenyl | |
| 219 | SC(S)NR'R | $CH_3$ | 4-(Morpholin-1'-yl)phenyl | |
| 220 | SC(S)NR'R | $CH_3$ | 4-(Piperidin-1'-yl)phenyl | |
| 221 | SC(S)NR'R | $CH_3$ | 4-(Pyridyl-2'-oxy)phenyl | |
| 222 | SC(S)NR'R | $CH_3$ | 2-Cyclopropylphenyl | |
| 223 | SC(S)NR'R | $CH_3$ | 3-Cyclopropylphenyl | |
| 224 | SC(S)NR'R | $CH_3$ | 4-Cyclopropylphenyl | |
| 225 | SC(S)NR'R | $CH_3$ | 3-Cyclohexylphenyl | |
| 226 | SC(S)NR'R | $CH_3$ | 4-Cyclohexylphenyl | |
| 227 | SC(S)NR'R | $CH_3$ | 4-Oxiranylphenyl | |
| 228 | SC(S)NR'R | $CH_3$ | 4-(1',3'-Dioxan-2'-yl)-phenyl | |
| 229 | SC(S)NR'R | $CH_3$ | 4-(Tetrahydropyran-2-yl-oxy)-phenyl | |
| 230 | SC(S)NR'R | $CH_3$ | 1-Naphthyl | |
| 231 | SC(S)NR'R | $CH_3$ | 2-Naphthyl | |
| 232 | SC(S)NR'R | $CH_3$ | Benzyl | 4,50 ($CH_2$) Amidrotamere |
| 233 | SC(S)NR'R | $CH_3$ | 2-Methylbenzyl | |
| 234 | SC(S)NR'R | $CH_3$ | 3-Methylbenzyl | |

| Nr. | D | R' | R | phys. Daten[a)] |
|---|---|---|---|---|
| 235 | SC(S)NR'R | $CH_3$ | 4-Methylbenzyl | |
| 236 | SC(S)NR'R | $CH_3$ | 4-tert.-Butylbenzyl | 1,31 (9H), 4,50 ($CH_2$) Amidrotamere |
| 237 | SC(S)NR'R | $CH_3$ | 2-Chlorbenzyl | |
| 238 | SC(S)NR'R | $CH_3$ | 3-Chlorbenzyl | |
| 239 | SC(S)NR'R | $CH_3$ | 4-Chlorbenzyl | |
| 240 | SC(S)NR'R | $CH_3$ | 2-Pyridyl | |
| 241 | SC(S)NR'R | $CH_3$ | 3-Pyridyl | |
| 242 | SC(S)NR'R | $CH_3$ | 4-Pyridyl | |
| 243 | SC(S)NR'R | $CH_3$ | 2,6-Pyrimidinyl | |
| 244 | SC(S)NR'R | $CH_3$ | 1,5-Pyrimidinyl | |
| 245 | SC(S)NR'R | $CH_3$ | 2-Thienyl | |
| 246 | SC(S)NR'R | $CH_3$ | 3-Thienyl | |
| 247 | SC(S)NR'R | $CH_3$ | 2-Furyl | |
| 248 | SC(S)NR'R | $CH_3$ | 3-Furyl | |
| 249 | SC(S)NR'R | $CH_3$ | 1-Pyrrolyl | |
| 250 | SC(S)NR'R | $CH_3$ | 1-Imidazolyl | |
| 251 | SC(S)NR'R | $CH_3$ | 1,2,4-Triazolyl | |
| 252 | SC(S)NR'R | $CH_3$ | 1,3,4-Triazolyl | |
| 253 | SC(S)NR'R | $CH_3$ | 4-Thiazolyl | |

| Nr. | D | R' | R | phys. Daten[a)] |
|---|---|---|---|---|
| 254 | SC(S)NR'R | $CH_3$ | 2-Benzothiazolyl | |
| 255 | SC(S)NR'R | $CH_3$ | 2-Pyridylmethyl | |
| 256 | SC(S)NR'R | $CH_3$ | 3-Pyridylmethyl | |
| 257 | SC(S)NR'R | $CH_3$ | 4-Pyridylmethyl | |
| 258 | SC(S)NR'R | Et | Phenyl | 1,26 (9H), 3,57, 3,72 (3H), 4,24 (2x2H) |
| 259 | SC(S)NR'R | Et | 2-Methylphenyl | |
| 260 | SC(S)NR'R | Et | 2-Chlor-phenyl | |
| 261 | SC(S)NR'R | Et | 4-Methylphenyl | 107 - 112°C |
| 262 | SC(S)NR'R | Et | 2-Naphtyl | |
| 263 | SC(S)SR | - | $CH_3$ | |
| 264 | SC(S)SR | - | $CH_2$-phenyl | |
| 265 | SC(S)SR | - | Phenyl | |
| 266 | SC(S)SR | - | A* | 3,67, 3,77 (3H), 4,55 (2H), 7,39 (1H) |
| 267 | SC(S)OR | - | $CH_3$ | |
| 268 | SC(S)OR | - | Phenyl | |
| 269 | SC(S)OR | - | 2-$CH_3$-phenyl | |
| 270 | SC(S)OR | - | 3-$CH_3$-phenyl | 2,38, 3,71, 3,84 (3H), 4,39 (2H) |
| 271 | SC(S)OR | - | 4-$CH_3$-phenyl | |
| 272 | SC(S)OR | - | 2-Cl-phenyl | |

| Nr. | D | R' | R | phys. Daten[a)] |
|---|---|---|---|---|
| 273 | SC(S)OR | - | 3-Cl-phenyl | |
| 274 | SC(S)OR | - | 4-Cl-phenyl | |
| 275 | SC(S)OR | - | $CH_2$-phenyl | 1707, 1633, 1256, 1197, 1126, 1054 |
| 276 | SC(S)OR | - | $CH_2$-(2-Me)-phenyl | 2,35, 3,69, 3,81 (3H), 4,32, 5,63 (2H), 7,58 (1H) |
| 277 | SC(S)OR | - | $CH_2$-(3-Me)-phenyl | |
| 278 | SC(S)OR | - | $CH_2$-(4-Me)-phenyl | |
| 279 | SC(S)OR | - | $CH_2$-(2-Cl)-phenyl | 1746, 1462, 1255, 1127, 1060 |
| 280 | SC(S)OR | - | $CH_2$-(3-Cl)phenyl | |
| 281 | SC(S)OR | - | $CH_2$-(4-Cl)phenyl | |
| 282 | OC(O)OR | - | $CH_3$ | |
| 283 | OC(O)OR | - | tert.-Butyl | |
| 284 | OC(O)OR | - | $CH_2$-phenyl | |
| 285 | OC(O)OR | - | Phenyl | |
| 286 | SC(S)NR'R | $CH_3$ | 3-Me, 4-F-phenyl | 2,25; 3,57; 3,72; 3,75 (4 x 3H); 4,35 (2H); 7,00-7,41 (7H); 7,51 (1H) |
| 287 | SC(S)NR'R | H | 2-Phenyl-eth-2-yl | 7,58 (1H) Amidrotamere |
| 288 | SC(S)NR'R | Me | 4-tert-Butoxybenzyl | 98 - 102°C |
| 289 | SC(S)NR'R | Et | 3,4-(-$OCH_2O$-)phenyl | 130 - 134°C |
| 290 | SC(S)OR | - | Ethyl | 1708, 1633, 1257, 1126, 1048 |

| Nr. | D | R' | R | phys. Daten[a)] |
|---|---|---|---|---|
| 291 | SC(S)OR | - | i-Propyl | 1707, 1632, 1250, 1226, 1224, 1089, 1037 |
| 292 | SC(S)OR | - | 4-Methoxybenzyl | 3,70, 3,80, 3,83 (3H), 4,57 (2H), 7,58 (1H) |
| 293 | SC(S)NR'R | Me | $CH_2$-(3-pyridyl) | 1705, 1633, 1480, 1432, 1384, 1258, 1198, 1125, 989 |

A* = 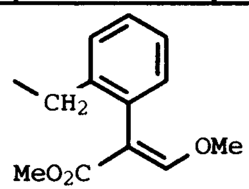

a) IR ($cm^{-1}$); $^1H$-NMR ($CDCl_3$/TMS); m. p.

Tabelle 2

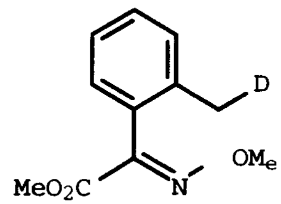

| Nr. | D | R' | R | phys. Daten[a)] |
|---|---|---|---|---|
| 1 | SC(S)NR'R | $CH_3$ | Methyl | |
| 2 | SC(S)NR'R | $CH_3$ | Ethyl | |
| 3 | SC(S)NR'R | $CH_3$ | n-Propyl | |
| 4 | SC(S)NR'R | $CH_3$ | tert.-Butyl | |
| 5 | SC(S)NR'R | $CH_3$ | Cyclopropyl | |
| 6 | SC(S)NR'R | $CH_3$ | Cyclohexyl | 2934, 1727, 1473, 1439, 1393, 1319, 1216, 1087, 1067, 1017 |
| 7 | SC(S)NR'R | $CH_3$ | Methoxymethyl | |
| 8 | SC(S)NR'R | $CH_3$ | Phenoxymethyl | |
| 9 | SC(S)NR'R | $CH_3$ | Methylthiomethyl | |
| 10 | SC(S)NR'R | $CH_3$ | Phenyl | 3,75; 3,76; 3,95 (3 x 3H); 4,32(2H); 7,08-7,51 (9H); |

| Nr. | D | R' | R | phys. Daten[a)] |
|---|---|---|---|---|
| 11 | SC(S)NR'R | $CH_3$ | 2-Fluorphenyl | |
| 12 | SC(S)NR'R | $CH_3$ | 3-Fluorphenyl | 3,73; 3,80; 3,99 (3 x 3H); 4,33 (2H); 6,95-7,50 (8H) |
| 13 | SC(S)NR'R | $CH_3$ | 4-Fluorphenyl | 3,72; 3,77; 3,97 (3 x 3H); 4,32 (2H); 7,07-7,48 (8H) |
| 14 | SC(S)NR'R | $CH_3$ | Pentafluorphenyl | |
| 15 | SC(S)NR'R | $CH_3$ | 2-Chlorphenyl | |
| 16 | SC(S)NR'R | $CH_3$ | 3-Chlorphenyl | 3,72; 3,80; 3,97 (3 x 3H); 4,34 (2H); 7,10-7,49 (8H) |
| 17 | SC(S)NR'R | $CH_3$ | 4-Chlorphenyl | 2,91, 3,72, 4,05 (3H), 4,30 (2H), 6,57-7,40 ppm (8H) |
| 18 | SC(S)NR'R | $CH_3$ | Pentachlorphenyl | |
| 19 | SC(S)NR'R | $CH_3$ | 2,3-Dichlorphenyl | |
| 20 | SC(S)NR'R | $CH_3$ | 2,,4-Dichlorphenyl | |
| 21 | SC(S)NR'R | $CH_3$ | 2,5-Dichlorphenyl | |
| 22 | SC(S)NR'R | $CH_3$ | 2,6-Dichlorphenyl | |
| 23 | SC(S)NR'R | $CH_3$ | 3,4-Dichlorphenyl | |
| 24 | SC(S)NR'R | $CH_3$ | 3,5-Dichlorphenyl | |
| 25 | SC(S)NR'R | $CH_3$ | 2,3,4-Trichlorphenyl | |
| 26 | SC(S)NR'R | $CH_3$ | 2,3,5-Trichlorphenyl | |
| 27 | SC(S)NR'R | $CH_3$ | 2,3,6-Trichlorphenyl | |

| Nr. | D | R' | R | phys. Daten[a)] |
|---|---|---|---|---|
| 28 | SC(S)NR'R | $CH_3$ | 2,4,5-Trichlorphenyl | |
| 29 | SC(S)NR'R | $CH_3$ | 2,4,6-Trichlorphenyl | |
| 30 | SC(S)NR'R | $CH_3$ | 3,4,5-Trichlorphenyl | |
| 31 | SC(S)NR'R | $CH_3$ | 2,3,4,6-Tetrachlorphenyl | |
| 32 | SC(S)NR'R | $CH_3$ | 2,3,5,6-Tetrachlorphenyl | |
| 33 | SC(S)NR'R | $CH_3$ | 2-Bromphenyl | |
| 34 | SC(S)NR'R | $CH_3$ | 3-Bromphenyl | |
| 35 | SC(S)NR'R | $CH_3$ | 4-Bromphenyl | |
| 36 | SC(S)NR'R | $CH_3$ | 2,4-Dibromphenyl | |
| 37 | SC(S)NR'R | $CH_3$ | 3-Brom-4-Fluorphenyl | |
| 38 | SC(S)NR'R | $CH_3$ | 3-Brom-4-Methoxyphenyl | |
| 39 | SC(S)NR'R | $CH_3$ | 2-Jodphenyl | |
| 40 | SC(S)NR'R | $CH_3$ | 3-Jodphenyl | |
| 41 | SC(S)NR'R | $CH_3$ | 4-Jodphenyl | |
| 42 | SC(S)NR'R | $CH_3$ | 2-Chlor-4-Fluorphenyl | |
| 43 | SC(S)NR'R | $CH_3$ | 2-Chlor-5-Fluorphenyl | |
| 44 | SC(S)NR'R | $CH_3$ | 2-Chlor-6-Fluorphenyl | |
| 45 | SC(S)NR'R | $CH_3$ | 2-Chlor-4-Bromphenyl | |
| 46 | SC(S)NR'R | $CH_3$ | 2-Brom-4-Chlorphenyl | |

| Nr. | D | R' | R | phys. Daten[a)] |
|---|---|---|---|---|
| 47 | SC(S)NR'R | $CH_3$ | 2-Brom-4-Fluorphenyl | |
| 48 | SC(S)NR'R | $CH_3$ | 3-Brom-4-Fluorphenyl | |
| 49 | SC(S)NR'R | $CH_3$ | 3-Chlor-4-Fluorphenyl | |
| 50 | SC(S)NR'R | $CH_3$ | 3-Fluor-4-Chlorphenyl | |
| 51 | SC(S)NR'R | $CH_3$ | 2-Cyanophenyl | |
| 52 | SC(S)NR'R | $CH_3$ | 3-Cyanophenyl | |
| 53 | SC(S)NR'R | $CH_3$ | 4-Cyanophenyl | |
| 54 | SC(S)NR'R | $CH_3$ | 2-Nitrophenyl | |
| 55 | SC(S)NR'R | $CH_3$ | 3-Nitrophenyl | |
| 56 | SC(S)NR'R | $CH_3$ | 4-Nitrophenyl | |
| 57 | SC(S)NR'R | $CH_3$ | 2-Methylphenyl | 2,17; 3,69; 3,76; 3,95; (4 x 3H); 4,33 (2H); 7,06-7,50 (8H) |
| 58 | SC(S)NR'R | $CH_3$ | 3-Methylphenyl | 2,36; 3,74; 3,77; 3,96; (4 x 3H); 4,32 (2H); 6,98-7,48 (8H) |
| 59 | SC(S)NR'R | $CH_3$ | 4-Methylphenyl | 2,35; 3,72; 3,77; 3,97; (4 x 3H); 4,30 (2H); 7,07-7,47 (8H) |
| 60 | SC(S)NR'R | $CH_3$ | 2,4-Dimethylphenyl | |
| 61 | SC(S)NR'R | $CH_3$ | 2,6-Dimethylphenyl | |
| 62 | SC(S)NR'R | $CH_3$ | 3,4-Dimethylphenyl | |
| 63 | SC(S)NR'R | $CH_3$ | 3,5-Dimethylphenyl | |

| Nr. | D | R' | R | phys. Daten[a]) |
|---|---|---|---|---|
| 64 | SC(S)NR'R | $CH_3$ | 2,3,4-Trimethylphenyl | |
| 65 | SC(S)NR'R | $CH_3$ | 2,3,5-Trimethylphenyl | |
| 66 | SC(S)NR'R | $CH_3$ | 2,3,6-Trimethylphenyl | |
| 67 | SC(S)NR'R | $CH_3$ | 2,4,5-Trimethylphenyl | |
| 68 | SC(S)NR'R | $CH_3$ | 2,4,6-Trimethylphenyl | |
| 69 | SC(S)NR'R | $CH_3$ | 3,4,5-Trimethylphenyl | |
| 70 | SC(S)NR'R | $CH_3$ | Pentamethylphenyl | |
| 71 | SC(S)NR'R | $CH_3$ | 2-Ethylphenyl | |
| 72 | SC(S)NR'R | $CH_3$ | 3-Ethylphenyl | |
| 73 | SC(S)NR'R | $CH_3$ | 4-Ethylphenyl | |
| 74 | SC(S)NR'R | $CH_3$ | 3,5-Diethylphenyl | |
| 75 | SC(S)NR'R | $CH_3$ | 2-n-Propylphenyl | |
| 76 | SC(S)NR'R | $CH_3$ | 3-n-Propylphenyl | |
| 77 | SC(S)NR'R | $CH_3$ | 4-n-Propylphenyl | |
| 78 | SC(S)NR'R | $CH_3$ | 2-iso-Propylphenyl | |
| 79 | SC(S)NR'R | $CH_3$ | 3-iso-Propylphenyl | |
| 80 | SC(S)NR'R | $CH_3$ | 4-iso-Propylphenyl | |
| 81 | SC(S)NR'R | $CH_3$ | 2,4-Di-iso-Propylphenyl | |
| 82 | SC(S)NR'R | $CH_3$ | 3,5-Di-iso-Propylphenyl | |

| Nr. | D | R' | R | phys. Daten[a)] |
|---|---|---|---|---|
| 83 | SC(S)NR'R | $CH_3$ | 4-n-Butyphenyl | |
| 84 | SC(S)NR'R | $CH_3$ | 4-sec.-Butylphenyl | |
| 85 | SC(S)NR'R | $CH_3$ | 4-iso-Butylphenyl | |
| 86 | SC(S)NR'R | $CH_3$ | 4-tert.-Butylphenyl | |
| 87 | SC(S)NR'R | $CH_3$ | 3-tert.-Butylphenyl | |
| 88 | SC(S)NR'R | $CH_3$ | 2-tert.-Butylphenyl | |
| 89 | SC(S)NR'R | $CH_3$ | 2,4-Di-tert.-Butylphenyl | |
| 90 | SC(S)NR'R | $CH_3$ | 3,5-Di-tert.-Butylphenyl | |
| 91 | SC(S)NR'R | $CH_3$ | 4-n-Hexylphenyl | |
| 92 | SC(S)NR'R | $CH_3$ | 4-n-Dodecylphenyl | |
| 93 | SC(S)NR'R | $CH_3$ | 2-Methyl-4-tert.-Butyl-phenyl | |
| 94 | SC(S)NR'R | $CH_3$ | 2-Methyl-6-tert.-Butyl-phenyl | |
| 95 | SC(S)NR'R | $CH_3$ | 2-Methyl-4-iso-Propylphenyl | |
| 96 | SC(S)NR'R | $CH_3$ | 2-Methyl-4-Cyclohexylphenyl | |
| 97 | SC(S)NR'R | $CH_3$ | 2-Methyl-4-Phenylphenyl | |
| 98 | SC(S)NR'R | $CH_3$ | 2-Methyl-4-Benzylphenyl | |
| 99 | SC(S)NR'R | $CH_3$ | 2-Methyl-4-Phenoxyphenyl | |
| 100 | SC(S)NR'R | $CH_3$ | 2-Methyl-4-Benzyloxyphenyl | |
| 101 | SC(S)NR'R | $CH_3$ | 2-Methyl-3-Chlorphenyl | |

| Nr. | D | R' | R | phys. Daten[a)] |
|---|---|---|---|---|
| 102 | SC(S)NR'R | $CH_3$ | 2-Methyl-4-Chlorphenyl | |
| 103 | SC(S)NR'R | $CH_3$ | 2-Methyl-5-Chlorphenyl | |
| 104 | SC(S)NR'R | $CH_3$ | 2-Methyl-6-Chlorphenyl | |
| 105 | SC(S)NR'R | $CH_3$ | 2-Methyl-4-Fluorphenyl | |
| 106 | SC(S)NR'R | $CH_3$ | 2-Methyl-3-Bromphenyl | |
| 107 | SC(S)NR'R | $CH_3$ | 2-Methyl-4-Bromphenyl | |
| 108 | SC(S)NR'R | $CH_3$ | 2-Methyl-3-Methoxyphenyl | |
| 109 | SC(S)NR'R | $CH_3$ | 2-Methyl-4-Methoxyphenyl | |
| 110 | SC(S)NR'R | $CH_3$ | 2-Methyl-5-Methoxyphenyl | |
| 111 | SC(S)NR'R | $CH_3$ | 2-Methyl-6-Methoxyphenyl | |
| 112 | SC(S)NR'R | $CH_3$ | 2-Methyl-4-iso-Propoxy-phenyl | |
| 113 | SC(S)NR'R | $CH_3$ | 2-Methyl-2,5-Dimethoxy-phenyl | |
| 114 | SC(S)NR'R | $CH_3$ | 2-Methoxyphenyl | |
| 115 | SC(S)NR'R | $CH_3$ | 3-Methoxyphenyl | |
| 116 | SC(S)NR'R | $CH_3$ | 4-Methoxyphenyl | |
| 117 | SC(S)NR'R | $CH_3$ | 2,3-Dimethoxyphenyl | |
| 118 | SC(S)NR'R | $CH_3$ | 2,4-Dimethoxyphenyl | |
| 119 | SC(S)NR'R | $CH_3$ | 2,5-Dimethoxyphenyl | |
| 120 | SC(S)NR'R | $CH_3$ | 2,6-Dimethoxyphenyl | |

| Nr. | D | R' | R | phys. Daten[a)] |
|---|---|---|---|---|
| 121 | SC(S)NR'R | $CH_3$ | 3,4-Dimethoxyphenyl | |
| 122 | SC(S)NR'R | $CH_3$ | 3,5-Dimethoxyphenyl | |
| 123 | SC(S)NR'R | $CH_3$ | 3,6-Dimethoxyphenyl | |
| 124 | SC(S)NR'R | $CH_3$ | 2,3,4-Trimethoxyphenyl | |
| 125 | SC(S)NR'R | $CH_3$ | 2,3,5-Trimethoxyphenyl | |
| 126 | SC(S)NR'R | $CH_3$ | 2,3,6-Trimethoxyphenyl | |
| 127 | SC(S)NR'R | $CH_3$ | 2,4,5-Trichlorphenyl | |
| 128 | SC(S)NR'R | $CH_3$ | 2,4,6-Trichloryphenyl | |
| 129 | SC(S)NR'R | $CH_3$ | 2,3,4,6-Tetrachlorphenyl | |
| 130 | SC(S)NR'R | $CH_3$ | 2,3,5,6-Tetrachlorphenyl | |
| 131 | SC(S)NR'R | $CH_3$ | 2-Bromphenyl | |
| 132 | SC(S)NR'R | $CH_3$ | 3-Bromphenyl | |
| 133 | SC(S)NR'R | $CH_3$ | 4-Bromphenyl | |
| 134 | SC(S)NR'R | $CH_3$ | 2,4-Dibromphenyl | |
| 135 | SC(S)NR'R | $CH_3$ | 3-Brom-4-Fluorphenyl | |
| 136 | SC(S)NR'R | $CH_3$ | 3-Brom-4-Methoxyphenyl | |
| 137 | SC(S)NR'R | $CH_3$ | 2-Jodphenyl | |
| 138 | SC(S)NR'R | $CH_3$ | 3-Jodphenyl | |
| 139 | SC(S)NR'R | $CH_3$ | 4-Jodphenyl | |

| Nr. | D | R' | R | phys. Daten[a)] |
|---|---|---|---|---|
| 140 | SC(S)NR'R | $CH_3$ | 2-Chlor-4-Fluorphenyl | |
| 141 | SC(S)NR'R | $CH_3$ | 2-Chlor-5-Fluorphenyl | |
| 142 | SC(S)NR'R | $CH_3$ | 2-Chlor-6-Fluorphenyl | |
| 143 | SC(S)NR'R | $CH_3$ | 2-Chlor-4-Bromphenyl | |
| 144 | SC(S)NR'R | $CH_3$ | 2-Brom-4-Chlorphenyl | |
| 145 | SC(S)NR'R | $CH_3$ | 2-Brom-4-Fluorphenyl | |
| 146 | SC(S)NR'R | $CH_3$ | 3-Brom-4-Chlorphenyl | |
| 147 | SC(S)NR'R | $CH_3$ | 3-Chlor-4-Fluorphenyl | |
| 148 | SC(S)NR'R | $CH_3$ | 3-Fluor-4-Chlorphenyl | |
| 149 | SC(S)NR'R | $CH_3$ | 2-Cyanophenyl | |
| 150 | SC(S)NR'R | $CH_3$ | 3-Cyanophenyl | |
| 151 | SC(S)NR'R | $CH_3$ | 4-Cyanophenyl | |
| 152 | SC(S)NR'R | $CH_3$ | 2-Nitrophenyl | |
| 153 | SC(S)NR'R | $CH_3$ | 3-Nitrophenyl | |
| 154 | SC(S)NR'R | $CH_3$ | 4-Nitrophenyl | |
| 155 | SC(S)NR'R | $CH_3$ | 2-Methylphenyl | |
| 156 | SC(S)NR'R | $CH_3$ | 3-Methylphenyl | |
| 157 | SC(S)NR'R | $CH_3$ | 4-Methylphenyl | |
| 158 | SC(S)NR'R | $CH_3$ | 2,4-Dimethylphenyl | |

| Nr. | D | R' | R | phys. Daten[a)] |
|---|---|---|---|---|
| 159 | SC(S)NR'R | $CH_3$ | 2,6-Dimethylphenyl | |
| 160 | SC(S)NR'R | $CH_3$ | 3,4-Dimethylphenyl | |
| 161 | SC(S)NR'R | $CH_3$ | 3,5-Dimethylphenyl | |
| 162 | SC(S)NR'R | $CH_3$ | 2,3,4-Trimethylphenyl | |
| 163 | SC(S)NR'R | $CH_3$ | 2,3,5-Trimethylphenyl | |
| 164 | SC(S)NR'R | $CH_3$ | 2,3,6-Trimethylphenyl | |
| 165 | SC(S)NR'R | $CH_3$ | 2,4,5-Trimethylphenyl | |
| 166 | SC(S)NR'R | $CH_3$ | 2,4,6-Trimethylphenyl | |
| 167 | SC(S)NR'R | $CH_3$ | 2,4,5-Trimethylphenyl | |
| 168 | SC(S)NR'R | $CH_3$ | Pentamethylphenyl | |
| 169 | SC(S)NR'R | $CH_3$ | 2-Ethylphenyl | |
| 170 | SC(S)NR'R | $CH_3$ | 3-Ethylphenyl | |
| 171 | SC(S)NR'R | $CH_3$ | 4-Ethylphenyl | |
| 172 | SC(S)NR'R | $CH_3$ | 3,5-Diethylphenyl | |
| 173 | SC(S)NR'R | $CH_3$ | 2-n-Propylphenyl | |
| 174 | SC(S)NR'R | $CH_3$ | 3-n-Propylphenyl | |
| 175 | SC(S)NR'R | $CH_3$ | 4-n-Propylphenyl | |
| 176 | SC(S)NR'R | $CH_3$ | 2,4,5-Trimethoxyphenyl | |
| 177 | SC(S)NR'R | $CH_3$ | 2,4,6-Trimethoxyphenyl | |

| Nr. | D | R' | R | phys. Daten[a)] |
|---|---|---|---|---|
| 178 | SC(S)NR'R | $CH_3$ | 3,4,5-Trimethoxyphenyl | |
| 179 | SC(S)NR'R | $CH_3$ | 2-Ethoxyphenyl | |
| 180 | SC(S)NR'R | $CH_3$ | 3-Ethoxyphenyl | |
| 181 | SC(S)NR'R | $CH_3$ | 4-Ethoxyphenyl | |
| 182 | SC(S)NR'R | $CH_3$ | 2-iso-Propoxyphenyl | |
| 183 | SC(S)NR'R | $CH_3$ | 3-iso-Propoxyphenyl | |
| 184 | SC(S)NR'R | $CH_3$ | 4-iso-Propoxyphenyl | |
| 185 | SC(S)NR'R | $CH_3$ | 3-tert.-Butoxyphenyl | |
| 186 | SC(S)NR'R | $CH_3$ | 4-tert.-Butoxyphenyl | |
| 187 | SC(S)NR'R | $CH_3$ | 2-Trifluormethoxyphenyl | |
| 188 | SC(S)NR'R | $CH_3$ | 3-Trifluormethoxyphenyl | |
| 189 | SC(S)NR'R | $CH_3$ | 4-Trifluormethoxyphenyl | |
| 190 | SC(S)NR'R | $CH_3$ | 3-(1',1',2',2'-Tetra-fluor)-ethoxyphenyl | |
| 191 | SC(S)NR'R | $CH_3$ | 4-(1',1',2',2'-Tetra-fluor)-ethoxyphenyl | |
| 192 | SC(S)NR'R | $CH_3$ | 2-Chlormethylphenyl | |
| 193 | SC(S)NR'R | $CH_3$ | 3-Chlormethylphenyl | |
| 194 | SC(S)NR'R | $CH_3$ | 4-Chlormethylphenyl | |
| 195 | SC(S)NR'R | $CH_3$ | 2-Trifluormethylphenyl | |

| Nr. | D | R' | R | phys. Daten[a)] |
|---|---|---|---|---|
| 196 | SC(S)NR'R | $CH_3$ | 3-Trifluormethylphenyl | |
| 197 | SC(S)NR'R | $CH_3$ | 4-Trifluormethylphenyl | |
| 198 | SC(S)NR'R | $CH_3$ | 2-(Methoxyiminomethyl)-phenyl | |
| 199 | SC(S)NR'R | $CH_3$ | 3-(Methoxyiminomethyl)-phenyl | |
| 200 | SC(S)NR'R | $CH_3$ | 4-(Methoxyiminomethyl)-phenyl | |
| 201 | SC(S)NR'R | $CH_3$ | 2-(Ethoxyiminomethyl)-phenyl | |
| 202 | SC(S)NR'R | $CH_3$ | 3-(Ethoxyiminomethyl)-phenyl | |
| 203 | SC(S)NR'R | $CH_3$ | 4-(Ethoxyiminomethyl)-phenyl | |
| 204 | SC(S)NR'R | $CH_3$ | 2-Methyl-4-Methoximinoethyl-phenyl | |
| 205 | SC(S)NR'R | $CH_3$ | 2-Methyl-4-Methoximinomethyl-phenyl | |
| 206 | SC(S)NR'R | $CH_3$ | 2,6-Dimethyl-4-Methoximino-methyl-phenyl | |
| 207 | SC(S)NR'R | $CH_3$ | 2,6-Dimethyl-4-Methoximino-ethyl-phenyl | |
| 208 | SC(S)NR'R | $CH_3$ | 2-Phenylphenyl | |
| 209 | SC(S)NR'R | $CH_3$ | 3-Phenylphenyl | |
| 210 | SC(S)NR'R | $CH_3$ | 4-Phenylphenyl | |
| 211 | SC(S)NR'R | $CH_3$ | 2-Phenoxyphenyl | |
| 212 | SC(S)NR'R | $CH_3$ | 3-Phenoxyphenyl | |

| Nr. | D | R' | R | phys. Daten[a)] |
|---|---|---|---|---|
| 213 | SC(S)NR'R | $CH_3$ | 4-Phenoxyphenyl | |
| 214 | SC(S)NR'R | $CH_3$ | 2-Benzyloxyphenyl | |
| 215 | SC(S)NR'R | $CH_3$ | 3-Benzyloxyphenyl | |
| 216 | SC(S)NR'R | $CH_3$ | 4-Benzyloxyphenyl | |
| 217 | SC(S)NR'R | $CH_3$ | 4-(Imidazol-1'-yl)phenyl | |
| 218 | SC(S)NR'R | $CH_3$ | 4-(Piperazin-1'-yl)phenyl | |
| 219 | SC(S)NR'R | $CH_3$ | 4-(Morpholin-1'-yl)phenyl | |
| 220 | SC(S)NR'R | $CH_3$ | 4-(Piperidin-1'-yl)phenyl | |
| 221 | SC(S)NR'R | $CH_3$ | 4-(Pyridyl-2'-oxy)phenyl | |
| 222 | SC(S)NR'R | $CH_3$ | 2-Cyclopropylphenyl | |
| 223 | SC(S)NR'R | $CH_3$ | 3-Cyclopropylphenyl | |
| 224 | SC(S)NR'R | $CH_3$ | 4-Cyclopropylphenyl | |
| 225 | SC(S)NR'R | $CH_3$ | 3-Cyclohexylphenyl | |
| 226 | SC(S)NR'R | $CH_3$ | 4-Cyclohexylphenyl | |
| 227 | SC(S)NR'R | $CH_3$ | 4-Oxiranylphenyl | |
| 228 | SC(S)NR'R | $CH_3$ | 4-(1',3'-Dioxan-2'-yl)-phenyl | |
| 229 | SC(S)NR'R | $CH_3$ | 4-(Tetrahydropyran-2-yl-oxy)-phenyl | |
| 230 | SC(S)NR'R | $CH_3$ | 1-Naphthyl | |
| 231 | SC(S)NR'R | $CH_3$ | 2-Naphthyl | |

| Nr. | D | R' | R | phys. Daten[a)] |
|---|---|---|---|---|
| 232 | SC(S)NR'R | $CH_3$ | Benzyl | 84 - 90°C |
| 233 | SC(S)NR'R | $CH_3$ | 2-Methylbenzyl | |
| 234 | SC(S)NR'R | $CH_3$ | 3-Methylbenzyl | |
| 235 | SC(S)NR'R | $CH_3$ | 4-Methylbenzyl | |
| 236 | SC(S)NR'R | $CH_3$ | 4-tert.-Butylbenzyl | 1,31 (9H), 4,50 (2H) Amidrotamere |
| 237 | SC(S)NR'R | $CH_3$ | 2-Chlorbenzyl | |
| 238 | SC(S)NR'R | $CH_3$ | 3-Chlorbenzyl | |
| 239 | SC(S)NR'R | $CH_3$ | 4-Chlorbenzyl | |
| 240 | SC(S)NR'R | $CH_3$ | 2-Pyridyl | |
| 241 | SC(S)NR'R | $CH_3$ | 3-Pyridyl | |
| 242 | SC(S)NR'R | $CH_3$ | 4-Pyridyl | |
| 243 | SC(S)NR'R | $CH_3$ | 2,6-Pyrimidinyl | |
| 244 | SC(S)NR'R | $CH_3$ | 1,5-Pyrimidinyl | |
| 245 | SC(S)NR'R | $CH_3$ | 2-Thienyl | |
| 246 | SC(S)NR'R | $CH_3$ | 3-Thienyl | |
| 247 | SC(S)NR'R | $CH_3$ | 2-Furyl | |
| 248 | SC(S)NR'R | $CH_3$ | 3-Furyl | |
| 249 | SC(S)NR'R | $CH_3$ | 1-Pyrrolyl | |
| 250 | SC(S)NR'R | $CH_3$ | 1-Imidazolyl | |

| Nr. | D | R' | R | phys. Daten[a)] |
|---|---|---|---|---|
| 251 | SC(S)NR'R | $CH_3$ | 1,2,4-Triazolyl | |
| 252 | SC(S)NR'R | $CH_3$ | 1,3,4-Triazolyl | |
| 253 | SC(S)NR'R | $CH_3$ | 4-Thiazolyl | |
| 254 | SC(S)NR'R | $CH_3$ | 2-Benzothiazolyl | |
| 255 | SC(S)NR'R | $CH_3$ | 2-Pyridylmethyl | |
| 256 | SC(S)NR'R | $CH_3$ | 3-Pyridylmethyl | |
| 257 | SC(S)NR'R | $CH_3$ | 4-Pyridylmethyl | |
| 258 | SC(S)NR'R | Et | Phenyl | 1,24 (3H), 3,76, 3,94 (3H), 4,03 ($2xCH_2$) |
| 259 | SC(S)NR'R | Et | 2-Methylphenyl | |
| 260 | SC(S)NR'R | Et | 2-Chlor-phenyl | |
| 261 | SC(S)NR'R | Et | 4-Methylphenyl | 1633, 1523, 1508, 1444, 1399, 1305, 1272, 1103, 1038 |
| 262 | SC(S)NR'R | Et | 2-Naphtyl | 110 - 114°C |
| 263 | SC(S)SR | - | $CH_3$ | |
| 264 | SC(S)SR | - | $CH_2$-phenyl | |
| 265 | SC(S)SR | - | Phenyl | |
| 266 | SC(S)SR | - | A* | 114 - 115°C |
| 267 | SC(S)OR | - | $CH_3$ | 3,85, 4,04, 4,14 (3H); 4,25 (2H) |
| 268 | SC(S)OR | - | Phenyl | |
| 269 | SC(S)OR | - | 2-$CH_3$-phenyl | |

| Nr. | D | R' | R | phys. Daten[a)] |
|---|---|---|---|---|
| 270 | SC(S)OR | - | 3-$CH_3$-phenyl | |
| 271 | SC(S)OR | - | 4-$CH_3$-phenyl | |
| 272 | SC(S)OR | - | 2-Cl-phenyl | |
| 273 | SC(S)OR | - | 3-Cl-phenyl | |
| 274 | SC(S)OR | - | 4-Cl-phenyl | |
| 275 | SC(S)OR | - | $CH_2$-phenyl | |
| 276 | SC(S)OR | - | $CH_2$-(2-Me)-phenyl | 1727, 1436, 1218, 1066, 1017 |
| 277 | SC(S)OR | - | $CH_2$-(3-Me)-phenyl | 1727, 1437, 1218, 1066, 1018 |
| 278 | SC(S)OR | - | $CH_2$-(4-Me)-phenyl | |
| 279 | SC(S)OR | - | $CH_2$-(2-Cl)-phenyl | 1727, 1442, 1221, 1065, 1017 |
| 280 | SC(S)OR | - | $CH_2$-(3-Cl)phenyl | 3,86, 4,04 (3H), 4,28, 5,58 (2H) |
| 281 | SC(S)OR | - | $CH_2$-(4-Cl)phenyl | |
| 282 | OC(O)OR | - | $CH_3$ | |
| 283 | OC(O)OR | - | tert.-Butyl | |
| 284 | OC(O)OR | - | $CH_2$-phenyl | |
| 285 | OC(O)OR | - | Phenyl | |
| 286 | SC(S)NR'R | $CH_3$ | 3-Me, 4-F-phenyl | 2,27; 3,71; 3,78; 3,97 (4 x 3H); 4,32 (2H); 7,00-7,49 (7H) |
| 287 | SC(S)NR'R | H | 2-Phenyl-eth-2-yl | 102 - 103°C |

| Nr. | D | R' | R | phys. Daten[a)] |
|---|---|---|---|---|
| 288 | SC(S)NR'R | Me | 4-tert-Butoxybenzyl | 2975, 1726, 1656, 1482, 1387, 1236, 1216, 1161, 1066, 1017 |
| 289 | SC(S)NR'R | Et | 3,4-(-$OCH_2O$-)phenyl | 108 - 113°C |
| 290 | SC(S)OR | - | Ethyl | 1727, 1437, 1321, 1218, 1066, 1047, 1017 |
| 291 | SC(S)OR | - | i-Propyl | 1726, 1436, 1218, 1090, 1066, 1040, 1017 |
| 292 | SC(S)OR | - | Benzyl | 3,87, 4,05 (3H), 4,27, 5,63 (2H) |

A* = 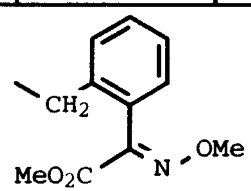

a) IR ($cm^{-1}$); $^1$H-NMR ($CDCl_3$/TMS); m. p.

Tabelle 3

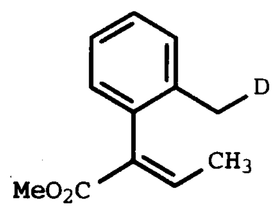

| Nr. | D | R' | R | phys. Daten[a)] |
|---|---|---|---|---|
| 1 | SC(S)NR'R | $CH_3$ | Methyl | |
| 2 | SC(S)NR'R | $CH_3$ | Ethyl | |
| 3 | SC(S)NR'R | $CH_3$ | n-Propyl | |
| 4 | SC(S)NR'R | $CH_3$ | tert.-Butyl | |
| 5 | SC(S)NR'R | $CH_3$ | Cyclopropyl | |
| 6 | SC(S)NR'R | $CH_3$ | Cyclohexyl | |
| 7 | SC(S)NR'R | $CH_3$ | Methoxymethyl | |
| 8 | SC(S)NR'R | $CH_3$ | Phenoxymethyl | |
| 9 | SC(S)NR'R | $CH_3$ | Methylthiomethyl | |
| 10 | SC(S)NR'R | $CH_3$ | Phenyl | |
| 11 | SC(S)NR'R | $CH_3$ | 2-Fluorphenyl | |
| 12 | SC(S)NR'R | $CH_3$ | 3-Fluorphenyl | |

| Nr. | D | R' | R | phys. Daten[a)] |
|---|---|---|---|---|
| 13 | SC(S)NR'R | $CH_3$ | 4-Fluorphenyl | |
| 14 | SC(S)NR'R | $CH_3$ | Pentafluorphenyl | |
| 15 | SC(S)NR'R | $CH_3$ | 2-Chlorphenyl | |
| 16 | SC(S)NR'R | $CH_3$ | 3-Chlorphenyl | |
| 17 | SC(S)NR'R | $CH_3$ | 4-Chlorphenyl | |
| 18 | SC(S)NR'R | $CH_3$ | Pentachlorphenyl | |
| 19 | SC(S)NR'R | $CH_3$ | 2,3-Dichlorphenyl | |
| 20 | SC(S)NR'R | $CH_3$ | 2,,4-Dichlorphenyl | |
| 21 | SC(S)NR'R | $CH_3$ | 2,5-Dichlorphenyl | |
| 22 | SC(S)NR'R | $CH_3$ | 2,6-Dichlorphenyl | |
| 23 | SC(S)NR'R | $CH_3$ | 3,4-Dichlorphenyl | |
| 24 | SC(S)NR'R | $CH_3$ | 3,5-Dichlorphenyl | |
| 25 | SC(S)NR'R | $CH_3$ | 2,3,4-Trichlorphenyl | |
| 26 | SC(S)NR'R | $CH_3$ | 2,3,5-Trichlorphenyl | |
| 27 | SC(S)NR'R | $CH_3$ | 2,3,6-Trichlorphenyl | |
| 28 | SC(S)NR'R | $CH_3$ | 2,4,5-Trichlorphenyl | |
| 29 | SC(S)NR'R | $CH_3$ | 2,4,6-Trichlorphenyl | |
| 30 | SC(S)NR'R | $CH_3$ | 3,4,5-Trichlorphenyl | |
| 31 | SC(S)NR'R | $CH_3$ | 2,3,4,6-Tetrachlorphenyl | |

| Nr. | D | R' | R | phys. Daten[a]) |
|---|---|---|---|---|
| 32 | SC(S)NR'R | $CH_3$ | 2,3,5,6-Tetrachlorphenyl | |
| 33 | SC(S)NR'R | $CH_3$ | 2-Bromphenyl | |
| 34 | SC(S)NR'R | $CH_3$ | 3-Bromphenyl | |
| 35 | SC(S)NR'R | $CH_3$ | 4-Bromphenyl | |
| 36 | SC(S)NR'R | $CH_3$ | 2,4-Dibromphenyl | |
| 37 | SC(S)NR'R | $CH_3$ | 3-Brom-4-Fluorphenyl | |
| 38 | SC(S)NR'R | $CH_3$ | 3-Brom-4-Methoxyphenyl | |
| 39 | SC(S)NR'R | $CH_3$ | 2-Jodphenyl | |
| 40 | SC(S)NR'R | $CH_3$ | 3-Jodphenyl | |
| 41 | SC(S)NR'R | $CH_3$ | 4-Jodphenyl | |
| 42 | SC(S)NR'R | $CH_3$ | 2-Chlor-4-Fluorphenyl | |
| 43 | SC(S)NR'R | $CH_3$ | 2-Chlor-5-Fluorphenyl | |
| 44 | SC(S)NR'R | $CH_3$ | 2-Chlor-6-Fluorphenyl | |
| 45 | SC(S)NR'R | $CH_3$ | 2-Chlor-4-Bromphenyl | |
| 46 | SC(S)NR'R | $CH_3$ | 2-Brom-4-Chlorphenyl | |
| 47 | SC(S)NR'R | $CH_3$ | 2-Brom-4-Fluorphenyl | |
| 48 | SC(S)NR'R | $CH_3$ | 3-Brom-4-Fluorphenyl | |
| 49 | SC(S)NR'R | $CH_3$ | 3-Chlor-4-Fluorphenyl | |
| 50 | SC(S)NR'R | $CH_3$ | 3-Fluor-4-Chlorphenyl | |

| Nr. | D | R' | R | phys. Daten[a)] |
|---|---|---|---|---|
| 51 | SC(S)NR'R | $CH_3$ | 2-Cyanophenyl | |
| 52 | SC(S)NR'R | $CH_3$ | 3-Cyanophenyl | |
| 53 | SC(S)NR'R | $CH_3$ | 4-Cyanophenyl | |
| 54 | SC(S)NR'R | $CH_3$ | 2-Nitrophenyl | |
| 55 | SC(S)NR'R | $CH_3$ | 3-Nitrophenyl | |
| 56 | SC(S)NR'R | $CH_3$ | 4-Nitrophenyl | |
| 57 | SC(S)NR'R | $CH_3$ | 2-Methylphenyl | |
| 58 | SC(S)NR'R | $CH_3$ | 3-Methylphenyl | |
| 59 | SC(S)NR'R | $CH_3$ | 4-Methylphenyl | |
| 60 | SC(S)NR'R | $CH_3$ | 2,4-Dimethylphenyl | |
| 61 | SC(S)NR'R | $CH_3$ | 2,6-Dimethylphenyl | |
| 62 | SC(S)NR'R | $CH_3$ | 3,4-Dimethylphenyl | |
| 63 | SC(S)NR'R | $CH_3$ | 3,5-Dimethylphenyl | |
| 64 | SC(S)NR'R | $CH_3$ | 2,3,4-Trimethylphenyl | |
| 65 | SC(S)NR'R | $CH_3$ | 2,3,5-Trimethylphenyl | |
| 66 | SC(S)NR'R | $CH_3$ | 2,3,6-Trimethylphenyl | |
| 67 | SC(S)NR'R | $CH_3$ | 2,4,5-Trimethylphenyl | |
| 68 | SC(S)NR'R | $CH_3$ | 2,4,6-Trimethylphenyl | |
| 69 | SC(S)NR'R | $CH_3$ | 3,4,5-Trimethylphenyl | |

| Nr. | D | R' | R | phys. Daten[a)] |
|---|---|---|---|---|
| 70 | SC(S)NR'R | $CH_3$ | Pentamethylphenyl | |
| 71 | SC(S)NR'R | $CH_3$ | 2-Ethylphenyl | |
| 72 | SC(S)NR'R | $CH_3$ | 3-Ethylphenyl | |
| 73 | SC(S)NR'R | $CH_3$ | 4-Ethylphenyl | |
| 74 | SC(S)NR'R | $CH_3$ | 3,5-Diethylphenyl | |
| 75 | SC(S)NR'R | $CH_3$ | 2-n-Propylphenyl | |
| 76 | SC(S)NR'R | $CH_3$ | 3-n-Propylphenyl | |
| 77 | SC(S)NR'R | $CH_3$ | 4-n-Propylphenyl | |
| 78 | SC(S)NR'R | $CH_3$ | 2-iso-Propylphenyl | |
| 79 | SC(S)NR'R | $CH_3$ | 3-iso-Propylphenyl | |
| 80 | SC(S)NR'R | $CH_3$ | 4-iso-Propylphenyl | |
| 81 | SC(S)NR'R | $CH_3$ | 2,4-Di-iso-Propylphenyl | |
| 82 | SC(S)NR'R | $CH_3$ | 3,5-Di-iso-Propylphenyl | |
| 83 | SC(S)NR'R | $CH_3$ | 4-n-Butyphenyl | |
| 84 | SC(S)NR'R | $CH_3$ | 4-sec.-Butylphenyl | |
| 85 | SC(S)NR'R | $CH_3$ | 4-iso-Butylphenyl | |
| 86 | SC(S)NR'R | $CH_3$ | 4-tert.-Butylphenyl | |
| 87 | SC(S)NR'R | $CH_3$ | 3-tert.-Butylphenyl | |
| 88 | SC(S)NR'R | $CH_3$ | 2-tert.-Butylphenyl | |

| Nr. | D | R' | R | phys. Daten[a)] |
|---|---|---|---|---|
| 89 | SC(S)NR'R | $CH_3$ | 2,4-Di-tert.-Butylphenyl | |
| 90 | SC(S)NR'R | $CH_3$ | 3,5-Di-tert.-Butylphenyl | |
| 91 | SC(S)NR'R | $CH_3$ | 4-n-Hexylphenyl | |
| 92 | SC(S)NR'R | $CH_3$ | 4-n-Dodecylphenyl | |
| 93 | SC(S)NR'R | $CH_3$ | 2-Methyl-4-tert.-Butyl-phenyl | |
| 94 | SC(S)NR'R | $CH_3$ | 2-Methyl-6-tert.-Butyl-phenyl | |
| 95 | SC(S)NR'R | $CH_3$ | 2-Methyl-4-iso-Propylphenyl | |
| 96 | SC(S)NR'R | $CH_3$ | 2-Methyl-4-Cyclohexylphenyl | |
| 97 | SC(S)NR'R | $CH_3$ | 2-Methyl-4-Phenylphenyl | |
| 98 | SC(S)NR'R | $CH_3$ | 2-Methyl-4-Benzylphenyl | |
| 99 | SC(S)NR'R | $CH_3$ | 2-Methyl-4-Phenoxyphenyl | |
| 100 | SC(S)NR'R | $CH_3$ | 2-Methyl-4-Benzyloxyphenyl | |
| 101 | SC(S)NR'R | $CH_3$ | 2-Methyl-3-Chlorphenyl | |
| 102 | SC(S)NR'R | $CH_3$ | 2-Methyl-4-Chlorphenyl | |
| 103 | SC(S)NR'R | $CH_3$ | 2-Methyl-5-Chlorphenyl | |
| 104 | SC(S)NR'R | $CH_3$ | 2-Methyl-6-Chlorphenyl | |
| 105 | SC(S)NR'R | $CH_3$ | 2-Methyl-4-Fluorphenyl | |
| 106 | SC(S)NR'R | $CH_3$ | 2-Methyl-3-Bromphenyl | |
| 107 | SC(S)NR'R | $CH_3$ | 2-Methyl-4-Bromphenyl | |

| Nr. | D | R' | R | phys. Daten[a)] |
|---|---|---|---|---|
| 108 | SC(S)NR'R | $CH_3$ | 2-Methyl-3-Methoxyphenyl | |
| 109 | SC(S)NR'R | $CH_3$ | 2-Methyl-4-Methoxyphenyl | |
| 110 | SC(S)NR'R | $CH_3$ | 2-Methyl-5-Methoxyphenyl | |
| 111 | SC(S)NR'R | $CH_3$ | 2-Methyl-6-Methoxyphenyl | |
| 112 | SC(S)NR'R | $CH_3$ | 2-Methyl-4-iso-Propoxy-phenyl | |
| 113 | SC(S)NR'R | $CH_3$ | 2-Methyl-2,5-Dimethoxy-phenyl | |
| 114 | SC(S)NR'R | $CH_3$ | 2-Methoxyphenyl | |
| 115 | SC(S)NR'R | $CH_3$ | 3-Methoxyphenyl | |
| 116 | SC(S)NR'R | $CH_3$ | 4-Methoxyphenyl | |
| 117 | SC(S)NR'R | $CH_3$ | 2,3-Dimethoxyphenyl | |
| 118 | SC(S)NR'R | $CH_3$ | 2,4-Dimethoxyphenyl | |
| 119 | SC(S)NR'R | $CH_3$ | 2,5-Dimethoxyphenyl | |
| 120 | SC(S)NR'R | $CH_3$ | 2,6-Dimethoxyphenyl | |
| 121 | SC(S)NR'R | $CH_3$ | 3,4-Dimethoxyphenyl | |
| 122 | SC(S)NR'R | $CH_3$ | 3,5-Dimethoxyphenyl | |
| 123 | SC(S)NR'R | $CH_3$ | 3,6-Dimethoxyphenyl | |
| 124 | SC(S)NR'R | $CH_3$ | 2,3,4-Trimethoxyphenyl | |
| 125 | SC(S)NR'R | $CH_3$ | 2,3,5-Trimethoxyphenyl | |
| 126 | SC(S)NR'R | $CH_3$ | 2,3,6-Trimethoxyphenyl | |

| Nr. | D | R' | R | phys. Daten[a)] |
|---|---|---|---|---|
| 127 | SC(S)NR'R | $CH_3$ | 2,4,5-Trichlorphenyl | |
| 128 | SC(S)NR'R | $CH_3$ | 2,4,6-Trichloryphenyl | |
| 129 | SC(S)NR'R | $CH_3$ | 2,3,4,6-Tetrachlorphenyl | |
| 130 | SC(S)NR'R | $CH_3$ | 2,3,5,6-Tetrachlorphenyl | |
| 131 | SC(S)NR'R | $CH_3$ | 2-Bromphenyl | |
| 132 | SC(S)NR'R | $CH_3$ | 3-Bromphenyl | |
| 133 | SC(S)NR'R | $CH_3$ | 4-Bromphenyl | |
| 134 | SC(S)NR'R | $CH_3$ | 2,4-Dibromphenyl | |
| 135 | SC(S)NR'R | $CH_3$ | 3-Brom-4-Fluorphenyl | |
| 136 | SC(S)NR'R | $CH_3$ | 3-Brom-4-Methoxyphenyl | |
| 137 | SC(S)NR'R | $CH_3$ | 2-Jodphenyl | |
| 138 | SC(S)NR'R | $CH_3$ | 3-Jodphenyl | |
| 139 | SC(S)NR'R | $CH_3$ | 4-Jodphenyl | |
| 140 | SC(S)NR'R | $CH_3$ | 2-Chlor-4-Fluorphenyl | |
| 141 | SC(S)NR'R | $CH_3$ | 2-Chlor-5-Fluorphenyl | |
| 142 | SC(S)NR'R | $CH_3$ | 2-Chlor-6-Fluorphenyl | |
| 143 | SC(S)NR'R | $CH_3$ | 2-Chlor-4-Bromphenyl | |
| 144 | SC(S)NR'R | $CH_3$ | 2-Brom-4-Chlorphenyl | |
| 145 | SC(S)NR'R | $CH_3$ | 2-Brom-4-Fluorphenyl | |

| Nr. | D | R' | R | phys. Daten[a)] |
|---|---|---|---|---|
| 146 | SC(S)NR'R | $CH_3$ | 3-Brom-4-Chlorphenyl | |
| 147 | SC(S)NR'R | $CH_3$ | 3-Chlor-4-Fluorphenyl | |
| 148 | SC(S)NR'R | $CH_3$ | 3-Fluor-4-Chlorphenyl | |
| 149 | SC(S)NR'R | $CH_3$ | 2-Cyanophenyl | |
| 150 | SC(S)NR'R | $CH_3$ | 3-Cyanophenyl | |
| 151 | SC(S)NR'R | $CH_3$ | 4-Cyanophenyl | |
| 152 | SC(S)NR'R | $CH_3$ | 2-Nitrophenyl | |
| 153 | SC(S)NR'R | $CH_3$ | 3-Nitrophenyl | |
| 154 | SC(S)NR'R | $CH_3$ | 4-Nitrophenyl | |
| 155 | SC(S)NR'R | $CH_3$ | 2-Methylphenyl | |
| 156 | SC(S)NR'R | $CH_3$ | 3-Methylphenyl | |
| 157 | SC(S)NR'R | $CH_3$ | 4-Methylphenyl | |
| 158 | SC(S)NR'R | $CH_3$ | 2,4-Dimethylphenyl | |
| 159 | SC(S)NR'R | $CH_3$ | 2,6-Dimethylphenyl | |
| 160 | SC(S)NR'R | $CH_3$ | 3,4-Dimethylphenyl | |
| 161 | SC(S)NR'R | $CH_3$ | 3,5-Dimethylphenyl | |
| 162 | SC(S)NR'R | $CH_3$ | 2,3,4-Trimethylphenyl | |
| 163 | SC(S)NR'R | $CH_3$ | 2,3,5-Trimethylphenyl | |
| 164 | SC(S)NR'R | $CH_3$ | 2,3,6-Trimethylphenyl | |

| Nr. | D | R' | R | phys. Daten[a)] |
|---|---|---|---|---|
| 165 | SC(S)NR'R | $CH_3$ | 2,4,5-Trimethylphenyl | |
| 166 | SC(S)NR'R | $CH_3$ | 2,4,6-Trimethylphenyl | |
| 167 | SC(S)NR'R | $CH_3$ | 2,4,5-Trimethylphenyl | |
| 168 | SC(S)NR'R | $CH_3$ | Pentamethylphenyl | |
| 169 | SC(S)NR'R | $CH_3$ | 2-Ethylphenyl | |
| 170 | SC(S)NR'R | $CH_3$ | 3-Ethylphenyl | |
| 171 | SC(S)NR'R | $CH_3$ | 4-Ethylphenyl | |
| 172 | SC(S)NR'R | $CH_3$ | 3,5-Diethylphenyl | |
| 173 | SC(S)NR'R | $CH_3$ | 2-n-Propylphenyl | |
| 174 | SC(S)NR'R | $CH_3$ | 3-n-Propylphenyl | |
| 175 | SC(S)NR'R | $CH_3$ | 4-n-Propylphenyl | |
| 176 | SC(S)NR'R | $CH_3$ | 2,4,5-Trimethoxyphenyl | |
| 177 | SC(S)NR'R | $CH_3$ | 2,4,6-Trimethoxyphenyl | |
| 178 | SC(S)NR'R | $CH_3$ | 3,4,5-Trimethoxyphenyl | |
| 179 | SC(S)NR'R | $CH_3$ | 2-Ethoxyphenyl | |
| 180 | SC(S)NR'R | $CH_3$ | 3-Ethoxyphenyl | |
| 181 | SC(S)NR'R | $CH_3$ | 4-Ethoxyphenyl | |
| 182 | SC(S)NR'R | $CH_3$ | 2-iso-Propoxyphenyl | |
| 183 | SC(S)NR'R | $CH_3$ | 3-iso-Propoxyphenyl | |

| Nr. | D | R' | R | phys. Daten[a)] |
|---|---|---|---|---|
| 184 | SC(S)NR'R | $CH_3$ | 4-iso-Propoxyphenyl | |
| 185 | SC(S)NR'R | $CH_3$ | 3-tert.-Butoxyphenyl | |
| 186 | SC(S)NR'R | $CH_3$ | 4-tert.-Butoxyphenyl | |
| 187 | SC(S)NR'R | $CH_3$ | 2-Trifluormethoxyphenyl | |
| 188 | SC(S)NR'R | $CH_3$ | 3-Trifluormethoxyphenyl | |
| 189 | SC(S)NR'R | $CH_3$ | 4-Trifluormethoxyphenyl | |
| 190 | SC(S)NR'R | $CH_3$ | 3-(1',1',2',2'-Tetrafluor)-ethoxyphenyl | |
| 191 | SC(S)NR'R | $CH_3$ | 4-(1',1',2',2'-Tetrafluor)-ethoxyphenyl | |
| 192 | SC(S)NR'R | $CH_3$ | 2-Chlormethylphenyl | |
| 193 | SC(S)NR'R | $CH_3$ | 3-Chlormethylphenyl | |
| 194 | SC(S)NR'R | $CH_3$ | 4-Chlormethylphenyl | |
| 195 | SC(S)NR'R | $CH_3$ | 2-Trifluormethylphenyl | |
| 196 | SC(S)NR'R | $CH_3$ | 3-Trifluormethylphenyl | |
| 197 | SC(S)NR'R | $CH_3$ | 4-Trifluormethylphenyl | |
| 198 | SC(S)NR'R | $CH_3$ | 2-(Methoxyiminomethyl)-phenyl | |
| 199 | SC(S)NR'R | $CH_3$ | 3-(Methoxyiminomethyl)-phenyl | |
| 200 | SC(S)NR'R | $CH_3$ | 4-(Methoxyiminomethyl)-phenyl | |
| 201 | SC(S)NR'R | $CH_3$ | 2-(Ethoxyiminomethyl)-phenyl | |

| Nr. | D | R' | R | phys. Daten[a)] |
|---|---|---|---|---|
| 202 | SC(S)NR'R | $CH_3$ | 3-(Ethoxyiminomethyl)-phenyl | |
| 203 | SC(S)NR'R | $CH_3$ | 4-(Ethoxyiminomethyl)-phenyl | |
| 204 | SC(S)NR'R | $CH_3$ | 2-Methyl-4-Methoximinoethyl-phenyl | |
| 205 | SC(S)NR'R | $CH_3$ | 2-Methyl-4-Methoximinomethyl-phenyl | |
| 206 | SC(S)NR'R | $CH_3$ | 2,6-Dimethyl-4-Methoximino-methyl-phenyl | |
| 207 | SC(S)NR'R | $CH_3$ | 2,6-Dimethyl-4-Methoximino-ethyl-phenyl | |
| 208 | SC(S)NR'R | $CH_3$ | 2-Phenylphenyl | |
| 209 | SC(S)NR'R | $CH_3$ | 3-Phenylphenyl | |
| 210 | SC(S)NR'R | $CH_3$ | 4-Phenylphenyl | |
| 211 | SC(S)NR'R | $CH_3$ | 2-Phenoxyphenyl | |
| 212 | SC(S)NR'R | $CH_3$ | 3-Phenoxyphenyl | |
| 213 | SC(S)NR'R | $CH_3$ | 4-Phenoxyphenyl | |
| 214 | SC(S)NR'R | $CH_3$ | 2-Benzyloxyphenyl | |
| 215 | SC(S)NR'R | $CH_3$ | 3-Benzyloxyphenyl | |
| 216 | SC(S)NR'R | $CH_3$ | 4-Benzyloxyphenyl | |
| 217 | SC(S)NR'R | $CH_3$ | 4-(Imidazol-1'-yl)phenyl | |
| 218 | SC(S)NR'R | $CH_3$ | 4-(Piperazin-1'-yl)phenyl | |

| Nr. | D | R' | R | phys. Daten[a)] |
|---|---|---|---|---|
| 219 | SC(S)NR'R | $CH_3$ | 4-(Morpholin-1'-yl)phenyl | |
| 220 | SC(S)NR'R | $CH_3$ | 4-(Piperidin-1'-yl)phenyl | |
| 221 | SC(S)NR'R | $CH_3$ | 4-(Pyridyl-2'-oxy)phenyl | |
| 222 | SC(S)NR'R | $CH_3$ | 2-Cyclopropylphenyl | |
| 223 | SC(S)NR'R | $CH_3$ | 3-Cyclopropylphenyl | |
| 224 | SC(S)NR'R | $CH_3$ | 4-Cyclopropylphenyl | |
| 225 | SC(S)NR'R | $CH_3$ | 3-Cyclohexylphenyl | |
| 226 | SC(S)NR'R | $CH_3$ | 4-Cyclohexylphenyl | |
| 227 | SC(S)NR'R | $CH_3$ | 4-Oxiranylphenyl | |
| 228 | SC(S)NR'R | $CH_3$ | 4-(1',3'-Dioxan-2'-yl)-phenyl | |
| 229 | SC(S)NR'R | $CH_3$ | 4-(Tetrahydropyran-2-yl-oxy)-phenyl | |
| 230 | SC(S)NR'R | $CH_3$ | 1-Naphthyl | |
| 231 | SC(S)NR'R | $CH_3$ | 2-Naphthyl | |
| 232 | SC(S)NR'R | $CH_3$ | Benzyl | |
| 233 | SC(S)NR'R | $CH_3$ | 2-Methylbenzyl | |
| 234 | SC(S)NR'R | $CH_3$ | 3-Methylbenzyl | |
| 235 | SC(S)NR'R | $CH_3$ | 4-Methylbenzyl | |
| 236 | SC(S)NR'R | $CH_3$ | 4-tert.-Butylbenzyl | |
| 237 | SC(S)NR'R | $CH_3$ | 2-Chlorbenzyl | |

| Nr. | D | R' | R | phys. Daten[a)] |
|---|---|---|---|---|
| 238 | SC(S)NR'R | $CH_3$ | 3-Chlorbenzyl | |
| 239 | SC(S)NR'R | $CH_3$ | 4-Chlorbenzyl | |
| 240 | SC(S)NR'R | $CH_3$ | 2-Pyridyl | |
| 241 | SC(S)NR'R | $CH_3$ | 3-Pyridyl | |
| 242 | SC(S)NR'R | $CH_3$ | 4-Pyridyl | |
| 243 | SC(S)NR'R | $CH_3$ | 2,6-Pyrimidinyl | |
| 244 | SC(S)NR'R | $CH_3$ | 1,5-Pyrimidinyl | |
| 245 | SC(S)NR'R | $CH_3$ | 2-Thienyl | |
| 246 | SC(S)NR'R | $CH_3$ | 3-Thienyl | |
| 247 | SC(S)NR'R | $CH_3$ | 2-Furyl | |
| 248 | SC(S)NR'R | $CH_3$ | 3-Furyl | |
| 249 | SC(S)NR'R | $CH_3$ | 1-Pyrrolyl | |
| 250 | SC(S)NR'R | $CH_3$ | 1-Imidazolyl | |
| 251 | SC(S)NR'R | $CH_3$ | 1,2,4-Triazolyl | |
| 252 | SC(S)NR'R | $CH_3$ | 1,3,4-Triazolyl | |
| 253 | SC(S)NR'R | $CH_3$ | 4-Thiazolyl | |
| 254 | SC(S)NR'R | $CH_3$ | 2-Benzothiazolyl | |
| 255 | SC(S)NR'R | $CH_3$ | 2-Pyridylmethyl | |
| 256 | SC(S)NR'R | $CH_3$ | 3-Pyridylmethyl | |

| Nr. | D | R' | R | phys. Daten[a)] |
|---|---|---|---|---|
| 257 | SC(S)NR'R | $CH_3$ | 4-Pyridylmethyl | |
| 258 | SC(S)NR'R | Et | Phenyl | |
| 259 | SC(S)NR'R | Et | 2-Methylphenyl | |
| 260 | SC(S)NR'R | Et | 2-Chlor-phenyl | |
| 261 | SC(S)NR'R | Et | 4-Methylphenyl | |
| 262 | SC(S)NR'R | Et | 2-Naphtyl | |
| 263 | SC(S)SR | - | $CH_3$ | |
| 264 | SC(S)SR | - | $CH_2$-phenyl | |
| 265 | SC(S)SR | - | Phenyl | |
| 266 | SC(S)SR | - | $A^*$ | |
| 267 | SC(S)OR | - | $CH_3$ | |
| 268 | SC(S)OR | - | Phenyl | |
| 269 | SC(S)OR | - | 2-$CH_3$-phenyl | |
| 270 | SC(S)OR | - | 3-$CH_3$-phenyl | |
| 271 | SC(S)OR | - | 4-$CH_3$-phenyl | |
| 272 | SC(S)OR | - | 2-Cl-phenyl | |
| 273 | SC(S)OR | - | 3-Cl-phenyl | |
| 274 | SC(S)OR | - | 4-Cl-phenyl | |
| 275 | SC(S)OR | - | $CH_2$-phenyl | |

| Nr. | D | R' | R | phys. Daten[a)] |
|---|---|---|---|---|
| 276 | SC(S)OR | - | $CH_2$-(2-Me)-phenyl | |
| 277 | SC(S)OR | - | $CH_2$-(3-Me)-phenyl | |
| 278 | SC(S)OR | - | $CH_2$-(4-Me)-phenyl | |
| 279 | SC(S)OR | - | $CH_2$-(2-Cl)-phenyl | |
| 280 | SC(S)OR | - | $CH_2$-(3-Cl)phenyl | |
| 281 | SC(S)OR | - | $CH_2$-(4-Cl)phenyl | |
| 282 | OC(O)OR | - | $CH_3$ | |
| 283 | OC(O)OR | - | tert.-Butyl | |
| 284 | OC(O)OR | - | $CH_2$-phenyl | |
| 285 | OC(O)OR | - | Phenyl | |
| 286 | SC(S)NR'R | $CH_3$ | 3-Me, 4-F-phenyl | |

A* = 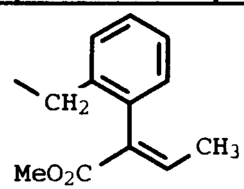

a) IR ($cm^{-1}$); $^1H$-NMR ($CDCl_3$/TMS); m. p.

Tabelle 4

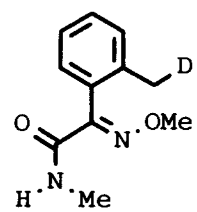

| Nr. | D | R' | R | phys. Daten[a)] |
|---|---|---|---|---|
| 1 | SC(S)NR'R | $CH_3$ | Methyl | |
| 2 | SC(S)NR'R | $CH_3$ | Ethyl | |
| 3 | SC(S)NR'R | $CH_3$ | n-Propyl | |
| 4 | SC(S)NR'R | $CH_3$ | tert.-Butyl | |
| 5 | SC(S)NR'R | $CH_3$ | Cyclopropyl | |
| 6 | SC(S)NR'R | $CH_3$ | Cyclohexyl | |
| 7 | SC(S)NR'R | $CH_3$ | Methoxymethyl | |
| 8 | SC(S)NR'R | $CH_3$ | Phenoxymethyl | |
| 9 | SC(S)NR'R | $CH_3$ | Methylthiomethyl | |
| 10 | SC(S)NR'R | $CH_3$ | Phenyl | |
| 11 | SC(S)NR'R | $CH_3$ | 2-Fluorphenyl | |
| 12 | SC(S)NR'R | $CH_3$ | 3-Fluorphenyl | |

| Nr. | D | R' | R | phys. Daten[a)] |
|---|---|---|---|---|
| 13 | SC(S)NR'R | $CH_3$ | 4-Fluorphenyl | 152 - 156°C |
| 14 | SC(S)NR'R | $CH_3$ | Pentafluorphenyl | |
| 15 | SC(S)NR'R | $CH_3$ | 2-Chlorphenyl | |
| 16 | SC(S)NR'R | $CH_3$ | 3-Chlorphenyl | 2,90 (d, 3H), 3,71, 3,89 (2x3H), 4,35 (2H), 6,72 (1H), 7,10-7,46 (8H) |
| 17 | SC(S)NR'R | $CH_3$ | 4-Chlorphenyl | |
| 18 | SC(S)NR'R | $CH_3$ | Pentachlorphenyl | |
| 19 | SC(S)NR'R | $CH_3$ | 2,3-Dichlorphenyl | |
| 20 | SC(S)NR'R | $CH_3$ | 2,,4-Dichlorphenyl | |
| 21 | SC(S)NR'R | $CH_3$ | 2,5-Dichlorphenyl | |
| 22 | SC(S)NR'R | $CH_3$ | 2,6-Dichlorphenyl | |
| 23 | SC(S)NR'R | $CH_3$ | 3,4-Dichlorphenyl | |
| 24 | SC(S)NR'R | $CH_3$ | 3,5-Dichlorphenyl | |
| 25 | SC(S)NR'R | $CH_3$ | 2,3,4-Trichlorphenyl | |
| 26 | SC(S)NR'R | $CH_3$ | 2,3,5-Trichlorphenyl | |
| 27 | SC(S)NR'R | $CH_3$ | 2,3,6-Trichlorphenyl | |
| 28 | SC(S)NR'R | $CH_3$ | 2,4,5-Trichlorphenyl | |
| 29 | SC(S)NR'R | $CH_3$ | 2,4,6-Trichlorphenyl | |
| 30 | SC(S)NR'R | $CH_3$ | 3,4,5-Trichlorphenyl | |
| 31 | SC(S)NR'R | $CH_3$ | 2,3,4,6-Tetrachlorphenyl | |

| Nr. | D | R' | R | phys. Daten[a)] |
|---|---|---|---|---|
| 32 | SC(S)NR'R | $CH_3$ | 2,3,5,6-Tetrachlorphenyl | |
| 33 | SC(S)NR'R | $CH_3$ | 2-Bromphenyl | |
| 34 | SC(S)NR'R | $CH_3$ | 3-Bromphenyl | |
| 35 | SC(S)NR'R | $CH_3$ | 4-Bromphenyl | |
| 36 | SC(S)NR'R | $CH_3$ | 2,4-Dibromphenyl | |
| 37 | SC(S)NR'R | $CH_3$ | 3-Brom-4-Fluorphenyl | |
| 38 | SC(S)NR'R | $CH_3$ | 3-Brom-4-Methoxyphenyl | |
| 39 | SC(S)NR'R | $CH_3$ | 2-Jodphenyl | |
| 40 | SC(S)NR'R | $CH_3$ | 3-Jodphenyl | |
| 41 | SC(S)NR'R | $CH_3$ | 4-Jodphenyl | |
| 42 | SC(S)NR'R | $CH_3$ | 2-Chlor-4-Fluorphenyl | |
| 43 | SC(S)NR'R | $CH_3$ | 2-Chlor-5-Fluorphenyl | |
| 44 | SC(S)NR'R | $CH_3$ | 2-Chlor-6-Fluorphenyl | |
| 45 | SC(S)NR'R | $CH_3$ | 2-Chlor-4-Bromphenyl | |
| 46 | SC(S)NR'R | $CH_3$ | 2-Brom-4-Chlorphenyl | |
| 47 | SC(S)NR'R | $CH_3$ | 2-Brom-4-Fluorphenyl | |
| 48 | SC(S)NR'R | $CH_3$ | 3-Brom-4-Fluorphenyl | |
| 49 | SC(S)NR'R | $CH_3$ | 3-Chlor-4-Fluorphenyl | |
| 50 | SC(S)NR'R | $CH_3$ | 3-Fluor-4-Chlorphenyl | |

| Nr. | D | R' | R | phys. Daten[a)] |
|---|---|---|---|---|
| 51 | SC(S)NR'R | $CH_3$ | 2-Cyanophenyl | |
| 52 | SC(S)NR'R | $CH_3$ | 3-Cyanophenyl | |
| 53 | SC(S)NR'R | $CH_3$ | 4-Cyanophenyl | |
| 54 | SC(S)NR'R | $CH_3$ | 2-Nitrophenyl | |
| 55 | SC(S)NR'R | $CH_3$ | 3-Nitrophenyl | |
| 56 | SC(S)NR'R | $CH_3$ | 4-Nitrophenyl | |
| 57 | SC(S)NR'R | $CH_3$ | 2-Methylphenyl | |
| 58 | SC(S)NR'R | $CH_3$ | 3-Methylphenyl | 2,36; 3,74; 3,88 (3 x 3H); 2,90 (d, 3H); 4,34 (2H); 6,69 (1H); 7,02-7,47 (8H) |
| 59 | SC(S)NR'R | $CH_3$ | 4-Methylphenyl | 2,35; 3,72; 3,88 (3 x 3H); 2,89 (d, 3H); 4,32 (2H); 6,67 (1H); 7,05-7,43 (8H) |
| 60 | SC(S)NR'R | $CH_3$ | 2,4-Dimethylphenyl | |
| 61 | SC(S)NR'R | $CH_3$ | 2,6-Dimethylphenyl | |
| 62 | SC(S)NR'R | $CH_3$ | 3,4-Dimethylphenyl | |
| 63 | SC(S)NR'R | $CH_3$ | 3,5-Dimethylphenyl | |
| 64 | SC(S)NR'R | $CH_3$ | 2,3,4-Trimethylphenyl | |
| 65 | SC(S)NR'R | $CH_3$ | 2,3,5-Trimethylphenyl | |
| 66 | SC(S)NR'R | $CH_3$ | 2,3,6-Trimethylphenyl | |
| 67 | SC(S)NR'R | $CH_3$ | 2,4,5-Trimethylphenyl | |
| 68 | SC(S)NR'R | $CH_3$ | 2,4,6-Trimethylphenyl | |

| Nr. | D | R' | R | phys. Daten[a)] |
|---|---|---|---|---|
| 69 | SC(S)NR'R | $CH_3$ | 3,4,5-Trimethylphenyl | |
| 70 | SC(S)NR'R | $CH_3$ | Pentamethylphenyl | |
| 71 | SC(S)NR'R | $CH_3$ | 2-Ethylphenyl | |
| 72 | SC(S)NR'R | $CH_3$ | 3-Ethylphenyl | |
| 73 | SC(S)NR'R | $CH_3$ | 4-Ethylphenyl | |
| 74 | SC(S)NR'R | $CH_3$ | 3,5-Diethylphenyl | |
| 75 | SC(S)NR'R | $CH_3$ | 2-n-Propylphenyl | |
| 76 | SC(S)NR'R | $CH_3$ | 3-n-Propylphenyl | |
| 77 | SC(S)NR'R | $CH_3$ | 4-n-Propylphenyl | |
| 78 | SC(S)NR'R | $CH_3$ | 2-iso-Propylphenyl | |
| 79 | SC(S)NR'R | $CH_3$ | 3-iso-Propylphenyl | |
| 80 | SC(S)NR'R | $CH_3$ | 4-iso-Propylphenyl | |
| 81 | SC(S)NR'R | $CH_3$ | 2,4-Di-iso-Propylphenyl | |
| 82 | SC(S)NR'R | $CH_3$ | 3,5-Di-iso-Propylphenyl | |
| 83 | SC(S)NR'R | $CH_3$ | 4-n-Butyphenyl | |
| 84 | SC(S)NR'R | $CH_3$ | 4-sec.-Butylphenyl | |
| 85 | SC(S)NR'R | $CH_3$ | 4-iso-Butylphenyl | |
| 86 | SC(S)NR'R | $CH_3$ | 4-tert.-Butylphenyl | |
| 87 | SC(S)NR'R | $CH_3$ | 3-tert.-Butylphenyl | |

| Nr. | D | R' | R | phys. Daten[a)] |
|---|---|---|---|---|
| 88 | SC(S)NR'R | $CH_3$ | 2-tert.-Butylphenyl | |
| 89 | SC(S)NR'R | $CH_3$ | 2,4-Di-tert.-Butylphenyl | |
| 90 | SC(S)NR'R | $CH_3$ | 3,5-Di-tert.-Butylphenyl | |
| 91 | SC(S)NR'R | $CH_3$ | 4-n-Hexylphenyl | |
| 92 | SC(S)NR'R | $CH_3$ | 4-n-Dodecylphenyl | |
| 93 | SC(S)NR'R | $CH_3$ | 2-Methyl-4-tert.-Butyl-phenyl | |
| 94 | SC(S)NR'R | $CH_3$ | 2-Methyl-6-tert.-Butyl-phenyl | |
| 95 | SC(S)NR'R | $CH_3$ | 2-Methyl-4-iso-Propylphenyl | |
| 96 | SC(S)NR'R | $CH_3$ | 2-Methyl-4-Cyclohexylphenyl | |
| 97 | SC(S)NR'R | $CH_3$ | 2-Methyl-4-Phenylphenyl | |
| 98 | SC(S)NR'R | $CH_3$ | 2-Methyl-4-Benzylphenyl | |
| 99 | SC(S)NR'R | $CH_3$ | 2-Methyl-4-Phenoxyphenyl | |
| 100 | SC(S)NR'R | $CH_3$ | 2-Methyl-4-Benzyloxyphenyl | |
| 101 | SC(S)NR'R | $CH_3$ | 2-Methyl-3-Chlorphenyl | |
| 102 | SC(S)NR'R | $CH_3$ | 2-Methyl-4-Chlorphenyl | |
| 103 | SC(S)NR'R | $CH_3$ | 2-Methyl-5-Chlorphenyl | |
| 104 | SC(S)NR'R | $CH_3$ | 2-Methyl-6-Chlorphenyl | |
| 105 | SC(S)NR'R | $CH_3$ | 2-Methyl-4-Fluorphenyl | |
| 106 | SC(S)NR'R | $CH_3$ | 2-Methyl-3-Bromphenyl | |

| Nr. | D | R' | R | phys. Daten[a)] |
|---|---|---|---|---|
| 107 | SC(S)NR'R | $CH_3$ | 2-Methyl-4-Bromphenyl | |
| 108 | SC(S)NR'R | $CH_3$ | 2-Methyl-3-Methoxyphenyl | |
| 109 | SC(S)NR'R | $CH_3$ | 2-Methyl-4-Methoxyphenyl | |
| 110 | SC(S)NR'R | $CH_3$ | 2-Methyl-5-Methoxyphenyl | |
| 111 | SC(S)NR'R | $CH_3$ | 2-Methyl-6-Methoxyphenyl | |
| 112 | SC(S)NR'R | $CH_3$ | 2-Methyl-4-iso-Propoxy-phenyl | |
| 113 | SC(S)NR'R | $CH_3$ | 2-Methyl-2,5-Dimethoxy-phenyl | |
| 114 | SC(S)NR'R | $CH_3$ | 2-Methoxyphenyl | |
| 115 | SC(S)NR'R | $CH_3$ | 3-Methoxyphenyl | |
| 116 | SC(S)NR'R | $CH_3$ | 4-Methoxyphenyl | |
| 117 | SC(S)NR'R | $CH_3$ | 2,3-Dimethoxyphenyl | |
| 118 | SC(S)NR'R | $CH_3$ | 2,4-Dimethoxyphenyl | |
| 119 | SC(S)NR'R | $CH_3$ | 2,5-Dimethoxyphenyl | |
| 120 | SC(S)NR'R | $CH_3$ | 2,6-Dimethoxyphenyl | |
| 121 | SC(S)NR'R | $CH_3$ | 3,4-Dimethoxyphenyl | |
| 122 | SC(S)NR'R | $CH_3$ | 3,5-Dimethoxyphenyl | |
| 123 | SC(S)NR'R | $CH_3$ | 3,6-Dimethoxyphenyl | |
| 124 | SC(S)NR'R | $CH_3$ | 2,3,4-Trimethoxyphenyl | |
| 125 | SC(S)NR'R | $CH_3$ | 2,3,5-Trimethoxyphenyl | |

| Nr. | D | R' | R | phys. Daten[a)] |
|---|---|---|---|---|
| 126 | SC(S)NR'R | $CH_3$ | 2,3,6-Trimethoxyphenyl | |
| 127 | SC(S)NR'R | $CH_3$ | 2,4,5-Trichlorphenyl | |
| 128 | SC(S)NR'R | $CH_3$ | 2,4,6-Trichloryphenyl | |
| 129 | SC(S)NR'R | $CH_3$ | 2,3,4,6-Tetrachlorphenyl | |
| 130 | SC(S)NR'R | $CH_3$ | 2,3,5,6-Tetrachlorphenyl | |
| 131 | SC(S)NR'R | $CH_3$ | 2-Bromphenyl | |
| 132 | SC(S)NR'R | $CH_3$ | 3-Bromphenyl | |
| 133 | SC(S)NR'R | $CH_3$ | 4-Bromphenyl | |
| 134 | SC(S)NR'R | $CH_3$ | 2,4-Dibromphenyl | |
| 135 | SC(S)NR'R | $CH_3$ | 3-Brom-4-Fluorphenyl | |
| 136 | SC(S)NR'R | $CH_3$ | 3-Brom-4-Methoxyphenyl | |
| 137 | SC(S)NR'R | $CH_3$ | 2-Jodphenyl | |
| 138 | SC(S)NR'R | $CH_3$ | 3-Jodphenyl | |
| 139 | SC(S)NR'R | $CH_3$ | 4-Jodphenyl | |
| 140 | SC(S)NR'R | $CH_3$ | 2-Chlor-4-Fluorphenyl | |
| 141 | SC(S)NR'R | $CH_3$ | 2-Chlor-5-Fluorphenyl | |
| 142 | SC(S)NR'R | $CH_3$ | 2-Chlor-6-Fluorphenyl | |
| 143 | SC(S)NR'R | $CH_3$ | 2-Chlor-4-Bromphenyl | |
| 144 | SC(S)NR'R | $CH_3$ | 2-Brom-4-Chlorphenyl | |

| Nr. | D | R' | R | phys. Daten[a)] |
|---|---|---|---|---|
| 145 | SC(S)NR'R | $CH_3$ | 2-Brom-4-Fluorphenyl | |
| 146 | SC(S)NR'R | $CH_3$ | 3-Brom-4-Chlorphenyl | |
| 147 | SC(S)NR'R | $CH_3$ | 3-Chlor-4-Fluorphenyl | |
| 148 | SC(S)NR'R | $CH_3$ | 3-Fluor-4-Chlorphenyl | |
| 149 | SC(S)NR'R | $CH_3$ | 2-Cyanophenyl | |
| 150 | SC(S)NR'R | $CH_3$ | 3-Cyanophenyl | |
| 151 | SC(S)NR'R | $CH_3$ | 4-Cyanophenyl | |
| 152 | SC(S)NR'R | $CH_3$ | 2-Nitrophenyl | |
| 153 | SC(S)NR'R | $CH_3$ | 3-Nitrophenyl | |
| 154 | SC(S)NR'R | $CH_3$ | 4-Nitrophenyl | |
| 155 | SC(S)NR'R | $CH_3$ | 2-Methylphenyl | |
| 156 | SC(S)NR'R | $CH_3$ | 3-Methylphenyl | |
| 157 | SC(S)NR'R | $CH_3$ | 4-Methylphenyl | |
| 158 | SC(S)NR'R | $CH_3$ | 2,4-Dimethylphenyl | |
| 159 | SC(S)NR'R | $CH_3$ | 2,6-Dimethylphenyl | |
| 160 | SC(S)NR'R | $CH_3$ | 3,4-Dimethylphenyl | |
| 161 | SC(S)NR'R | $CH_3$ | 3,5-Dimethylphenyl | |
| 162 | SC(S)NR'R | $CH_3$ | 2,3,4-Trimethylphenyl | |
| 163 | SC(S)NR'R | $CH_3$ | 2,3,5-Trimethylphenyl | |

| Nr. | D | R' | R | phys. Daten[a)] |
|---|---|---|---|---|
| 164 | SC(S)NR'R | $CH_3$ | 2,3,6-Trimethylphenyl | |
| 165 | SC(S)NR'R | $CH_3$ | 2,4,5-Trimethylphenyl | |
| 166 | SC(S)NR'R | $CH_3$ | 2,4,6-Trimethylphenyl | |
| 167 | SC(S)NR'R | $CH_3$ | 2,4,5-Trimethylphenyl | |
| 168 | SC(S)NR'R | $CH_3$ | Pentamethylphenyl | |
| 169 | SC(S)NR'R | $CH_3$ | 2-Ethylphenyl | |
| 170 | SC(S)NR'R | $CH_3$ | 3-Ethylphenyl | |
| 171 | SC(S)NR'R | $CH_3$ | 4-Ethylphenyl | |
| 172 | SC(S)NR'R | $CH_3$ | 3,5-Diethylphenyl | |
| 173 | SC(S)NR'R | $CH_3$ | 2-n-Propylphenyl | |
| 174 | SC(S)NR'R | $CH_3$ | 3-n-Propylphenyl | |
| 175 | SC(S)NR'R | $CH_3$ | 4-n-Propylphenyl | |
| 176 | SC(S)NR'R | $CH_3$ | 2,4,5-Trimethoxyphenyl | |
| 177 | SC(S)NR'R | $CH_3$ | 2,4,6-Trimethoxyphenyl | |
| 178 | SC(S)NR'R | $CH_3$ | 3,4,5-Trimethoxyphenyl | |
| 179 | SC(S)NR'R | $CH_3$ | 2-Ethoxyphenyl | |
| 180 | SC(S)NR'R | $CH_3$ | 3-Ethoxyphenyl | |
| 181 | SC(S)NR'R | $CH_3$ | 4-Ethoxyphenyl | |
| 182 | SC(S)NR'R | $CH_3$ | 2-iso-Propoxyphenyl | |

| Nr. | D | R' | R | phys. Daten[a)] |
|---|---|---|---|---|
| 183 | SC(S)NR'R | $CH_3$ | 3-iso-Propoxyphenyl | |
| 184 | SC(S)NR'R | $CH_3$ | 4-iso-Propoxyphenyl | |
| 185 | SC(S)NR'R | $CH_3$ | 3-tert.-Butoxyphenyl | |
| 186 | SC(S)NR'R | $CH_3$ | 4-tert.-Butoxyphenyl | |
| 187 | SC(S)NR'R | $CH_3$ | 2-Trifluormethoxyphenyl | |
| 188 | SC(S)NR'R | $CH_3$ | 3-Trifluormethoxyphenyl | |
| 189 | SC(S)NR'R | $CH_3$ | 4-Trifluormethoxyphenyl | |
| 190 | SC(S)NR'R | $CH_3$ | 3-(1',1',2',2'-Tetra-fluor)-ethoxyphenyl | |
| 191 | SC(S)NR'R | $CH_3$ | 4-(1',1',2',2'-Tetra-fluor)-ethoxyphenyl | |
| 192 | SC(S)NR'R | $CH_3$ | 2-Chlormethylphenyl | |
| 193 | SC(S)NR'R | $CH_3$ | 3-Chlormethylphenyl | |
| 194 | SC(S)NR'R | $CH_3$ | 4-Chlormethylphenyl | |
| 195 | SC(S)NR'R | $CH_3$ | 2-Trifluormethylphenyl | |
| 196 | SC(S)NR'R | $CH_3$ | 3-Trifluormethylphenyl | |
| 197 | SC(S)NR'R | $CH_3$ | 4-Trifluormethylphenyl | |
| 198 | SC(S)NR'R | $CH_3$ | 2-(Methoxyiminomethyl)-phenyl | |
| 199 | SC(S)NR'R | $CH_3$ | 3-(Methoxyiminomethyl)-phenyl | |
| 200 | SC(S)NR'R | $CH_3$ | 4-(Methoxyiminomethyl)-phenyl | |

| Nr. | D | R' | R | phys. Daten[a)] |
|---|---|---|---|---|
| 201 | SC(S)NR'R | $CH_3$ | 2-(Ethoxyiminomethyl)-phenyl | |
| 202 | SC(S)NR'R | $CH_3$ | 3-(Ethoxyiminomethyl)-phenyl | |
| 203 | SC(S)NR'R | $CH_3$ | 4-(Ethoxyiminomethyl)-phenyl | |
| 204 | SC(S)NR'R | $CH_3$ | 2-Methyl-4-Methoximinoethyl-phenyl | |
| 205 | SC(S)NR'R | $CH_3$ | 2-Methyl-4-Methoximinomethyl-phenyl | |
| 206 | SC(S)NR'R | $CH_3$ | 2,6-Dimethyl-4-Methoximino-methyl-phenyl | |
| 207 | SC(S)NR'R | $CH_3$ | 2,6-Dimethyl-4-Methoximino-ethyl-phenyl | |
| 208 | SC(S)NR'R | $CH_3$ | 2-Phenylphenyl | |
| 209 | SC(S)NR'R | $CH_3$ | 3-Phenylphenyl | |
| 210 | SC(S)NR'R | $CH_3$ | 4-Phenylphenyl | |
| 211 | SC(S)NR'R | $CH_3$ | 2-Phenoxyphenyl | |
| 212 | SC(S)NR'R | $CH_3$ | 3-Phenoxyphenyl | |
| 213 | SC(S)NR'R | $CH_3$ | 4-Phenoxyphenyl | |
| 214 | SC(S)NR'R | $CH_3$ | 2-Benzyloxyphenyl | |
| 215 | SC(S)NR'R | $CH_3$ | 3-Benzyloxyphenyl | |
| 216 | SC(S)NR'R | $CH_3$ | 4-Benzyloxyphenyl | |
| 217 | SC(S)NR'R | $CH_3$ | 4-(Imidazol-1'-yl)phenyl | |

| Nr. | D | R' | R | phys. Daten[a)] |
|---|---|---|---|---|
| 218 | SC(S)NR'R | $CH_3$ | 4-(Piperazin-1'-yl)phenyl | |
| 219 | SC(S)NR'R | $CH_3$ | 4-(Morpholin-1'-yl)phenyl | |
| 220 | SC(S)NR'R | $CH_3$ | 4-(Piperidin-1'-yl)phenyl | |
| 221 | SC(S)NR'R | $CH_3$ | 4-(Pyridyl-2'-oxy)phenyl | |
| 222 | SC(S)NR'R | $CH_3$ | 2-Cyclopropylphenyl | |
| 223 | SC(S)NR'R | $CH_3$ | 3-Cyclopropylphenyl | |
| 224 | SC(S)NR'R | $CH_3$ | 4-Cyclopropylphenyl | |
| 225 | SC(S)NR'R | $CH_3$ | 3-Cyclohexylphenyl | |
| 226 | SC(S)NR'R | $CH_3$ | 4-Cyclohexylphenyl | |
| 227 | SC(S)NR'R | $CH_3$ | 4-Oxiranylphenyl | |
| 228 | SC(S)NR'R | $CH_3$ | 4-(1',3'-Dioxan-2'-yl)-phenyl | |
| 229 | SC(S)NR'R | $CH_3$ | 4-(Tetrahydropyran-2-yl-oxy)-phenyl | |
| 230 | SC(S)NR'R | $CH_3$ | 1-Naphthyl | |
| 231 | SC(S)NR'R | $CH_3$ | 2-Naphthyl | |
| 232 | SC(S)NR'R | $CH_3$ | Benzyl | |
| 233 | SC(S)NR'R | $CH_3$ | 2-Methylbenzyl | |
| 234 | SC(S)NR'R | $CH_3$ | 3-Methylbenzyl | |
| 235 | SC(S)NR'R | $CH_3$ | 4-Methylbenzyl | |
| 236 | SC(S)NR'R | $CH_3$ | 4-tert.-Butylbenzyl | |

| Nr. | D | R' | R | phys. Daten[a)] |
|---|---|---|---|---|
| 237 | SC(S)NR'R | $CH_3$ | 2-Chlorbenzyl | |
| 238 | SC(S)NR'R | $CH_3$ | 3-Chlorbenzyl | |
| 239 | SC(S)NR'R | $CH_3$ | 4-Chlorbenzyl | |
| 240 | SC(S)NR'R | $CH_3$ | 2-Pyridyl | |
| 241 | SC(S)NR'R | $CH_3$ | 3-Pyridyl | |
| 242 | SC(S)NR'R | $CH_3$ | 4-Pyridyl | |
| 243 | SC(S)NR'R | $CH_3$ | 2,6-Pyrimidinyl | |
| 244 | SC(S)NR'R | $CH_3$ | 1,5-Pyrimidinyl | |
| 245 | SC(S)NR'R | $CH_3$ | 2-Thienyl | |
| 246 | SC(S)NR'R | $CH_3$ | 3-Thienyl | |
| 247 | SC(S)NR'R | $CH_3$ | 2-Furyl | |
| 248 | SC(S)NR'R | $CH_3$ | 3-Furyl | |
| 249 | SC(S)NR'R | $CH_3$ | 1-Pyrrolyl | |
| 250 | SC(S)NR'R | $CH_3$ | 1-Imidazolyl | |
| 251 | SC(S)NR'R | $CH_3$ | 1,2,4-Triazolyl | |
| 252 | SC(S)NR'R | $CH_3$ | 1,3,4-Triazolyl | |
| 253 | SC(S)NR'R | $CH_3$ | 4-Thiazolyl | |
| 254 | SC(S)NR'R | $CH_3$ | 2-Benzothiazolyl | |
| 255 | SC(S)NR'R | $CH_3$ | 2-Pyridylmethyl | |

| Nr. | D | R' | R | phys. Daten[a)] |
|---|---|---|---|---|
| 256 | SC(S)NR'R | $CH_3$ | 3-Pyridylmethyl | |
| 257 | SC(S)NR'R | $CH_3$ | 4-Pyridylmethyl | |
| 258 | SC(S)NR'R | Et | Phenyl | 162°C |
| 259 | SC(S)NR'R | Et | 2-Methylphenyl | |
| 260 | SC(S)NR'R | Et | 2-Chlor-phenyl | |
| 261 | SC(S)NR'R | Et | 4-Methylphenyl | 1663, 1523, 1508, 1444, 1399, 1385, 1272, 1103, 1038 |
| 262 | SC(S)NR'R | Et | 2-Naphtyl | |
| 263 | SC(S)SR | - | $CH_3$ | |
| 264 | SC(S)SR | - | $CH_2$-phenyl | |
| 265 | SC(S)SR | - | Phenyl | |
| 266 | SC(S)SR | - | A* | |
| 267 | SC(S)OR | - | $CH_3$ | |
| 268 | SC(S)OR | - | Phenyl | |
| 269 | SC(S)OR | - | 2-$CH_3$-phenyl | |
| 270 | SC(S)OR | - | 3-$CH_3$-phenyl | |
| 271 | SC(S)OR | - | 4-$CH_3$-phenyl | |
| 272 | SC(S)OR | - | 2-Cl-phenyl | |
| 273 | SC(S)OR | - | 3-Cl-phenyl | |
| 274 | SC(S)OR | - | 4-Cl-phenyl | |

| Nr. | D | R' | R | phys. Daten[a)] |
|---|---|---|---|---|
| 275 | SC(S)OR | – | $CH_2$-phenyl | |
| 276 | SC(S)OR | – | $CH_2$-(2-Me)-phenyl | |
| 277 | SC(S)OR | – | $CH_2$-(3-Me)-phenyl | |
| 278 | SC(S)OR | – | $CH_2$-(4-Me)-phenyl | |
| 279 | SC(S)OR | – | $CH_2$-(2-Cl)-phenyl | |
| 280 | SC(S)OR | – | $CH_2$-(3-Cl)phenyl | |
| 281 | SC(S)OR | – | $CH_2$-(4-Cl)phenyl | |
| 282 | OC(O)OR | – | $CH_3$ | |
| 283 | OC(O)OR | – | tert.-Butyl | |
| 284 | OC(O)OR | – | $CH_2$-phenyl | |
| 285 | OC(O)OR | – | Phenyl | 5,13 ppm ($CH_2$) |
| 286 | SC(S)NR'R | $CH_3$ | 3-Me, 4-F-phenyl | 2,29; 3,73; 3,90 (3 x 3H), 2,92 (d, 3H), 4,35 2H), 6,70 (1H), 6,98-7,46 (7H) |
| 287 | OC(O)NR'R | H | i-Propyl | 138 - 140°C |
| 288 | OC(O)NR'R | H | tert.-Butyl | 143 - 145°C |
| 289 | OC(O)NR'R | H | $C(Me)_2C{\equiv}C-H$ | 117 - 119°C |
| 290 | OC(O)NR'R | H | 2-Me-phenyl | 140 - 141°C |
| 291 | OC(O)NR'R | H | 3-Me-phenyl | 150 - 152°C |
| 292 | OC(O)NR'R | H | 4-Me-phenyl | 204 - 205°C |
| 293 | OC(O)NR'R | H | 4-F-phenyl | 136 - 139°C |

| Nr. | D | R' | R | phys. Daten[a)] |
|---|---|---|---|---|
| 294 | OC(O)NR'R | H | 2,6-$Cl_2$-phenyl | 195 - 198°C |
| 295 | OC(O)NR'R | H | 3,5-$Cl_2$-phenyl | 162 - 164°C |

A* = 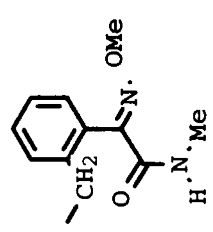

a) IR ($cm^{-1}$); $^1H$-NMR ($CDCl_3$/TMS); m. p.

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwekken; sie sollten in

jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz-und Nußschalenmehl, Cellulosepulver oder andere feste Trägerstoffe.

Beispiele für solche Zubereitungen sind:

I. eine Lösung aus 90 Gew.-Teilen der Verbindung Nr. 16 aus Tabelle 4 und 10 Gew.-Teilen N-Methyl-a-pyrrolidon, die zur Anwendung in Form kleinster Tropfen geeignet ist;

II. eine Mischung aus 20 Gew.-Teilen der Verbindung Nr. 58 aus Tabelle 4, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 59 aus Tabelle 4, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 157 aus Tabelle 4, 25 Gew.-Teilen Cyclohexanol, 65 Gew.-Teilen einer Mineralölfraktion vom Siedepunkt 210 bis 280'C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 286 aus Tabelle 4, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 16 aus Tabelle 4 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 58 aus Tabelle 4, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 59 aus Tabelle 4, 10 Gew.-Teilen des Natriumsalzes eines Phenolsulfonsäure-harn-stoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 157 aus Tabelle 4, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüse-pflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Anwendungsbeispiele

Als Vergleichswirkstoffe wurden α-[2-(Phenylthiomethyl)-phenyl]-β-methoxyacrylsäuremethylester (A) und 2-(Phenylthiomethyl)-α-methoxyimino-phenylessigsäuremethylester (B) verwendet.

Anwendungsbeispiel 1

Wirksamkeit gegen Weizenmehltau

Blätter von in Töpfen gewachsenen Weizenkeimlingen der Sorte "Frühgold' wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewachshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Das Ergebnis zeigt, daß der Wirkstoff aus Tabelle 1, Nr. 16 bei der Anwendung als 63 ppm Wirkstoff enthaltende Spritzbrühe eine bessere fungizide Wirkung zeigt (15 % Befall der Blätter) als der bekannte Vergleichswirkstoff A (40 % Befall der Blätter).

Anwendungsbeispiel 2

Wirksamkeit gegen Plasmopara viticola

Blätter von Topfreben der Sorte "Müller Thurgau' wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blatter mit einer Zoosporenaufschwemmung von Plasmopara viticola (Rebenperononspora) infiziert. Danach wurden die Reben zunächst für 48 Stunden in einer wasserdampfgesättigten Kammer bei 24 °C und anschließend für 5 Tage in einem Gewächshaus bei Temperaturen zwischen 20 und 30 °C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß der Wirkstoff aus Tabelle 2, Nr. 13 bei der Anwendung als 63 ppm Wirkstoff enthaltende Spritz-brühe eine bessere fungizide Wirkung zeigt (15 % Befall der Blätter) als der bekannte Wirkstoff B (40 % Befall der Blätter).

**Patentansprüche**

**1.** Benzylderivat der Formel I

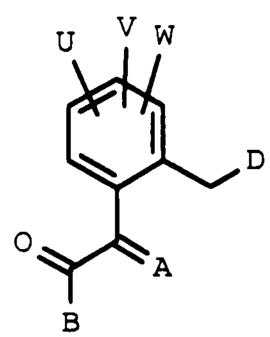

I

in der

A

$CH_2$, CHCl, CH-$C_1$-$C_4$-Alkyl, CH-$C_1$-$C_4$-Alkoxy, CH-$C_1$-$C_4$-Alkylthio, N-$C_1$-$C_4$-Alkoxy bedeutet,

B

OH, $C_1$-$C_4$-Alkylthio, $C_1$-$C_4$-Alkoxy, $C_1$-$C_4$-Alkylamino bedeutet,

U, V, W

gleich oder verschieden sind und Wasserstoff, Halogen, $C_1$-$C_4$-Alkyl oder $C_1$-$C_4$-Alkoxy bedeuten,

D

die Gruppierung

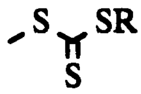 , 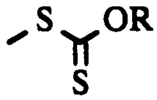 , 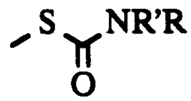 , 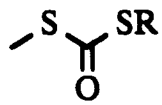

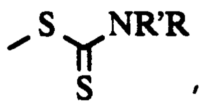 , 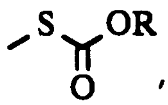 , 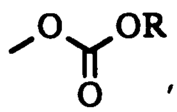 , 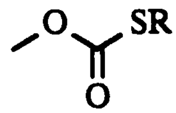

oder 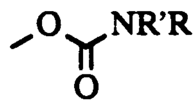

bedeutet,
wobei
R'
Wasserstoff oder $C_1$-$C_4$ -Alkyl bedeutet und
R
Wasserstoff, $C_1$-$C_{10}$-Alkyl, $C_3$-$C_6$-Cycloalkyl, $C_1$-$C_4$-Halogenalkyl, $C_3$-$C_6$-Halogencycloalkyl, $C_3$-$C_6$-Cycloalkyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, ggf. subst. Arylthio-$C_1$-$C_4$-alkyl, ggf. subst. Aryloxy-$C_1$-$C_4$-alkyl, ggf. subst. Aryl, ggf. subst. Aryl-$C_1$-$C_4$-alkyl, ggf. subst. Hetaryl, ggf. subst. Hetaryl-$C_1$-$C_4$-alkyl, ggf. subst. Hetaryloxy-$C_1$-$C_4$-alkyl, ggf. subst. Heterocyclyl bedeutet, wobei "ggf. subst." neben Wasserstoff die Reste Halogen, Cyano, $CO_2$($C_1$-$C_4$-Alkyl), CO($C_1$-$C_4$-Alkyl), Nitro, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoximino-$C_1$-$C_2$-alkyl, Aryl, Aryloxy, Benzyloxy, Hetaryl, Hetaryloxy, $C_3$-$C_6$-Cycloalkyl, Heterocyclyl, Heterocyclyloxy bedeuten.

**2.** Benzylderivat der Formel I gemäß Anspruch 1, in der A CH-$OCH_3$ und B Methoxy bedeutet, und U, V, W und D die in Anspruch 1 angegebenen Bedeutungen haben.

**3.** Benzylderivat der Formel I gemäß Anspruch 1, in der A CH-Methyl und B Methoxy bedeuten und U, V, W und D die in Anspruch 1 angegebene Bedeutung haben.

**4.** Benzylderivat der Formel I gemäß Anspruch 1, in der A N-Methoxy und B Methoxy bedeuten und U, V, W und D die in Anspruch 1 angegebene Bedeutung haben.

**5.** Benzylderivat der Formel I gemäß Anspruch 1, in der A N-Methoxy und B N-Methyl bedeuten und U, V, W und D die in Anspruch 1 angegebene Bedeutung haben.

**6.** Verfahren zur Herstellung von Benzylderivaten der Formel I gemäß Anspruch 1, dadurch gekennzeichnet, daß man ein Benzylderivat der Formel 6

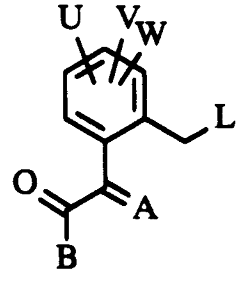 6

in der A und B die in Anspruch 1 genannte Bedeutung haben und L eine Abgangsgruppe bedeutet, mit einem Kohlensäurederivat 10

$M^{\oplus}D^{\ominus}$ 10

in der M für ein Metall steht und D die in Anspruch 1 angegebene Bedeutung hat, umsetzt.

**7.** Fungizid, enthaltend einen inerten Trägerstoff und eine fungizid wirksame Menge eines Benzylderivats der Formel I

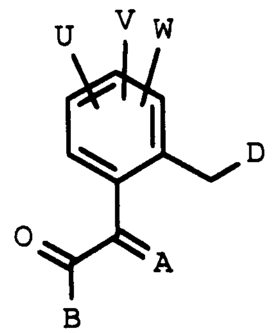

I

in der

A

$CH_2$, CHCl, CH-$C_1$-$C_4$-Alkyl, CH-$C_1$-$C_4$-Alkoxy, CH-$C_1$-$C_4$-Alkylthio, N-$C_1$-$C_4$-Alkoxy bedeutet,

B

OH, $C_1$-$C_4$-Alkylthio, $C_1$-$C_4$-Alkoxy, $C_1$-$C_4$-Alkylamino bedeutet,

U, V, W

gleich oder verschieden sind und Wasserstoff, Halogen, $C_1$-$C_4$-Alkyl oder $C_1$-$C_4$-Alkoxy bedeuten,

D

die Gruppierung

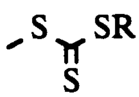 , 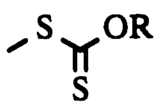 , 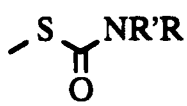 , 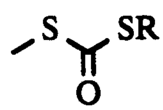

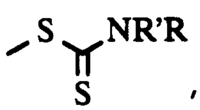 , 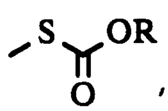 , 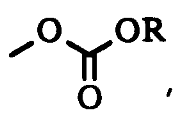 , 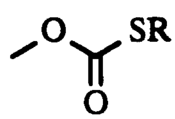

oder 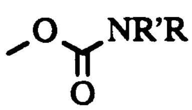

bedeutet,

wobei

R'

Wasserstoff oder $C_1$-$C_4$-Alkyl bedeutet und

R

Wasserstoff, $C_1$-$C_{10}$-Alkyl, $C_3$-$C_6$-Cycloalkyl, $C_1$-$C_4$-Halogenalkyl, $C_3$-$C_6$-Halogencycloalkyl, $C_3$-$C_6$-Cycloalkyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, ggf. subst. Arylthio-$C_1$-$C_4$-alkyl, ggf. subst. Aryloxy-$C_1$-$C_4$-alkyl, ggf. subst. Aryl, ggf. subst. Aryl-$C_1$-$C_4$-alkyl, ggf. subst. Hetaryl, ggf. subst. Hetaryl-$C_1$-$C_4$-alkyl, ggf. subst. Hetaryloxy-$C_1$-$C_4$-alkyl, ggf. subst. Heterocyclyl bedeutet, wobei "ggf. subst." neben Wasserstoff die Reste Halogen, Cyano, $CO_2$($C_1$-$C_4$-Alkyl), CO($C_1$-$C_4$-Alkyl), Nitro, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoximino-$C_1$-$C_2$-alkyl, Aryl, Aryloxy, Benzyloxy, Hetaryl, Hetaryloxy, $C_3$-$C_6$-Cycloalkyl, Heterocyclyl, Heterocyclyloxy bedeuten.

8. Verfahren zur Bekämpfung von Pilzen, dadurch gekennzeichnet, daß man die Pilze oder die von Pilzbefall bedrohten Materialien, Pflanzen, Saatgut oder den Erdboden behandelt mit einer fungizid wirksamen Menge einer Verbindung der Formel I

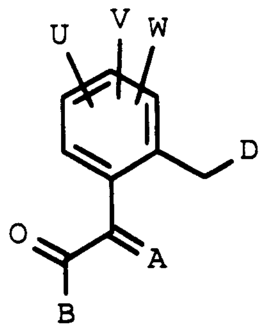

I

in der
A
$CH_2$, CHCl, CH-$C_1$-$C_4$-Alkyl, CH-$C_1$-$C_4$-Alkoxy, CH-$C_1$-$C_4$-Alkylthio, N-$C_1$-$C_4$-Alkoxy bedeutet,
B
OH, $C_1$-$C_4$-Alkylthio, $C_1$-$C_4$-Alkoxy, $C_1$-$C_4$-Alkylamino bedeutet,
U, V, W
gleich oder verschieden sind und Wasserstoff, Halogen, $C_1$-$C_4$-Alkyl oder $C_1$-$C_4$-Alkoxy bedeuten,
D
die Gruppierung

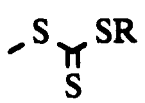 , 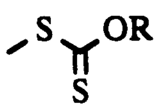 , 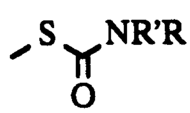 , 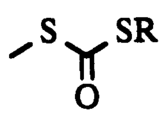

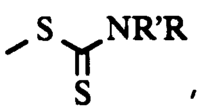 , 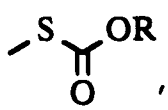 , 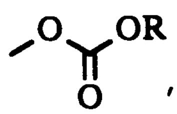 , 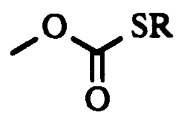

oder 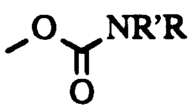

bedeutet,
wobei
R'
Wasserstoff oder $C_1$-$C_4$-Alkyl bedeutet und
R
Wasserstoff, $C_1$-$C_{10}$-Alkyl, $C_3$-$C_6$-Cycloalkyl, $C_1$-$C_4$-Halogenalkyl, $C_3$-$C_6$-Halogencycloalkyl, $C_3$-$C_6$-Cycloalkyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, ggf. subst. Arylthio-$C_1$-$C_4$-alkyl, ggf. subst. Aryloxy-$C_1$-$C_4$-alkyl, ggf. subst. Aryl, ggf. subst. Aryl-$C_1$-$C_4$-alkyl, ggf. subst. Hetaryl, ggf. subst. Hetaryl-$C_1$-$C_4$-alkyl, ggf. subst. Hetaryloxy-$C_1$-$C_4$-alkyl, ggf. subst. Heterocyclyl bedeutet, wobei "ggf. subst." neben Wasserstoff die Reste Halogen, Cyano, $CO_2$($C_1$-$C_4$-Alkyl), CO($C_1$-$C_4$-Alkyl), Nitro, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoximino-$C_1$-$C_2$-alkyl, Aryl, Aryloxy, Benzyloxy, Hetaryl, Hetaryloxy, $C_3$-$C_6$-Cycloalkyl, Heterocyclyl, Heterocyclyloxy bedeuten.

9. Verfahren zur Bekämpfung von Insekten, dadurch gekennzeichnet, daß man die Insekten oder den Ort an dem sich die Insekten aufhalten mit einer insektizid wirksamen Menge einer Verbindung der Formel

I behandelt,

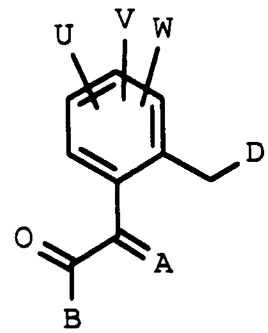

I

in der
A
$CH_2$, CHCl, CH-$C_1$-$C_4$-Alkyl, CH-$C_1$-$C_4$-Alkoxy, CH-$C_1$-$C_4$-Alkylthio, N-$C_1$-$C_4$-Alkoxy bedeutet,
B
OH, $C_1$-$C_4$-Alkylthio, $C_1$-$C_4$-Alkoxy, $C_1$-$C_4$-Alkylamino bedeutet,
U, V, W
gleich oder verschieden sind und Wasserstoff, Halogen, $C_1$-$C_4$-Alkyl oder $C_1$-$C_4$-Alkoxy bedeuten,
D
die Gruppierung

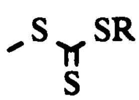 , 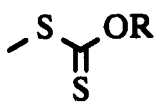 , 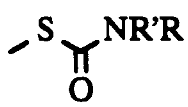 , 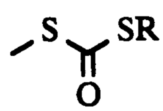

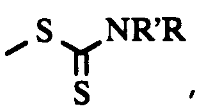 , 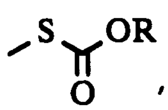 , 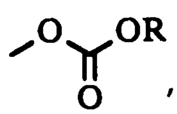 , 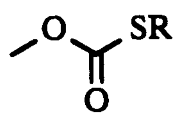

oder 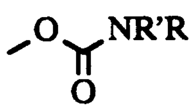

bedeutet,
wobei
R'
Wasserstoff oder $C_1$-$C_4$-Alkyl bedeutet und
R
Wasserstoff, $C_1$-$C_{10}$-Alkyl, $C_3$-$C_6$-Cycloalkyl, $C_1$-$C_4$-Halogenalkyl, $C_3$-$C_6$-Halogencycloalkyl, $C_3$-$C_6$-Cycloalkyl-$C_1$-$C_4$-alkyl, $C_1$-$C_4$-Alkoxy-$C_1$-$C_4$-alkyl, $C_1$-$C_4$-Alkylthio-$C_1$-$C_4$-alkyl, ggf. subst. Arylthio-$C_1$-$C_4$-alkyl, ggf. subst. Aryloxy-$C_1$-$C_4$-alkyl, ggf. subst. Aryl, ggf. subst. Aryl-$C_1$-$C_4$-alkyl, ggf. subst. Hetaryl, ggf. subst. Hetaryl-$C_1$-$C_4$-alkyl, ggf. subst. Hetaryloxy-$C_1$-$C_4$-alkyl, ggf. subst. Heterocyclyl bedeutet, wobei "ggf. subst." neben Wasserstoff die Reste Halogen, Cyano, $CO_2$($C_1$-$C_4$-Alkyl), CO($C_1$-$C_4$-Alkyl), Nitro, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoximino-$C_1$-$C_2$-alkyl, Aryl, Aryloxy, Benzyloxy, Hetaryl, Hetaryloxy, $C_3$-$C_6$-Cycloalkyl, Heterocyclyl, Heterocyclyloxy bedeuten.

**10.** Verbindung der Formel I gemäß Anspruch 1, dadurch gekennzeichnet, daß
A N-Methoxy,
B N-Methyl,

D

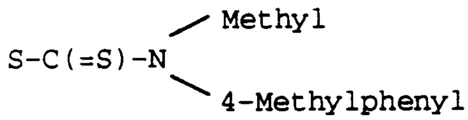

U, V und W Wasserstoff bedeuten.

**Claims**

1. A benzyl derivative of the formula I

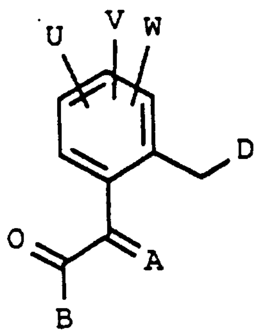

I

where
A is $CH_2$, CHCl, CH-$C_1$-$C_4$-alkyl, CH-$C_1$-$C_4$-alkoxy, CH-$C_1$-$C_4$-alkylthio or N-$C_1$-$C_4$-alkoxy,
B is OH, $C_1$-$C_4$-alkylthio, $C_1$-$C_4$-alkoxy or $C_1$-$C_4$-alkylamino,
U, V and W are identical or different and are each hydrogen, halogen, $C_1$-$C_4$-alkyl or $C_1$-$C_4$-alkoxy,
D is

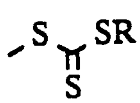 , 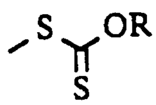 , 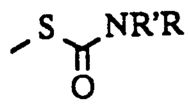 , 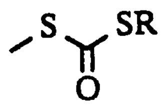

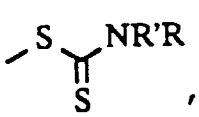 , 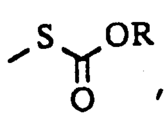 , 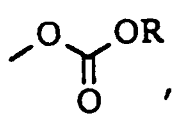 , 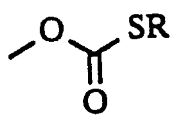

or 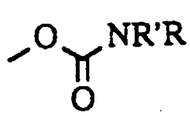

where
R' is hydrogen or $C_1$-$C_4$-alkyl and
R is hydrogen, $C_1$-$C_{10}$-alkyl, $C_3$-$C_6$-cycloalkyl, $C_1$-$C_4$-haloalkyl, $C_3$-$C_6$-halocycloalkyl, $C_3$-$C_6$-cycloalkyl-$C_1$-$C_4$-alkyl, $C_1$-$C_4$-alkoxy-$C_1$-$C_4$-alkyl, $C_1$-$C_4$-alkylthio-$C_1$-$C_4$-alkyl, unsubstituted or substituted arylthio-$C_1$-$C_4$-alkyl, unsubstituted or substituted aryloxy-$C_1$-$C_4$-alkyl, unsubstituted or substituted aryl, unsub-

stituted or substituted aryl-$C_1$-$C_4$-alkyl, unsubstituted or substituted hetaryl, unsubstituted or substituted hetaryl-$C_1$-$C_4$-alkyl, unsubstituted or substituted hetaryloxy-$C_1$-$C_4$-alkyl or unsubstituted or substituted heterocyclyl, substituents being halogen, cyano, $CO_2(C_1$-$C_4$-alkyl), CO($C_1$-$C_4$-alkyl), nitro, $C_1$-$C_4$-alkyl, $C_1$-$C_4$-alkoxy, $C_1$-$C_4$-haloalkyl, $C_1$-$C_4$-haloalkoxy, $C_1$-$C_4$-alkoximino-$C_1$- or $C_2$-alkyl, aryl, aryloxy, benzyloxy, hetaryl, hetaryloxy, $C_3$-$C_6$-cycloalkyl, heterocyclyl or heterocyclyloxy.

2. A benzyl derivative of the formula I as claimed in claim 1, wherein A is CH-$OCH_3$, B is methoxy and U, V, W and D have the meanings stated in claim 1.

3. A benzyl derivative of the formula I as claimed in claim 1, wherein A is CH-methyl, B is methoxy and U, V, W and D have the meanings stated in claim 1.

4. A benzyl derivative of the formula I as claimed in claim 1, wherein A is N-methoxy, B is methoxy and U, V, W and D have the meanings stated in claim 1.

5. A benzyl derivative of the formula I as claimed in claim 1, wherein A is N-methoxy, B is N-methyl and U, V, W and D have the meanings stated in claim 1.

6. A process for the preparation of a benzyl derivative of the formula I as claimed in claim 1, wherein a benzyl derivative of the formula 6

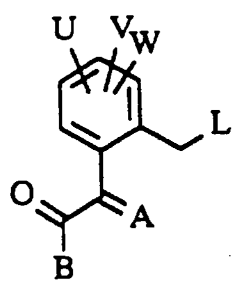

6

where A and B have the meanings stated in claim 1 and L is a leaving group, is reacted with a carbonic acid derivative 10

$M^{\oplus}D^{\ominus}$ 10

where M is a metal and D has the meanings stated in claim 1.

7. A fungicide containing an inert carrier and a fungicidal amount of a benzyl derivative of the formula I

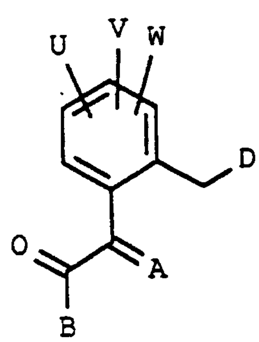

I

where
A is $CH_2$, CHCl, CH-$C_1$-$C_4$-alkyl, CH-$C_1$-$C_4$-alkoxy, CH-$C_1$-$C_4$-alkylthio or N-$C_1$-$C_4$-alkoxy,
B is OH, $C_1$-$C_4$-alkylthio, $C_1$-$C_4$-alkoxy or $C_1$-$C_4$-alkylamino,
U, V and W are identical or different and are each hydrogen, halogen, $C_1$-$C_4$-alkyl or $C_1$-$C_4$-alkoxy,

D is

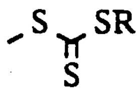 , 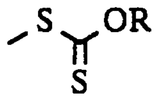 , 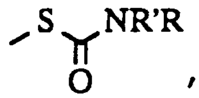 , 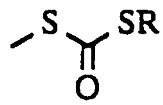

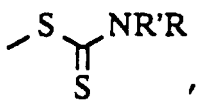 , 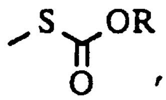 , 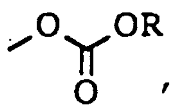 , 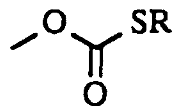

or 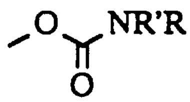

where

R' is hydrogen or $C_1$-$C_4$-alkyl and

R is hydrogen, $C_1$-$C_{10}$-alkyl, $C_3$-$C_6$-cycloalkyl, $C_1$-$C_4$-haloalkyl, $C_3$-$C_6$-halocycloalkyl, $C_3$-$C_6$-cycloalkyl-$C_1$-$C_4$-alkyl, $C_1$-$C_4$-alkoxy-$C_1$-$C_4$-alkyl, $C_1$-$C_4$-alkylthio-$C_1$-$C_4$-alkyl, unsubstituted or substituted arylthio-$C_1$-$C_4$-alkyl, unsubstituted or substituted aryloxy-$C_1$-$C_4$-alkyl, unsubstituted or substituted aryl, unsubstituted or substituted aryl-$C_1$-$C_4$-alkyl, unsubstituted or substituted hetaryl, unsubstituted or substituted hetaryl-$C_1$-$C_4$-alkyl, unsubstituted or substituted hetaryloxy-$C_1$-$C_4$-alkyl or unsubstituted or substituted heterocyclyl, substituents being halogen, cyano, $CO_2(C_1$-$C_4$-alkyl), CO($C_1$-$C_4$-alkyl), nitro, $C_1$-$C_4$-alkyl, $C_1$-$C_4$-alkoxy, $C_1$-$C_4$-haloalkyl, $C_1$-$C_4$-haloalkoxy, $C_1$-$C_4$-alkoximino-$C_1$- or $C_2$-alkyl, aryl, aryloxy, benzyloxy, hetaryl, hetaryloxy, $C_3$-$C_6$-cycloalkyl, heterocyclyl or heterocyclyloxy.

**8.** A method for controlling fungi, wherein the fungi or the materials, plants or seed threatened by fungal attack or the soil is or are treated with a fungicidal amount of a compound of the formula I

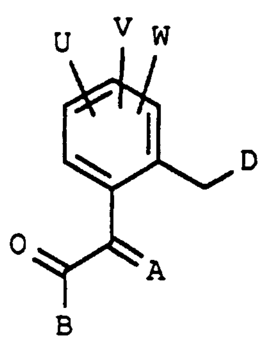 I

where

A is $CH_2$, CHCl, CH-$C_1$-$C_4$-alkyl, CH-$C_1$-$C_4$-alkoxy, CH-$C_1$-$C_4$-alkylthio or N-$C_1$-$C_4$-alkoxy,

B is OH, $C_1$-$C_4$-alkylthio, $C_1$-$C_4$-alkoxy or $C_1$-$C_4$-alkylamino,

U, V and W are identical or different and are each hydrogen, halogen, $C_1$-$C_4$-alkyl or $C_1$-$C_4$-alkoxy,

D is

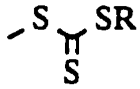 , 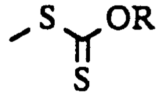 , 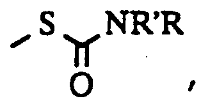 , 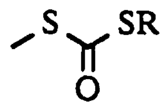

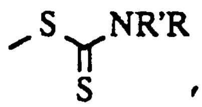 , 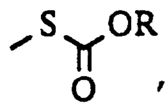 , 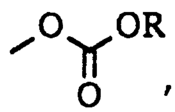 , 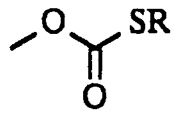

or 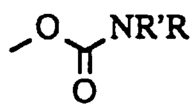

where
R' is hydrogen or $C_1$-$C_4$-alkyl and
R is hydrogen, $C_1$-$C_{10}$-alkyl, $C_3$-$C_6$-cycloalkyl, $C_1$-$C_4$-haloalkyl, $C_3$-$C_6$-halocycloalkyl, $C_3$-$C_6$-cycloalkyl-$C_1$-$C_4$-alkyl, $C_1$-$C_4$-alkoxy-$C_1$-$C_4$-alkyl, $C_1$-$C_4$-alkylthio-$C_1$-$C_4$-alkyl, unsubstituted or substituted arylthio-$C_1$-$C_4$-alkyl, unsubstituted or substituted aryloxy-$C_1$-$C_4$-alkyl, unsubstituted or substituted aryl, unsubstituted or substituted aryl-$C_1$-$C_4$-alkyl, unsubstituted or substituted hetaryl, unsubstituted or substituted hetaryl-$C_1$-$C_4$-alkyl, unsubstituted or substituted hetaryloxy-$C_1$-$C_4$-alkyl or unsubstituted or substituted heterocyclyl, substituents being halogen, cyano, $CO_2$($C_1$-$C_4$-alkyl), CO($C_1$-$C_4$-alkyl), nitro, $C_1$-$C_4$-alkyl, $C_1$-$C_4$-alkoxy, $C_1$-$C_4$-haloalkyl, $C_1$-$C_4$-haloalkoxy, $C_1$-$C_4$-alkoximino-$C_1$- or $C_2$-alkyl, aryl, aryloxy, benzyloxy, hetaryl, hetaryloxy, $C_3$-$C_6$-cycloalkyl, heterocyclyl or heterocyclyloxy.

**9.** A method for controlling insects, wherein the insects or the place where the insects are present is or are treated with an insecticidal amount of a compound of the formula I

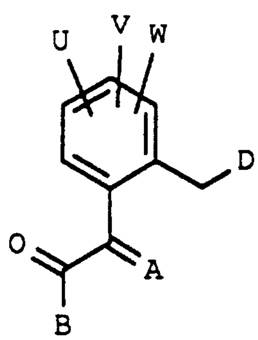 I

where
A is $CH_2$, CHCl, CH-$C_1$-$C_4$-alkyl, CH-$C_1$-$C_4$-alkoxy, CH-$C_1$-$C_4$-alkylthio or N-$C_1$-$C_4$-alkoxy,
B is OH, $C_1$-$C_4$-alkylthio, $C_1$-$C_4$-alkoxy or $C_1$-$C_4$-alkylamino,
U, V and W are identical or different and are each hydrogen, halogen, $C_1$-$C_4$-alkyl or $C_1$-$C_4$-alkoxy,
D is

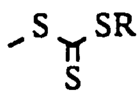, 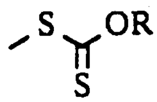, 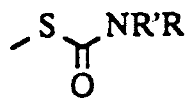, 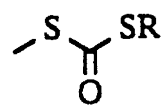

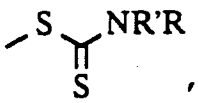, 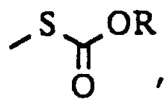, 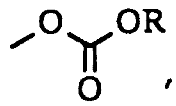, 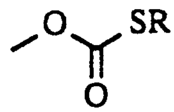

or 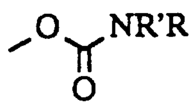

where
R' is hydrogen or $C_1$-$C_4$-alkyl and
R is hydrogen, $C_1$-$C_{10}$-alkyl, $C_3$-$C_6$-cycloalkyl, $C_1$-$C_4$-haloalkyl, $C_3$-$C_6$-halocycloalkyl, $C_3$-$C_6$-cycloalkyl-$C_1$-$C_4$-alkyl, $C_1$-$C_4$-alkoxy-$C_1$-$C_4$-alkyl, $C_1$-$C_4$-alkylthio-$C_1$-$C_4$-alkyl, unsubstituted or substituted arylthio-$C_1$-$C_4$-alkyl, unsubstituted or substituted aryloxy-$C_1$-$C_4$-alkyl, unsubstituted or substituted aryl, unsubstituted or substituted aryl-$C_1$-$C_4$-alkyl, unsubstituted or substituted hetaryl, unsubstituted or substituted hetaryl-$C_1$-$C_4$-alkyl, unsubstituted or substituted hetaryloxy-$C_1$-$C_4$-alkyl or unsubstituted or substituted heterocyclyl, substituents being halogen, cyano, $CO_2$($C_1$-$C_4$-alkyl), CO($C_1$-$C_4$-alkyl), nitro, $C_1$-$C_4$-alkyl, $C_1$-$C_4$-alkoxy, $C_1$-$C_4$-haloalkyl, $C_1$-$C_4$-haloalkoxy, $C_1$-$C_4$-alkoximino-$C_1$- or $C_2$-alkyl, aryl, aryloxy, benzyloxy, hetaryl, hetaryloxy, $C_3$-$C_6$-cycloalkyl, heterocyclyl or heterocyclyloxy.

**10.** A compound of the formula I as claimed in claim 1, wherein
A is N-methoxy,
B is N-methyl,
D is

```
              methyl
             /
S-C(=S)-N
             \
              4-methylphenyl
```

and
U, V and W are each hydrogen.

## Revendications

1. Dérivé benzylique de formule I

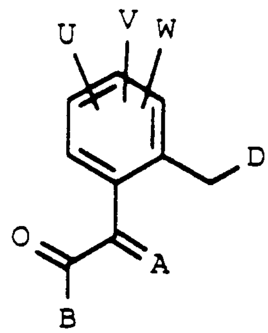

I

dans laquelle
A représente $CH_2$, CHCl, CH-alkyle en C1-C4, CH-alcoxy en C1-C4, CH-alkylthio en C1-C4, N-alcoxy en C1-C4,
B représente OH, alkyle en C1-C4 thio, alcoxy en C1-C4, alkyle en C1-C4 amino,
U, V, W sont identiques ou différents et représentent, hydrogène, halogène, alkyle en C1-C4 ou alcoxy en C1-C4,
D représente le groupement

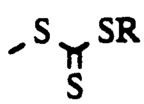 , 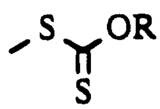 , 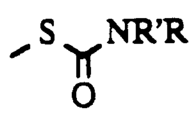 , 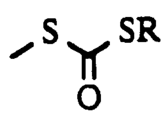

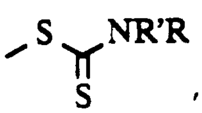 , 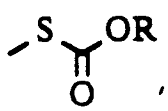 , 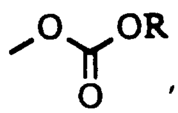 , 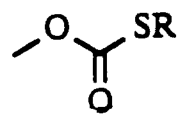

ou 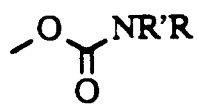

R' représente hydrogène ou alkyle en C1-C4 et
R représente hydrogène, alkyle en C1-C10, cycloalkyle en C3-C6, halogènalkyle en C1-C4, halogéno-cycloalkyle en C3-C6, cycloalkyle en C3-C6, alkyle en C1-C4, alcoxy en C1-C4-alkyle en C1-C4, alkyle en C1-C4 thio-alkyle en C1-C4, arylthio-alkyle en C1-C4 éventuellement substitué aryloxy-alkyle en C1-C4, aryle éventuellement substitué aryl-alkyle en C1-C4 éventuellement substitué hétaryle, éventuellement substitué hétaryl-alkyle en C1-C4, éventuellement substitué hétaryloxy-alkyle en C1-C4, éventuellement substitué hétérocyclyle éventuellement substitué représente, outre hydrogène, les restes halogène, cyano, $CO_2$-(alkyle en C1-C4, CO(alkyle en C1-C4), nitro, alkyle en C1-C4, alcoxy en C1-C4, halogènalkyle en C1-C4, halogènalcoxy en C1-C4, iminoalcoxy en C1-C4-alkyle en C1-C2, aryle, aryloxy, benzyloxy, hétaryle, hétaryloxy, cycloalkyle en C3-C6, hétérocyclyle, hétérocyclyloxy.

2. Dérivé benzylique de formule I selon la revendication 1, dans laquelle A représente CH-$OCH_3$ et B méthoxy et U, V, W et D ont les représentations indiquées dans la revendication 1.

3. Dérivé benzylique de formule I selon la revendication 1, dans laquelle A représente CH-méthyle et B méthoxy et U, V, W et D ont les représentations indiquées dans la revendication 1.

4. Dérivé benzylique de formule I selon la revendication 1, dans laquelle A représente N-méthoxy et B méthoxy et U, V, W et D ont les représentations indiquées dans la revendication 1.

**5.** Dérivé benzylique de formule I selon la revendication 1, dans laquelle A représente N-méthoxy et B N-méthyl et U, V, W et D ont les représentations indiquées dans la revendication 1.

**6.** Procédé de préparation de dérivés benzyliques de la formule I selon la revendication 1, caractérisé par le fait que l'on fait réagir un dérivé benzylique de formule 6

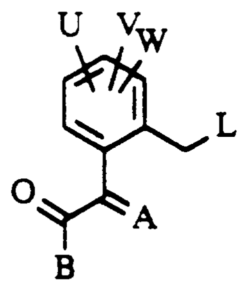

6

dans laquelle A et B ont les significations indiquées dans la revendication 1 et L représente un groupe éliminable, avec un dérivé d'acide carboxylique 10

$M^{\oplus}D^{\ominus}$ 10

dans lequel M est mis pour un métal et D a la signification donnée dans la revendication 1.

**7.** Fongicide contenant un support inerte et une quantité à effet fongicide d'un dérivé benzylique de formule I

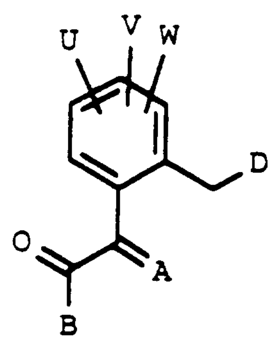

I

dans laquelle
A représente CH2, CHCl, CH-alkyle en C1-C4, CH-alcoxy en C1-C4, CH-alkylthio en C1-C4, N-alcoxy en C1-C4,
B représente OH, alkyle en C1-C4 thio, alcoxy en C1-C4, alkyle en C1-C4 amino,
U, V, W sont identiques ou différents et représentent, hydrogène, halogène, alkyle en C1-C4 ou alcoxy en C1-C4,
D représente le groupement

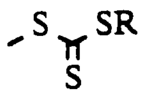 , 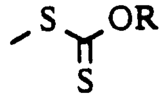 , 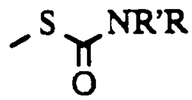 , 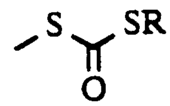

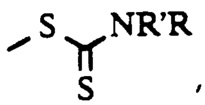 , 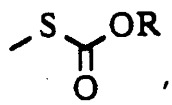 , 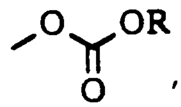 , 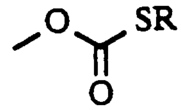

ou 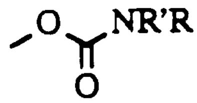

R' représente hydrogène ou alkyle en C1-C4 et
R représente hydrogène, alkyle en C1-C10, cycloalkyle en C3-C6, halogènalkyle en C1-C4, halogèncycloalkyle en C3-C6, cycloalkyle en C3-C6, alkyle en C1-C4, alcoxy en C1-C4-alkyle en C1-C4, alkyle en C1-C4 thio-alkyle en C1-C4, arylthio-alkyle en C1-C4 éventuellement substitué aryloxy-alkyle en C1-C4, aryle éventuellement substitué arylalkyle en C1-C4 éventuellement substitué hétaryle, éventuellement substitué hétaryl-alkyle en C1-C4, éventuellement substitué hétaryloxy-alkyle en C1-C4, éventuellement substitué hétérocyclyle éventuellement substitué représente, outre hydrogène, les restes halogène, cyano $CO_2$-(alkyle en C1-C4, CO(alkyle en C1-C4), nitro, alkyle en C1-C4, alcoxy en C1-C4, halogènalkyle en C1-C4, halogènalcoxy en C1-C4, iminoalcoxy en C1-C4-alkyle en C1-C2, aryle, aryloxy, benzyloxy, hétaryle, hétaryloxy, cycloalkyle en C3-C6, hétérocyclyle, hétérocyclyloxy.

**8.** Procédé de lutte contre les champignons, caractérisé par le fait que l'on traite les champignons ou les matériaux, plantes, semences ou leur biotope menacés d'une attaque des champignons, avec une quantité à effet fongicide d'un dérivé benzylique de formule I

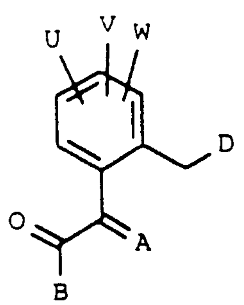

I

dans laquelle
A représente $CH_2$, CHCl, CH-alkyle en C1-C4, CH-alcoxy en C1-C4, CH-alkylthio en C1-C4, N-alcoxy en C1-C4,
B représente OH, alkyle en C1-C4 thio, alcoxy en C1-C4, alkyle en C1-C4 amino,
U, V, W sont identiques ou différents et représentent, hydrogène, halogène, alkyle en C1-C4 ou alcoxy en C1-C4,
D représente le groupement

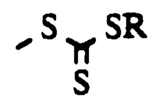, 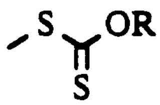, 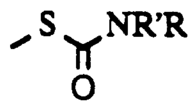, 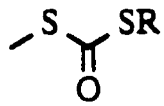

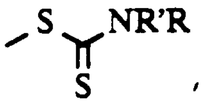, 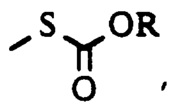, 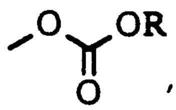, 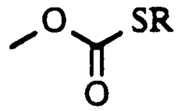

ou 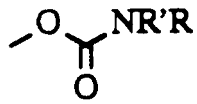

R' représente hydrogène ou alkyle en C1-C4 et
R représente hydrogène, alkyle en C1-C10, cycloalkyle en C3-C6, halogènalkyle en C1-C4, halogéno-cycloalkyle en C3-C6, cycloalkyle en C3-C6, alkyle en C1-C4, alcoxy en C1-C4-alkyle en C1-C4, alkyle en C1-C4 thio-alkyle en C1-C4, arylthio-alkyle en C1-C4 éventuellement substitué aryloxy-alkyle en C1-C4, aryle éventuellement substitué aryl-alkyle en C1-C4 éventuellement substitué hétaryle, éventuellement substitué hétaryl-alkyle en C1-C4, éventuellement substitué hétaryloxy-alkyle en C1-C4, éventuellement substitué hétérocyclyle éventuellement substitué représente, outre hydrogène, les restes halogène, cyano $CO_2$-(alkyle en C1-C4, CO(alkyle en C1-C4), nitro, alkyle en C1-C4, alcoxy en C1-C4, halogènalkyle en C1-C4, halogènalcoxy en C1-C4, iminoalcoxy en C1-C4-alkyle en C1-C2, aryle, aryloxy, benzyloxy, hétaryle, hétaryloxy, cycloalkyle en C3-C6, hétérocyclyle, hétérocyclyloxy.

**9.** Procédé de lutte contre les insectes, caractérisé par le fait que l'on traite les insectes ou le lieu sur lequel ils se posent avec une quantité à effet insecticide d'un dérivé benzylique de formule I

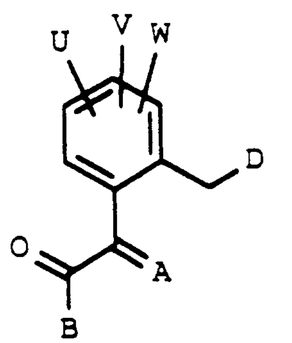

I

dans laquelle
A représente CH2, CHCl, CH-alkyle en C1-C4, CH-alcoxy en C1-C4, CH-alkylthio en C1-C4, N-alcoxy en C1-C4,
B représente OH, alkyle en C1-C4 thio, alcoxy en C1-C4, alkyle en C1-C4 amino,
U, V, W sont identiques ou différents et représentent, hydrogène, halogène, alkyle en C1-C4 ou alcoxy en C1-C4,
D représente le groupement

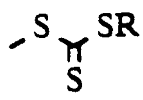, 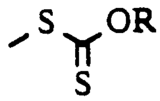, 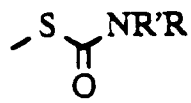, 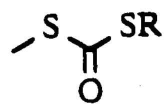

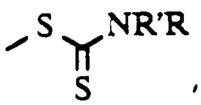, 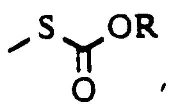, 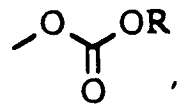, 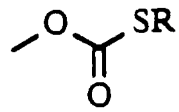

ou 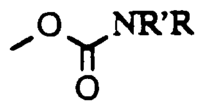

R' représente hydrogène ou alkyle en C1-C4 et
R représente hydrogène, alkyle en C1-C10, cycloalkyle en C3-C6, halogènalkyle en C1-C4, halogénocycloalkyle en C3-C6, cycloalkyle en C3-C6, alkyle en C1-C4, alcoxy en C1-C4-alkyle en C1-C4, alkyle en C1-C4 thio-alkyle en C1-C4, arylthio-alkyle en C1-C4 éventuellement substitué aryloxy-alkyle en C1-C4, aryle éventuellement substitué aryl-alkyle en C1-C4 éventuellement substitué hétaryle, éventuellement substitué hétaryl-alkyle en C1-C4, éventuellement substitué hétaryloxy-alkyle en C1-C4, éventuellement substitué hétérocyclyle éventuellement substitué représente, outre hydrogène, les restes halogène, cyano, $CO_2$-(alkyle en C1-C4, CO(alkyle en C1-C4), nitro, alkyle en C1-C4, alcoxy en C1-C4, halogènalkyle en C1-C4, halogènalcoxy en C1-C4, iminoalcoxy en C1-C7-alkyle en C1-C2, aryle, aryloxy, benzyloxy, hétaryle, hétaryloxy, cycloalkyle en C3-C6, hétérocyclyle, hétérocyclyloxy.

**10.** Composé de formule I selon la revendication 1 caractérisé par le fait que
A représente N-méthoxy
B représente N-méthyle
D représente

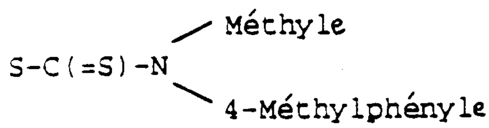

U, V, et W représentent hydrogène.